# EUROPEAN PATENT SPECIFICATION

(11) **EP 3 250 590 B1**
(45) Date of publication and mention of the grant of the patent: **15.09.2021**
(21) Application number: 16744256.5
(22) Date of filing: 30.01.2016
(51) Int. Cl.: C07K 16/28, C07K 16/30, C12P 19/14, A61P 35/00

(54) **COMPOSITIONS AND METHODS RELATING TO UNIVERSAL GLYCOFORMS FOR ENHANCED ANTI-SSEA4 ANTIBODY EFFICACY**
ZUSAMMENSETZUNGEN UND VERFAHREN IM ZUSAMMENHANG MIT UNIVERSELLEN GLYKOFORMEN FÜR ERHÖHTE ANTI-SSEA4 ANTIKÖRPEREFFIZIENZ
COMPOSITIONS ET PROCÉDÉS CONCERNANT DES GLYCOFORMES UNIVERSELLES POUR UNE EFFICACITÉ D'ANTICORPS ANTI-SSEA4 AMÉLIORÉE

(30) Priority: 30.01.2015 US 201562110338 P; 27.05.2015 US 201514723297; 13.07.2015 US 201514798312
(43) Date of publication of application: 06.12.2017
(73) Proprietor: Academia Sinica, Nankang, Taipei 115 (TW)
(72) Inventor: WONG, Chi-Huey, Rancho Santa Fe, CA 92067 (US); WU, Chung-Yi, 221 New Taipei City (TW); MA, Che, 115 Taipei (TW); WU, Han-Chung, 115 Taipei (TW)
(74) Representative: Lavoix
(86) International application number: PCT/US2016/015858
(87) International publication number: WO 2016/123593

(56) References cited:
- WO-A1-2008/133857
- WO-A1-2011/074621
- WO-A1-2013/120066
- WO-A1-2014/078373
- WO-A1-2015/184009
- WO-A1-2016/114819
- WO-A2-2015/109180
- US-A1- 2006 286 637
- US-A1- 2011 263 828
- US-A1- 2013 196 356
- US-A1- 2016 280 794
- US-A1- 2016 289 340
- Y.-W. LOU ET AL: "Stage-specific embryonic antigen-4 as a potential therapeutic target in glioblastoma multiforme and other cancers", PROCEEDINGS OF THE NATIONAL ACADEMY OF SCIENCES, vol. 111, no. 7, 3 February 2014 (2014-02-03), pages 2482-2487, XP55218264, ISSN: 0027-8424, DOI: 10.1073/pnas.1400283111
- WEI HUANG ET AL: "Chemoenzymatic Glycoengineering of Intact IgG Antibodies for Gain of Functions", JOURNAL OF THE AMERICAN CHEMICAL SOCIETY, vol. 134, no. 29, 16 July 2012 (2012-07-16), pages 12308-12318, XP055425387, US ISSN: 0002-7863, DOI: 10.1021/ja3051266
- LOU ET AL.: 'Stage-specific embryonic antigen-4 as a potential therapeutic target in glioblastoma multiforme and other cancers' PROC NATL ACAD SCI USA vol. 111, no. 7, 2014, pages 2482 - 7, XP055218264
- ZOU ET AL.: 'Chemoenzymatic synthesis and Fcgamma receptor binding of homogeneous glycoforms of antibody Fc domain. Presence of a bisecting sugar moiety enhances the affinity of Fc to Fcllla receptor' J AM CHEM SOC. vol. 133, no. 46, 2011, pages 18975 - 91, XP055061687
- SCALLON ET AL: "Higher levels of sialylated Fc glycans in immunoglobulin G molecules can adversely impact functionality", MOLECULAR IMMUNOLOGY, PERGAMON, GB, vol. 44, no. 7, 26 November 2006 (2006-11-26), pages 1524-1534, XP005792683, ISSN: 0161-5890, DOI: 10.1016/J.MOLIMM.2006.09.005

## Description

### FIELD

The present disclosure relates to selected universal Fc glycoforms tuned to the desired binding/effector activity for enhancing the therapeutic efficacies of antibodies directed against many diseases, including cancers, inflammatory disorders and infectious diseases. Particularly, the selected and/or directed optimized universal Fc glycoforms can be generated and/or incorporated to the design and/or the generation of monoclonal antibodies for enhanced therapeutic efficacy.

### BACKGROUND

Antibody-based therapies have a proven record of efficacy against many diseases including inflammatory disorders, cancers, infectious diseases, and solid organ transplant rejection. Currently, more than 40 therapeutic monoclonal antibodies (mAbs) are approved for clinical use in USA, EU and several other countries. Most of those are for therapy of cancer and immune diseases. Examples of therapeutic antibodies with anti-tumor activities include anti-CD20, anti-Her2, anti-EGFR, anti-CD40, anti-CTLA-4, and anti-PD-1 antibodies.

The majority of approved biopharmaceuticals are produced in mammalian cell culture systems to deliver proteins with desired glycosylation patterns and thus ensure reduced immunogenicity and higher in vivo efficacy and stability. Non-human mammalian expression systems such as CHO or NS0 cells have the machinery required to add complex, human-type glycans. However, glycans produced in these systems can differ from glycans produced in humans. Their glycosylation machinery often adds undesired carbohydrate determinants which may alter protein folding, induce immunogenicity, and reduce circulatory life span of the drug.

Furthermore, mammalian cell culture delivers a heterogeneous mixture of glycosylation patterns which do not all have the same properties. Properties like safety, efficacy and the serum half-life of therapeutic proteins can be affected by these glycosylation patterns. The mammalian cell culture system delivers heterogeneous mixtures of glycosylation patterns which do not all have the same properties. Lou Y.-W. et al., PNAS, 2014, 111(7), 2482-2487, relates to stage-specific embryonic antigen-4 (SSEA-4) as a therapeutic target in glioblastoma multiforme and other cancers. WO 2015/184009 relates to universal glycoforms for enhanced antibody efficacy and antibodies comprising a glycoengineered Fc region, wherein the Fc region comprises an N-gylcan of the structure Sia2(α2-6)Gal2GlcNAc2Man3GlcNAc2.

### SUMMARY

The invention is as defined in the claims.

Fc glycosylation has been an important subject in the field of therapeutic monoclonal antibodies. Fc glycosylation can significantly modify Fc effector functions such as Fc receptor binding and complement activation, and thus affect the in vivo safety and efficacy profiles of therapeutic antibodies. Diversity in Fc glycosylation within an antibody will correspond to diversity in Fc effector functions. Thus, this heterogeneity in Fc glycans has a functional consequence as it influences binding of IgG molecules to Fc receptors and thereby impacts antibody effector functions, and may trigger undesired effects in patients thus deeming them a safety concern.

There is a need for improved monoclonal antibody therapy against many diseases including inflammatory disorders, cancers and infectious diseases. Some specific glycoforms in Fc can confer desired biological functions with improved effector functions, such as antibody-dependent cellular cytotoxicity (ADCC). Thus, it is useful to generate therapeutic antibodies with optimized Fc glycoforms.

Accordingly, the present disclosure provide selected universal Fc glycoforms tuned to the desired binding/effector activity for enhancing the efficacy of therapeutic antibodies against many diseases, including cancers, inflammatory disorders and infectious diseases. The selected and/or directed optimized universal Fc glycoforms can be applied and/or incorporated to the design and/or the generation of monoclonal antibodies (preferably, therapeutic monoclonal antibodies) for enhanced therapeutic efficacy.

In one aspect, the present invention provides an isolated monoclonal antibody (MC41) or a binding fragment thereof that binds to Neu5Acα2→ 3Galβ1→ 3GalNAcβ1→ 3Galα1→ 4Galβ1→ 4Glcβ1 (SSEA-4 hexasaccharide), wherein the isolated monoclonal antibody or the binding fragment thereof is a defucosylated antibody comprising a Fc glycoform attached to the Fc region, wherein said glycoform has the formula:

Sia2(α2-6)Gal2GlcNAc2Man3GlcNAc2

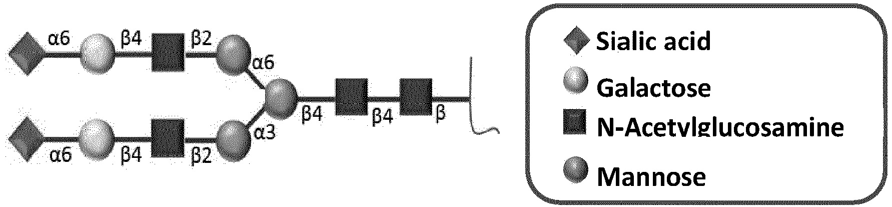

wherein the isolated monoclonal antibody or the binding fragment thereof comprises an H-CDR1, H-CDR2, H-CDR3, L-CDR1, L-CDR2, and L-CDR3, wherein:
(i) the H-CDR1 has the SEQ ID NO:207;
(ii) the H-CDR2 has the SEQ ID NO: 208;
(iii) the H-CDR3 thas the SEQ ID NO: 209;
(iv) the L-CDR1 has the SEQ ID NO: 204;
(v) the L-CDR2 has the SEQ ID NO:205;
(vi) the L-CDR3 thas the SEQ ID NO: 206.

In a preferred embodiment, the isolated monoclonal antibody or the binding fragment thereof comprises a VH having SEQ ID NO: 202 and a VL having SEQ ID NO: 203.

In another aspect, the present invention provides a monoclonal antibody (MC41) or a binding fragment thereof that binds to Neu5Acα2→ 3Galβ1→ 3GalNAcβ1→ 3Galα1→ 4Galβ1→ 4Glcβ1 (SSEA-4 hexasaccharide), wherein the isolated monoclonal antibody or the binding fragment thereof is a defucosylated antibody comprising a Fc glycoform attached to the Fc region, wherein said glycoform has the formula:

Sia₂(α2-6)Gal₂GlcNAc₂Man₃GlcNAc₂

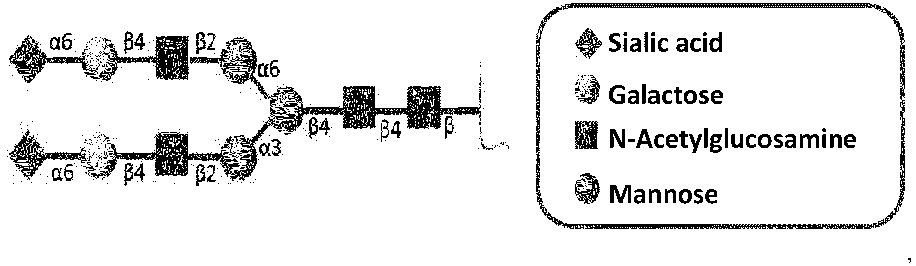

wherein the antibody or the fragment thereof comprises an H-CDR1, H-CDR2, H-CDR3, L-CDR1, L-CDR2, and L-CDR3, wherein:
(i) the H-CDR1 has the sequence of SEQ ID NO: 217;
(ii) the H-CDR2 has the sequence of SEQ ID NO: 218;
(iii) the H-CDR3 has the sequence of SEQ ID NO: 219;
(iv) the L-CDR1 has the sequence of SEQ ID NO: 214;
(v) the L-CDR2 has the sequence of SEQ ID NO: 215; and
(vi) the L-CDR3 has the sequence of SEQ ID NO: 216.

In a preferred embodiment, the isolated monoclonal antibody or the binding fragment thereof comprises a VH having SEQ ID NO: 212 and a VL having SEQ ID NO: 213.

In another aspect, the present invention provides an isolated monoclonal antibody (MC41) or a binding fragment thereof that binds to Neu5Acα2→ 3Galβ1→ 3GalNAcβ1→ 3Galα1→ 4Galβ1→ 4Glcβ1 (SSEA-4 hexasaccharide), wherein the isolated monoclonal antibody or the binding fragment thereof is a defucosylated antibody comprising a Fc glycoform attached to the Fc region, wherein said glycoform has the formula: 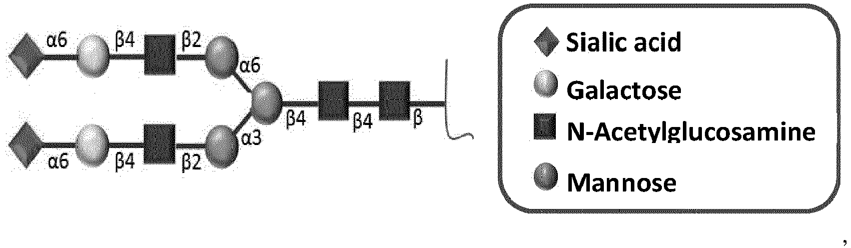 Sia₂(α2-6)Gal₂GlcNAc₂Man₃GlcNAc₂

wherein the isolated monoclonal antibody or the binding fragment thereof comprises an H-CDR1, H-CDR2, H-CDR3, L-CDR1, L-CDR2, and L-CDR3, wherein:
(i) the H-CDR1 has the sequence of SEQ ID NO: 227;
(ii) the H-CDR2 has the sequence of SEQ ID NO: 228;
(iii) the H-CDR3 has the sequence of SEQ ID NO: 229;
(iv) the L-CDR1 has the sequence of SEQ ID NO: 224;
(v) the L-CDR2 has the sequence of SEQ ID NO: 225; and
(vi) the L-CDR3 has the sequence of SEQ ID NO: 226.

In a preferred embodiment, the isolated monoclonal antibody or the binding fragment thereof comprises a VH having SEQ ID NO: 222 and a VL having SEQ ID NO: 223.

In a preferred embodiment, the Fc glycoform is attached to Asn-297 of the Fc region.

In a preferred embodiment, the antibody is a humanized antibody.

In a preferred embodiment, the isolated monoclonal antibody is a multispecific antibody that further binds to one or more of the antigens selected from the group consisting of Globo H and SSEA-3.

In some embodiments, the antibody as defined in the claims is a mouse, chimeric, humanized, and/or human MC41 antibody comprising the following sequences:

**Table 1-1. Amino acid and nucleotide sequences of anti-SSEA-4 murine, MC41.**

| SEQ ID NO | DESCRIPTIO N | SEQUENCE |
|---|---|---|
| 200 | MC41 VH nucleotide sequence | CAGGTGCAGCTGAAGGAAAGCGGACCCGGACTGGTCGC CCCCTCTAAGTCTCTGTCTATTACTTGTACTGTGAGCGG ATTCTCTCTGAGCTCCCAGGGCGTGTACTGGGTGAGGCA GCCACCTGGCAAGGGCCTGGAGTGGCTGGGAGCCATCT GGGCAGGAGGCAGCACCAACTATAATTCCGCCCTGATG |
| | | TCTCGCCTGTCTATCAGCAAGGACAACTCCAAGTCTCAG GTGTTCCTGAAGATGAACAGCCTGCAGACCGACGATAC AGCCATGTACTATTGCGCCCGGTGGACGGCTACAGAG GCTATAACATGGATTACTGGGGCCAGGGCACCAGCGTG ACAGTGTCTAGC |
| 201 | MC41 VL nucleotide sequence | GAGAATGTGCTGACACAGTCCCCAGCAATCATGAGCGC CTCCCCAGGAGAGAAGGTGACCATGACATGTTCCGCCT CCTCTAGCGTGTCTTACATGCACTGGTATCAGCAGAAGT CCTCTACCAGCCCTAAGCTGTGGATCTACGACACAAGC AAGCTGGCCTCCGGCGTGCCCGGCCGGTTTCTGGCAGC GGCTCCGGCAACTCTTATAGCCTGACCATCAGCAGCAT GGAGGCCGAGGATGTGGCCACATACTATTGCTTTCAGG GCTCTGGCTACCCACTGACATTCGGGGCTGGAACTAAA CTGGAACTGAAG CGA |
| 202 | MC41 VH amino acid sequence | QVQLKESGPGLVAPSKSLSITCTVSGFSLSSQGVYWVRQPPGKGLEWL GAIWAGGSTNYNSALMSRLSISKDNSKSQVFLKMNSLQTDDTAMYYC ARVDGYRGYNMDYWGQGTSVTVSS |
| 203 | MC41 VL amino acid sequence | ENVLTQSPAIMSASPGEKVTMTCSASSSVSYMHWYQQKSSTSPKLWIY DTSKLASGVPGRFSGSGSGNSYSLTISSMEAEDVATYYCFQGSGYPLTF GAGTKLELKR |
| 204 | MC41 VL CDR1 | SSVSY |
| 205 | MC41 VL CDR2 | DTS |
| 206 | MC41 VL CDR3 | FQGSGYPLT |
| 207 | MC41 VH CDR1 | GFSLSSQG |
| 208 | MC41 VH CDR2 | IWAGGST |
| 209 | MC41 VH CDR3 | ARVDGYRGYNMDY |

**Table 1-2. Amino acid and nucleotide sequences of 2^{nd} humanized monoclonal antibody, hMC41. 2^{nd}**

| SEQ ID NO | DESCRIPTIO N | SEQUENCE |
|---|---|---|
| 210 | MC41 VH nucleotide sequence | CAGGTGCAGCTGAAGGAGTCCGGACCAGGACTGGTGGC ACCATCTAAGACCCTGAGCCTGACCTGCACAGTGAGCG GCTTCTCCCTGAGCTCCCAGGGCGTGTACTGGATCAGGC AGCCACCTGGCAAGGGACTGGAGTGGATCGGCGCCATC TGGGCCGGCGGCTCTACAAACTATAATTCCGCCCTGATG TCTCGCCTGTCTATCAGCAAGGACAACTCCAAGTCTCAG GTGTTTCTGAAGATGAATAGCCTGCAGACCGACGATAC AGCCATGTACTATTGCGCCCGGGTGGACGGCTACAGAG GCTATAACATGGATTATTGGGGCCAGGGCACCCTGGTG ACAGTGTCTAGC |
| 211 | MC41 VL nucleotide sequence | GAGAATGTGCTGACCCAGTCTCCTGCCATCATGAGCGC CACACCAGGCGAGAAGGTGACCATGACATGTTCCGCCT CCTCTAGCGTGTCTTACCTGCACTGGTATCAGCAGAAGT CCTCTACCAGCCCCAAGCTGTGGATCTACGACACAAGC AAGCTGGCATCCGGAGTGCCTGGCCGGTTCAGCGGATC |
| | | CGGATCTGGAAACAGCTATACCCTGACAATCAGCTCCA TGGAGGCCGAGGATGTGGCCACCTACTATTGTTTCCAG GGATCCGGATACCCACTGACCTTTGGCGCCGGCACAAA GCTGGAGATCAAGCGT |
| 212 | MC41 VH amino acid sequence | QVQLKESGPGLVAPSKTLSLTCTVSGFSLSSQGVYWIRQPPGKGLEWI GAIWAGGSTNYNSALMSRLSISKDNSKSQVFLKMNSLQTDDTAMYYC ARVDGYRGYNMDYWGQGTLVTVSS |
| 213 | MC41 VL amino acid sequence | ENVLTQSPAIMSATPGEKVTMTCSASSSVSYLHWYQQKSSTSPKLWIY DTSKLASGVPGRFSGSGSGNSYTLTISSMEAEDVATYYCFQGSGYPLTF GAGTKLEIKR |
| 214 | MC41 VL CDR1 | SSVSY |
| 215 | MC41 VL CDR2 | DTS |
| 216 | MC41 VL CDR3 | FQGSGYPLT |
| 217 | MC41 VH CDR1 | GFSLSSQG |
| 218 | MC41 VH CDR2 | IWAGGST |
| 219 | MC41 VH CDR3 | ARVDGYRGYNMDY |

**Table 1-3. Amino acid and nucleotide sequences of 3^{rd} humanized monoclonal antibody, hMC41. 3^{rd}**

| SEQ ID NO | DESCRIPTIO N | SEQUENCE |
|---|---|---|
| 220 | MC41 VH nucleotide sequence | CAGGTGCAGCTGAAGGAGTCCGGACCAGGACTGGTGGC ACCATCTAAGACCCTGAGCCTGACCTGCACAGTGAGCG GCTTCTCCCTGAGCTCCCAGGGCGTGTACTGGATCAGGC AGCCACCTGGCAAGGGACTGGAGTGGATCGGCGCCATC TGGGCCGGCGGCTCTACAAACTATAATTCCGCCCTGATG TCTCGCCTGTCTATCAGCAAGGACAACTCCAAGTCTCAG GTGTTTCTGAAGATGAATAGCCTGCAGACCGACGATAC AGCCATGTACTATTGCGCCCGGGTGGACGGCTACAGAG GCTATAACATGGATTATTGGGGCCAGGGCACCtcGGTGA CAGTGTCTAGC |
| 221 | MC41 VL nucleotide sequence | GAGAATGTGCTGACCCAGTCTCCTGCCATCATGAGCGC CACACCAGGCGAGAAGGTGACCATGACATGTTCCGCCT CCTCTAGCGTGTCTTACATGCACTGGTATCAGCAGAAGT CCTCTACCAGCCCCAAGCTGTGGATCTACGACACAAGC AAGCTGGCATCCGGAGTGCCTGGCCGGTTCAGCGGATC CGGATCTGGAAACAGCTATACCCTGACAATCAGCTCCA TGGAGGCCGAGGATGTGGCCACCTACTATTGTTTCCAG GGATCCGGATACCCACTGACCTTTGGCGCCGGCACAAA GCTGGAGATCAAGCGT |
| 222 | MC41 VH amino acid sequence | QVQLKESGPGLVAPSKTLSLTCTVSGFSLSSQGVYWIRQPPGKGLEWI GAIWAGGSTNYNSALMSRLSISKDNSKSQVFLKMNSLQTDDTAMYYC ARVDGYRGYNMDYWGQGTSVTVSS |
| 223 | MC41 VL amino acid | ENVLTQSPAIMSATPGEKVTMTCSASSSVSYMHWYQQKSSTSPKLWIY DTSKLASGVPGRFSGSGSGNSYTLTISSMEAEDVATYYCFQGSGYPLTF GAGTKLEIKR |
| | sequence | |
| 224 | MC41 VL CDR1 | SSVSY |
| 225 | MC41 VL CDR2 | DTS |
| 226 | MC41 VL CDR3 | FQGSGYPLT |
| 227 | MC41 VH CDR1 | GFSLSSQG |
| 228 | MC41 VH CDR2 | IWAGGST |
| 229 | MC41 VH CDR3 | ARVDGYRGYNMDY |

In one aspect, the present invention provides an isolated monoclonal antibody (MC48) or a binding fragment thereof that binds to Neu5Acα2→ 3Galβ1→ 3GalNAcβ1 → 3Galα1→ 4Galβ1→ 4Glcβ1(SSEA-4 hexasaccharide) wherein the isolated monoclonal antibody or the binding fragment thereof is a defucosylated antibody comprising a Fc glycoform attached to the Fc region, wherein said glycoform has the formula:

Sia₂(α2-6)Gal₂GlcNAc₂Man₃GlcNAc₂

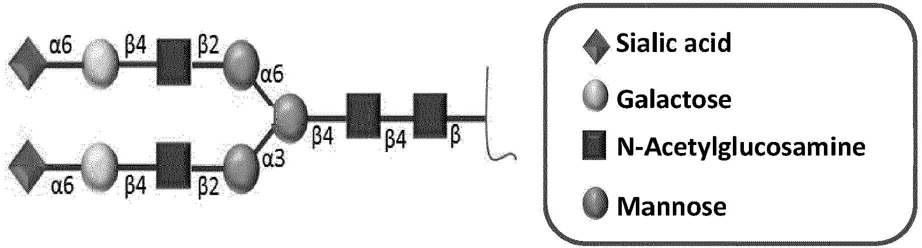

wherein the antibody comprises a VH having SEQ ID NO: 147 and a VL having SEQ ID NO: 148.

In a preferred embodiment the antibody is an IgG1.

In a preferred embodiment, the Fc glycoform is attached to Asn-297 of the Fc region.

In one embodiment, the antibody is a human antibody.

In one embodiment, the antibody is a humanized antibody.

In one embodiment, the antigen binding fragment is a Fab fragment, a F(ab')2 fragment, or a single-chain Fv fragment.

In a preferred embodiment, the isolated monoclonal antibody is a multispecific antibody that further binds to one or more of the antigens selected from the group consisting of Globo H and SSEA-3. Amino acid and nucleic acid structures/sequences of anti-SSEA4 antibodies disclosed herein are provided below:

**Table 17-0. Amino Acid and Nucleotide Sequences of Mouse Monoclonal Antibody MC48.**

| SEQ ID NO | DESCRIPTIO N | SEQUENCE |
|---|---|---|
| 41 | MC48 VH nucleotide sequence | CAGGTGCAGCTGAAGGAGTCAGGACCTGGCCTGGTGGC GCCCTCACAGAGCCTGTCCATCACATGCACTGTCTCAGG GTTCTCATTAACCAGCTATGGTGTAAGCTGGGTTCGCCA GCCTCCAGGAAAGGGTCTGGAGTGGCTGGGAGTAATAT GGGGTGAGGGGAGCACAAATTATCATTCAGTTCTCATA TCCAGACTGACCATTAGTAAGGATAACTCCAAGAGCCA AGTTTTCTTAAAACTGAACAGTCTGCAAACTGATGACAC AGCCACGTACTACTGTGCCATGACTGGGACAGCTTACT GGGGCCAAGGGACTCTGGTCACTGTCTCTGCA |
| 42 | MC48 VL nucleotide | CAAATTGTTCTCACCCAGTCTCCAGCAATCATGTCTGCA TCTCCAGGGGAGAAGGTCACCATGACCTGCAGTGCCAG CTCAAGTGTAAGTTACATGCACTGGTACCAGCAGAAGT |
| | sequence | CAGGCACCTCCCCCAAAAGATGGATTTATGACACATCC AAACTGTCTTCTGGAGTCCCTGGTCGCTTCAGTGGCAGT GGGTCTGGGACCTCTTACTCTCTCACAATCAGCAGGTTG GAGGCTGAAGATGCTGCCACTTATTACTGCCATCAGTG GAGTAGTAGTCCACACACGTTCGGAGGGGGGACCAAGT TGGAGATAAAA |
| 43 | MC48 VH amino acid sequence | QVQLKESGPGLVAPSQSLSITCTVSGFSLTSYGVSWVRQPPGKGLEWL GVIWGEGSTNYHSVLISRLTISKDNSKSQVFLKLNSLQTDDTATYYCA MTGTAYWGQGTLVTVSA |
| 44 | MC48 VL amino acid sequence | QIVLTQSPAIMSASPGEKVTMTCSASSSVSYMHWYQQKSGTSPKRWIY DTSKLSSGVPGRFSGSGSGTSYSLTISRLEAEDAATYYCHQWSSSPHTF GGGTKLEIK |
| 45 | MC48 VL CDR1 | SSVSY |
| 46 | MC48 VL CDR2 | DTS |
| 47 | MC48 VL CDR3 | HQWSSSPHT |
| 48 | MC48 VH CDR1 | GFSLTSYG |
| 49 | MC48 VH CDR2 | IWGEGST |
| 50 | MC48 VH CDR3 | AMTGTAY |

**Table 17-1 Amino Acid and Nucleotide Sequences of Humanized Monoclonal Antibody MC48 (1^{st})**

| SEQ ID NO | DESCRIPTION | SEQUENCE |
|---|---|---|
| 115 | hMC48 VH nucleotide sequence | CAGGTGCAGCTGCAAGAGTCAGGACCTGGCCTGGTGAA ACCCTCAGAAACTCTGTCCCTTACATGCACTGTCTCAGG GTTCTCATTAACCAGCTATGGTGTAAGCTGGATTCGCCA GCCTCCAGGAAAGGGTCTGGAGTGGATTGGAGTAATAT GGGGTGAGGGGAGCACAAATTATCATTCAGTTCTCATA TCCAGACTGACCATTAGTGTGGATACCTCCAAGAATCA ATTTAGCTTAAAACTGAGCAGTGTTACCGCTGCTGACAC AGCCGTTTACTACTGTGCCATGACTGGGACAGCTTACTG GGGCCAAGGGACTCTGGTCACTGTCTCTAGC |
| 116 | hMC48 VL nucleotide sequence | GAGATTGTGCTGACCCAGAGCCCTGCCACACTGTCACT GAGCCCAGGCGAGCGAGCCACACTGTCCTGTTCTGCTA GCTCCTCTGTCTCCTACATGCATTGGTATCAGCAGAAGC CAGGACTGGCACCACGACTGCTGATCTATGACACTTCTA AACTGAGTTCAGGCATTCCCGCCAGATTCAGTGGCTCA GGGAGCGGAACCGACTTTACTCTGACCATTAGCTCCCTG GAGCCTGAAGATTTCGCCGTGTACTATTGCCATCAGTGG TCATCAAGCCCTCATACCTTCGGGGGGGGGACTAAGGT GGAAATCAAACGC |
| 117 | hMC48 VH amino acid sequence | QVQLQESGPGLVKPSETLSLTCTVSGFSLTSYGVSWIRQPP GKGLEWIGVIWGEGSTNYHSVLISRLTISVDTSKNQFSLKL SSVTAADTAVYYCAMTGTAYWGQGTLVTVSS |
| 118 | hMC48 VL amino acid sequence | EIVLTQSPATLSLSPGERATLSCSASSSVSYMHWYQQKPG LAPRLLIYDTSKLSSGIPARFSGSGSGTDFTLTISSLEPEDF AVYYCHQWSSSPHTFGGGTKVEIKR |
| 119 | hMC48 VL | SSVSY |
| | CDR1 | |
| 120 | hMC48 VL CDR2 | DTS |
| 121 | hMC48 VL CDR3 | HQWSSSPHT |
| 122 | hMC48 VH CDR1 | GFSLTSYG |
| 123 | hMC48 VH CDR2 | IWGEGST |
| 124 | hMC48 VH CDR3 | AMTGT A Y |

**Table 17-2. Amino Acid and Nucleotide Sequences of Humanized Monoclonal Antibody MC48 (2^{nd})**

| SEQ ID NO | DESCRIPTIO N | SEQUENCE |
|---|---|---|
| 125 | hMC48 VH nucleotide sequence | CAGGTGCAGCTGAAGCAGAGCGGACCTGGCCTGGTGCA GCCCTCACAGAGCCTGAGCATCACTTGTACCGTCAGTG GATTCTCCCTGACATCTTACGGCGTGTCTTGGGTCAGGC AGAGCCCTGGCAAGGGGCTGGAGTGGCTGGGCGTGATC TGGGGAGAAGGCTCAACTAACTATCACAGCGTCCTGAT CAGTCGCCTGTCAATTAACAAGGACAATTCTAAAAGTC AGGTGTTCTTTAAAATGAACAGCCTGCAGTCCAATGAT ACCGCCATCTACTATTGCGCTATGACCGGCACAGCATAC TGGGGGCAGGGAACACTGGTGACTGTCTCCGCT |
| 126 | hMC48 VL nucleotide | GAGATTGTGCTGACCCAGAGCCCTGCCACACTGTCACT GAGCCCAGGCGAGCGAGCCACACTGTCCTGTTCTGCTA |
| | sequence | GCTCCTCTGTCTCCTACATGCATTGGTATCAGCAGAAGC CAGGACTGGCACCACGACTGCTGATCTATGACACTTCTA AACTGAGTTCAGGCATTCCCGCCAGATTCAGTGGCTCA GGGAGCGGAACCGACTTTACTCTGACCATTAGCTCCCTG GAGCCTGAAGATTTCGCCGTGTACTATTGCCATCAGTGG TCATCAAGCCCTCATACCTTCGGGGGGGGACTAAGCT GGAAATCAAACGC |
| 127 | hMC48 VH amino acid sequence | QVQLKQSGPGLVQPSQSLSITCTVSGFSLTSYGVSWVRQSPGKGLEWL GVIWGEGSTNYHSVLISRLSINKDNSKSQVFFKMNSLQSNDTAIYYCA MTGTAYWGQGTLVTVSA |
| 128 | hMC48 VL amino acid sequence | EIVLTQSPATLSLSPGERATLSCSASSSVSYMHWYQQKPGLAPRLLIY DTSKLSSGIPARFSGSGSGTDFTLTISSLEPEDFAVYYCHQWSSSPHTFG GGTKVLEIKR |
| 129 | hMC48 VL CDR1 | SSVSY |
| 130 | hMC48 VL CDR2 | DTS |
| 131 | hMC48 VL CDR3 | HQWSSSPHT |
| 132 | hMC48 VH CDR1 | GFSLTSYG |
| 133 | hMC48 VH CDR2 | IWGEGST |
| 134 | hMC48 VH CDR3 | AMTGT A Y |

**Table 17-3. Amino Acid and Nucleotide Sequences of Humanized Monoclonal Antibody MC48 (3^{rd})**

| SEQ ID NO | DESCRIPTIO N | SEQUENCE |
|---|---|---|
| 135 | hMC48 VH nucleotide sequence | CAGGTGCAGCTGCAGGAAAGCGGACCCGGACTGGTGAA ACCTAGCGAAACACTGAGCCTGACTTGTACCGTGAGCG GATTTTCCCTGACCTCTTATGGAGTGAGCTGGATCAGAC AGCCCCCTGGCAAGGGACTGGAGTGGATCGGCGTGATT TGGGGAGAAGGCTCCACAAACTATCACAGTGTCCTGAT CTCACGACTGACTATTTCTAAGGACAACTCTAAAAGTCA GGTCTTCCTGAAACTGAATAGTCTGCAGACTGACGATA CCGCTACATACTATTGCGCAATGACAGGGACAGCATAC TGGGGACAGGGAACCCTGGTGACAGTCAGCTCC |
| 136 | hMC48 VL nucleotide sequence | CAGATCGTGCTGACACAGTCCCCTGCAATTATGTCAGCC AGCCCAGGGGAAAAGGTGACAATGACTTGTAGTGCTTC TAGTTCAGTCTCATACATGCATTGGTATCAGCAGAAGCC AGGCCTGGCCCCCAGACTGCTGATCTACGACACCTCCA AACTGAGCTCCGGCGTGCCCGGGAGATTTCCGGCTCTG GGAGTGGAACTTCATATAGCCTGACCATTTCTAGGCTGG AGGCCGAAGATGCCGCTACATACTATTGCCACCAGTGG AGCAGTAGCCCCCATACATTCGGAGGCGGGACCAAAGT GGAAATCAAACGC |
| 137 | hMC48 VH amino acid sequence | QVQLQESGPGLVKPSETLSLTCTVSGFSLTSYGVSWIRQPPGKGLEWI GVIWGEGSTNYHSVLISRLTISKDNSKSQVFLKLNSLQTDDTATYYCA MTGTAYWGQGTLVTVSS |
| 138 | hMC48 VL amino acid sequence | QIVLTQSPAIMSASPGEKVTMTCSASSSVSYMHWYQQKPGLAPRLLIY DTSKLSSGVPGRFSGSGSGTSYSLTISRLEAEDAATYYCHQWSSSPHTF GGGTKVEIKR |
| | | |
| 139 | hMC48 VLCDR1 | SSVSY |
| 140 | hMC48 VL CDR2 | DTS |
| 141 | hMC48 VL CDR3 | HQWSSSPHT |
| 142 | hMC48 VH CDR1 | GFSLTSYG |
| 143 | hMC48 VH CDR2 | IWGEGST |
| 144 | hMC48 VH CDR3 | AMTGT A Y |

**Table 17-4. Amino Acid and Nucleotide Sequences of Humanized Monoclonal Antibody MC48 (4^{th})**

| SEQ ID NO | DESCRIPTIO N | SEQUENCE |
|---|---|---|
| 145 | hMC48 VH nucleotide sequence | CAGGTCCAGCTGAAAGAGAGCGGCCCCGGACTGGTCGC CCCTTCACAGAGCCTGAGCATTACTTGCACCGTGAGCG GATTTTCACTGACCAGCTACGGAGTGAGCTGGATTAGA CAGCCTCCTGGCAAGGGACTGGAGTGGATCGGCGTGAT TTGGGGAGAAGGCAGCACCAACTATCACAGTGTCCTGA TCTCACGCCTGACAATTTCCAAGGACAACAGCAAATCC CAGGTCTTCCTGAAACTGAATTCTCTGCAGACTGACGAT ACCGCTACATACTATTGCGCAATGACAGGGACAGCATA |
| | | CTGGGGACAGGGAACCCTGGTGACAGTCAGTAGT |
| 146 | hMC48 VL nucleotide sequence | CAGATCGTGCTGACACAGTCCCCAGCAATTATGTCTGCC AGTCCCGGGGAGAAGGTGACAATGACTTGTAGTGCCAG CTCCTCTGTCTCATACATGCATTGGTATCAGCAGAAGTC CGGCACATCTCCTAAACGGTGGATCTACGACACTTCTAA ACTGAGTTCAGGCGTGCCCGGGAGATTTTCAGGCAGCG GGTCCGGAACTTCTTATAGTCTGACCATTTCCCGACTGG AGGCCGAAGATGCCGCTACCTACTATTGCCATCAGTGG TCTTCAAGCCCTCATACTTTTGGGGGGGGAACTAAGGTG GAAATCAAGCGA |
| 147 | hMC48 VH amino acid sequence | QVQLKESGPGLVAPSQSLSITCTVSGFSLTSYGVSWIRQPPGKGLEWIG VIWGEGSTNYHSVLISRLTISKDNSKSQVFLKLNSLQTDDTATYYCAMT GTAYWGQGTLVTVSS |
| 148 | hMC48 VL amino acid sequence | QIVLTQSPAIMSASPGEKVTMTCSASSSVSYMHWYQQKSGTSPKRWIY DTSKLSSGVPGRFSGSGSGTSYSLTISRLEAEDAATYYCHQWSSSPHTF GGGTKVEIKR |
| 149 | hMC48 VL CDR1 | SSVSY |
| 150 | hMC48 VL CDR2 | DTS |
| 151 | hMC48 VL CDR3 | HQWSSSPHT |
| 152 | hMC48 VH CDR1 | GFSLTSYG |
| 153 | hMC48 VH | IWGEGST |
| | CDR2 | |
| 154 | hMC48 VH CDR3 | AMTGT A Y |
| 155 | hMC48 VL FR1 | QIVLTQSPAIMSASPGEKVTMTCSAS |
| 156 | hMC48 VL FR2 | MHWYQQKSGTSPKRWIY |
| 157 | hMC48 VL FR3 | KLSSGVPGRFSGSGSGTSYSLTISRLEAEDAATYYC |
| 158 | hMC48 VL FR4 | FGGGTKVEIKR |
| 159 | hMC48 VH FR1 | QVQLKESGPGLVAPSQSLSITCTVS |
| 160 | hMC48 VH FR2 | VSWIRQPPGKGLEWIGV |
| 161 | hMC48 VH FR3 | NYHSVLISRLTISKDNSKSQVFLKLNSLQTDDTATYYC |
| 162 | hMC48 VH FR4 | WGQGTLVTVSS |

In a further aspect, the present invention relates to a pharmaceutical composition as recited in the claims, comprising the isolated monoclonal antibody or a binding fragment thereof and a pharmaceutically acceptable carrier.

In a preferred embodiment, the composition is for use in the treatment against a hyperproliferative disease.

In another aspect, the presen invention relates to the isolated monoclonal antibody or a binding fragment thereof as recited in the claims for use in a method of treating cancer in a subject in need thereof, wherein the isolated monoclonal antibody or a binding fragment thereof enhances ADCC activity in said subject.

In a preferred embodiment, the cancer is selected from the group consisting of brain cancer, lung cancer, breast cancer, oral cancer, esophageal cancer, stomach cancer, liver cancer, bile duct cancer, pancreatic cancer, colon cancer, kidney cancer, bone cancer, skin cancer, cervical cancer, ovarian cancer, and prostate cancer.

In a preferred embodiment, the monoclonal antibody or the binding fragment thereof is administered with at least one other chemotherapeutic agent.

The treatment results in reduction of tumor size, elimination of malignant cells, prevention of metastasis, prevention of relapse, reduction or killing of disseminated cancer, prolongation of survival and/or prolongation of time to tumor cancer progression.

In some embodiments, the composition described herein is formulated an injectible. In some embodiments, the composition is administered subcutaneously.

In another aspect, the present invention relates to making the isolated monoclonal antibody or the binding fragment thereof as recited in the claims, comprising:
(a) contacting an isolated monoclonal antibody or a binding fragment thereof with an α-fucosidase and at least one endoglycosidase, wherein the isolated monoclonal antibody or the binding fragment thereof comprises VH having SEQ ID NO: 147 and VL having SEQ ID NO: 148, or H-CDR1, H-CDR2, H-CDR3, L-CDR1, L-CDR2, and L-CDR3 as defined above;
(b) generating a defucosylated antibody having a single N-acetylglucosamine (GlcNAc); and (c) adding a glycan to GlcNAc of Fc region of antibody to form the isolated monoclonal antibody or the binding fragment thereof with said glycoform as defined above.

The details of certain embodiments of the invention are set forth herein. Other features, objects, and advantages of the invention will be apparent from the Detailed Description, the Figures, the Examples, and the Claims.

### BRIEF DESCRIPTION OF THE DRAWINGS

**Figure 1****.** Structure of optimized universal Fc glycan of therapeutic antibodies.
**Figure 2****.** General strategy for the preparation of homogeneous antibody with optimized universal glycan at the Fc region for the improvement of its therapeutic activity.
**Figure 3****.** Demonstrates the enhanced anti-viral antibody-dependent cell-mediated cytotoxicity (ADCC) results of anti-influenza virus antibodies.
**Figure 4****.** Table listing exemplary enhanced ADCC activities of anti-CD20 GAbs as compared to Rituximab.
**Figure 5****.** Six anti-CD20 GAbs
**Figure 6A** **and** **6B****.** Figure 6A is top of table, figure 6B is bottom of table. Table lists exemplary FcyRIIIA binding of anti-CD20 GAbs and Rituximab. FcyRIIIA binding may be measured using assays known in the art. Exemplary assays are described in the examples. The Fc receptor binding may be determined as the relative ratio of anti-CD20 GAb vs Rituximab. Fc receptor binding in exemplary embodiments is increased by at least 1.2-fold, 2-fold, 3-fold, 4-fold, 5-fold, 6-fold, 7-fold, 8-fold, 9-fold, 10-fold, 15-fold or 20-fold, 30-fold, 40-fold, 50-fold, 100-fold or higher.
**Figure 7****.** Binding activities of different homogeneous antibodies with different cells with CD20. Figure 7 shows CDC effects of Rituxan-SCT (Gab101) and Rituxan mono-GlcNAc to Ramos cells.
**Figure 8****.** Binding activities of different homogeneous antibodies with different cells with CD20. Figure 8 shows CDC effects of Rituxan-SCT (Gab101) and Rituxan mono-GlcNAc to Raji cells.
**Figure 9****.** Binding activities of different homogeneous antibodies with different cells with CD20. Figure 9 shows CDC effects of Rituxan-SCT (Gab101) and Rituxan mono-GlcNAc to SU-DHL-4 cells.
**Figure 10****.** Depletion of human SU-DHL-4 B cells as analyzed on FACS. Cells were cultured in the absence or presence of 15% autologous plasma with anti-CD20 Gabs Rituxan-SCT, Rituxan-GlcNAc and Rituximab at different concentrations. After wash cells were stained with anti-CD2-PE and anti-CD 19-FITC. B cell depletion was analyzed on FACS, based on the CD19+ CD2- B cells (Figure 13).
**Figure 11****.** Depletion of human Ramos B cells as analyzed on FACS. Cells were cultured in the absence or presence of 15% autologous plasma with anti-CD20 Gabs Rituxan-SCT, Rituxan-GlcNAc and Rituximab at different concentrations. After wash cells were stained with anti-CD2-PE and anti-CD19-FITC. B cell depletion was analyzed on FACS, based on the CD19+ CD2- B cells (Figure 13).
**Figure 12****.** Depletion of human Raji B cells as analyzed on FACS. Cells were cultured in the absence or presence of 15% autologous plasma with anti-CD20 Gabs Rituxan-SCT, Rituxan-GlcNAc and Rituximab at different concentrations. After wash cells were stained with anti-CD2-PE and anti-CD19-FITC. B cell depletion was analyzed on FACS, based on the CD19+ CD2- B cells (Figure 13).
**Figure 13****.** Depletion of human B cells by different homogeneous antibodies.
**Figure 14****.** Table listing exemplary enhanced ADCC activities of anti-HER2 GAbs as compared to Trastuzumab.
**Figure 15****.** Table listing exemplary FcyRIIIA binding of anti-HER2 GAbs and Rituximab.
**Figure 16****.** Solid-based ELISA coating SSEA-4 to determine the binding activity of humanized MC41 phage clones
**Figure 16B****.** Solid-based ELISA coating BSA to determine the binding activity of humanized MC41 phage clones
**Figure 17A****.** To evaluate the binding activity by intact humanized MC41 IgG, intact IgGs of 1st, 2nd, 3rd humanized MC41, and chimeric MC41 (chMC41) are contructed. The ELISA results show that the humanized 2nd and 3rd MC41 could react to SSEA-4 (Fig. 17A) but not to BSA (Fig. 17B) in a dose-dependent pattern, same results were observed for chMC41.
**Figure 17B****.** To evaluate the binding activity by intact humanized MC41 IgG, intact IgGs of 1st, 2nd, 3rd humanized MC41, and chimeric MC41 (chMC41) are contructed. The ELISA results show that the humanized 2nd and 3rd MC41 could react to SSEA-4 (Fig. 17A) but not to BSA (Fig. 17B) in a dose-dependent pattern, same results were observed for chMC41.
**Figure 18A** **and** **Figure 18B****.** Figure 18A shows the legend for bar graph of Figure 18B. In order to determine the binding specificity of chMC41 and hMC41, glycan array is performed. Results are shown in Figure 18B. The chimeric and humanized MC41 show more specific binding than commercial SSEA4 antibody (MC813). They only recognized SSEA4 or glycolyl modified SSEA4.

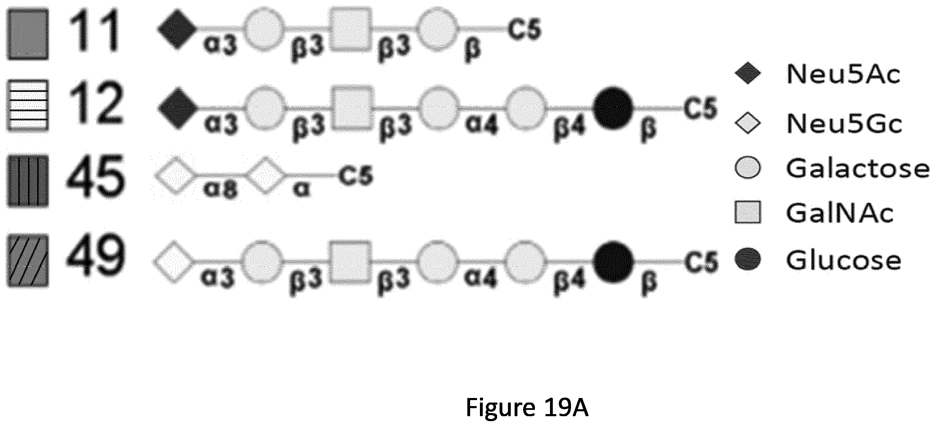

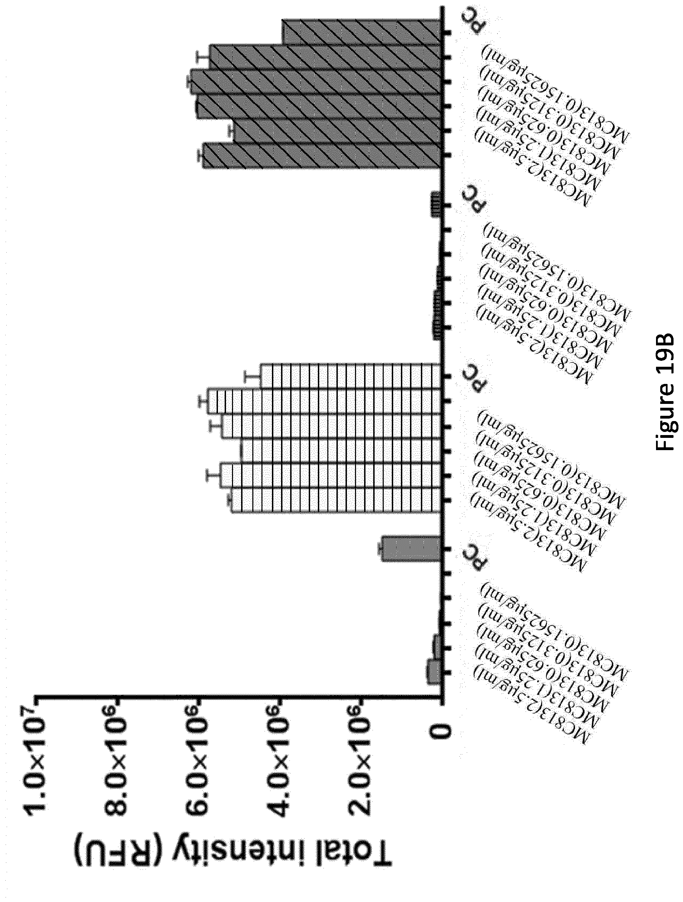

**Figure 19A** **and** **19B****.** Figure 19A shows the legend for the bar graph of Figure 19B. In order to determine the binding specificity of chMC41 and hMC41, glycan array is performed. Results are shown in Figure 19B. The chimeric and humanized MC41 show more specific binding than commercial SSEA4 antibody (MC813). They only recognized SSEA4 or glycolyl modified SSEA4.

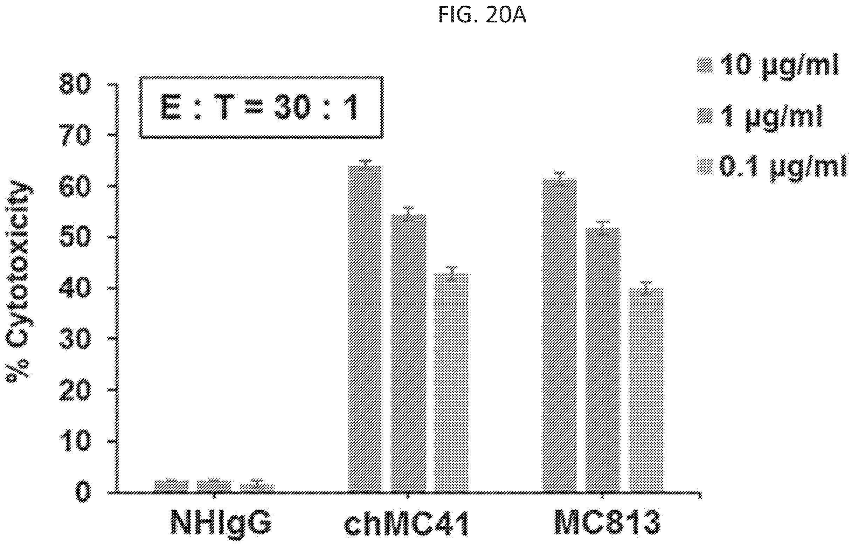

**Figure 20A****.** To investigate the effector function of chMC41 and hMC41, ADCC and CDC assays were performed. HPAC pancreatic cancer cell line was used to evaluate the ADCC and CDC activities of chMC41, hMC41, positive control MC813 or negative controls NHIgG and NMIgG.

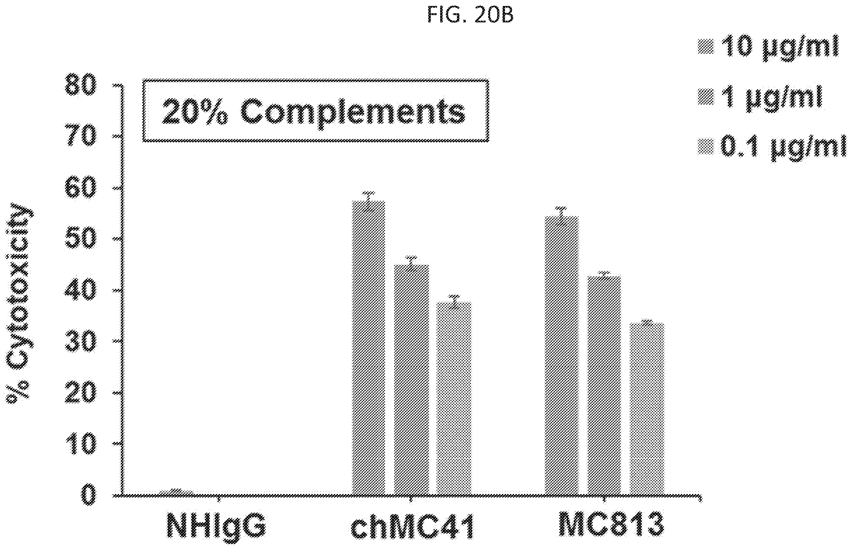

**Figure 20B****.** To investigate the effector function of chMC41 and hMC41, ADCC and CDC assays were performed. HPAC pancreatic cancer cell line was used to evaluate the ADCC and CDC activities of chMC41, hMC41, positive control MC813 or negative controls NHIgG and NMIgG.

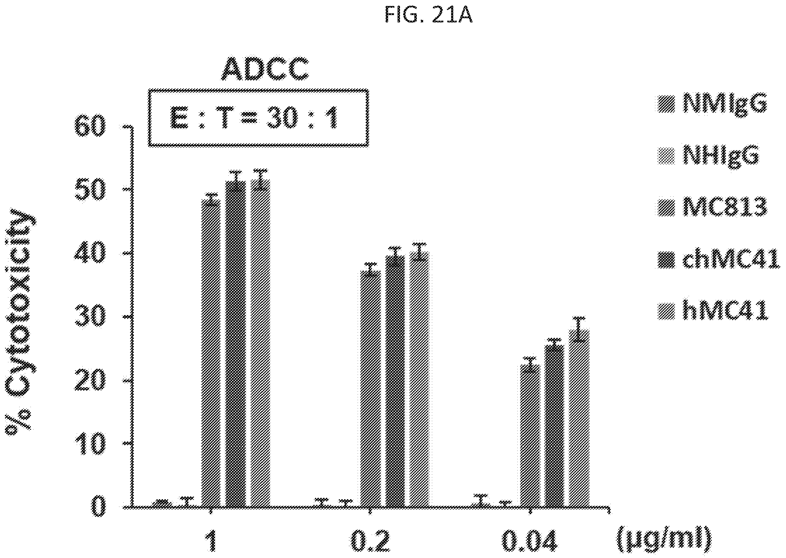

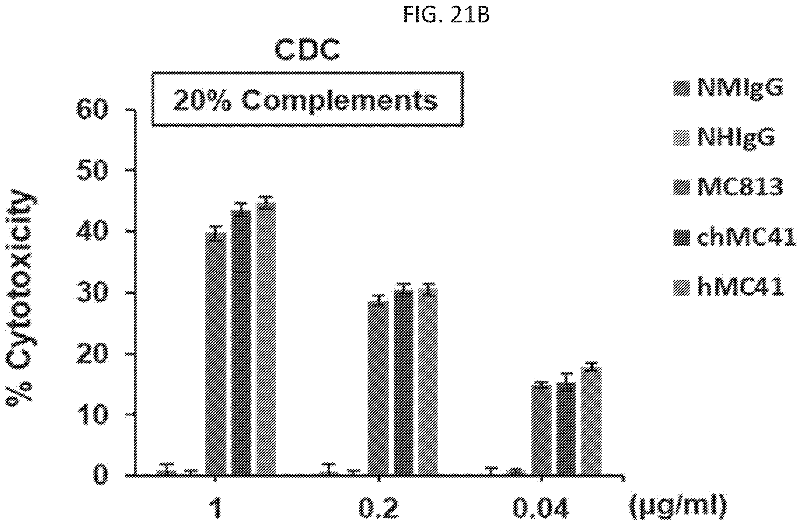

**Figure 21A** **and** **Figure 21B****.** To investigate the effector function of chMC41 and hMC41, ADCC and CDC assays were performed. HPAC pancreatic cancer cell line was used to evaluate the ADCC and CDC activities of chMC41, hMC41, positive control MC813 or negative controls NHIgG and NMIgG. Figure 21A shows cancer cell killing activity through ADCC. Figure 21B shows cancer cell killing activity through CDC.

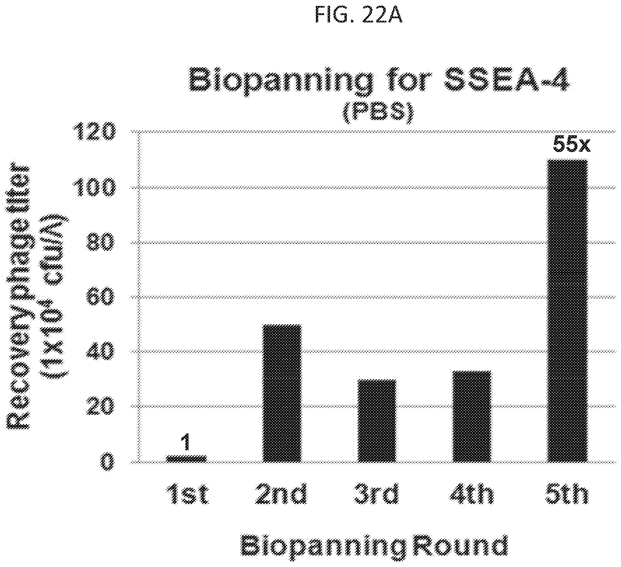

**Figure 22A****.** To identify the antibodies that bind to SSEA-4, we used phage-displayed human naive scFv library containing 2×1010 members, which was established as described in our previous report (Lu et al., 2011). This library was first removed by Dynabeads-binding phages, and then SSEA-4-binding phages were selected by SSEA-4-PEG-conjugated Dynabeads. We used two buffer systems, PBS and PBS containing 0.01% Tween20 (PBST0.01), during biopanning. After five rounds of affinity selection, the phage recovery of the fifth round increased by about 55-fold and 80-fold, compared to that of the first round in PBS and PBST0.01 system, respectively.

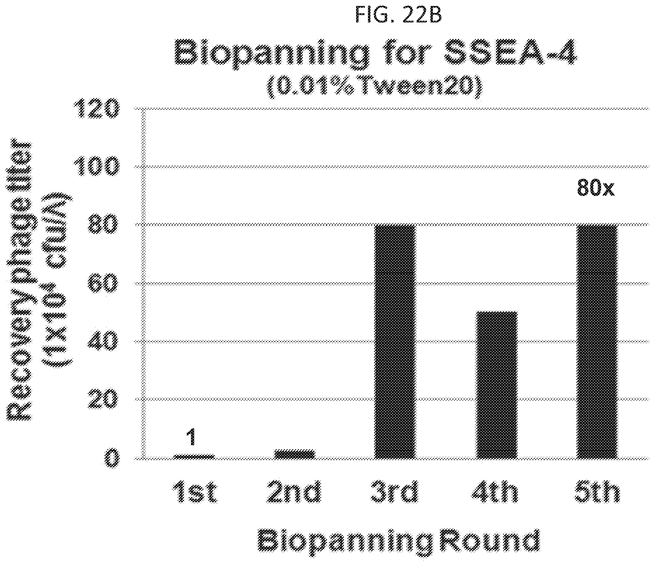

**Figure 22B****.** To identify the antibodies that bind to SSEA-4, we used phage-displayed human naive scFv library containing 2×1010 members, which was established as described in our previous report (Lu et al., 2011). This library was first removed by Dynabeads-binding phages, and then SSEA-4-binding phages were selected by SSEA-4-PEG-conjugated Dynabeads. We used two buffer systems, PBS and PBS containing 0.01% Tween20 (PBST0.01), during biopanning. After five rounds of affinity selection, the phage recovery of the fifth round increased by about 55-fold and 80-fold, compared to that of the first round in PBS and PBST0.01 system, respectively.

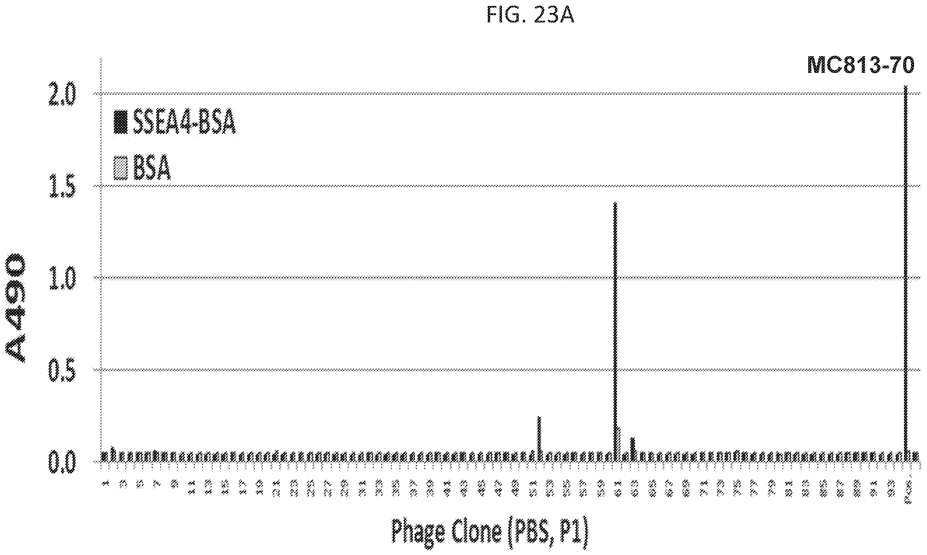

**Figure 23A****.** The phage clones were randomly selected and tested for SSEA-4 binding by ELISA

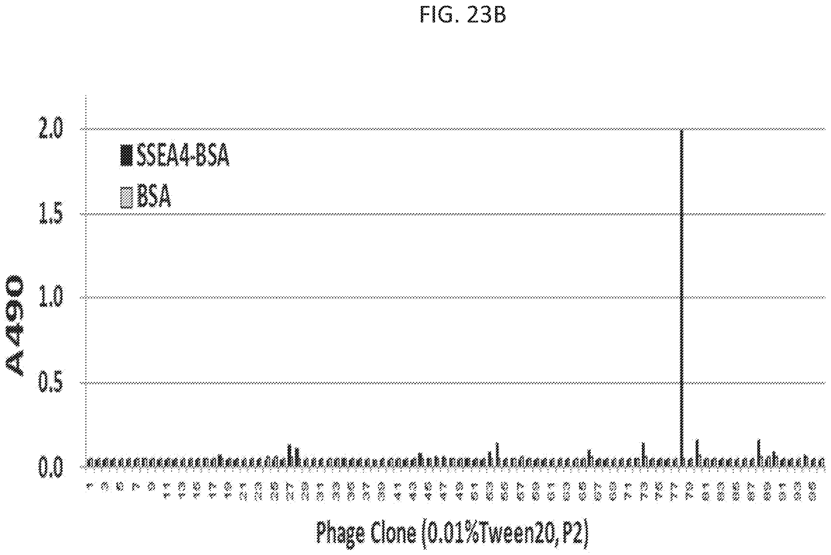

**Figure 23B****.** The phage clones were randomly selected and tested for SSEA-4 binding by ELISA

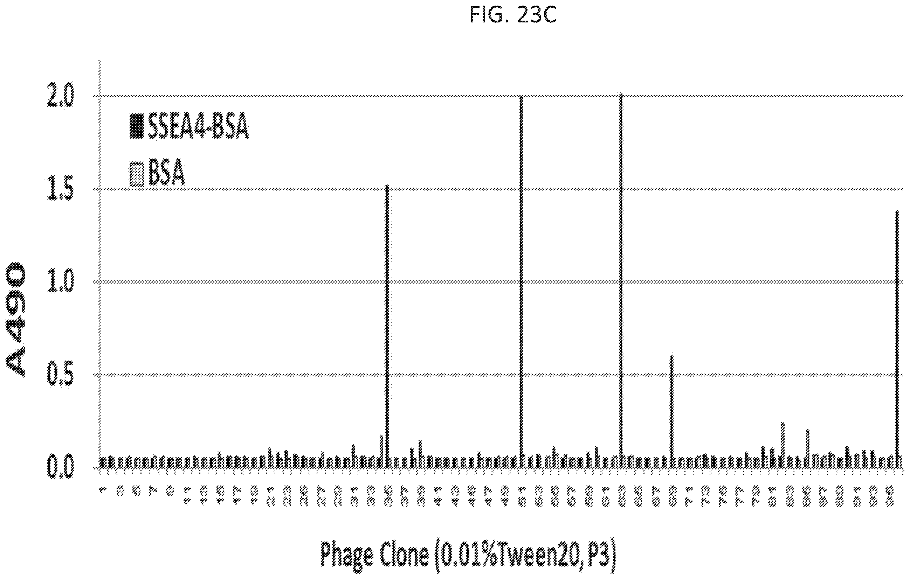

**Figure 23C****.** The phage clones were randomly selected and tested for SSEA-4 binding by ELISA

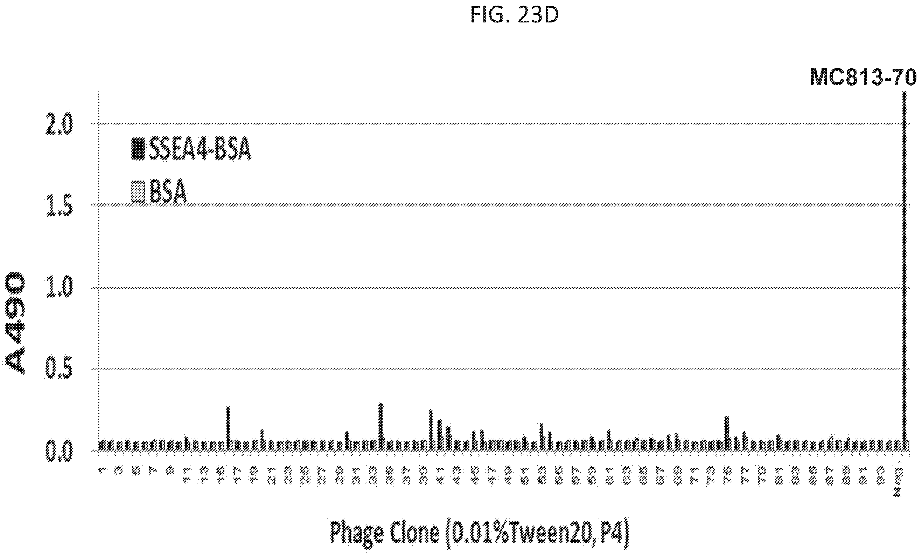

**Figure 23D****.** The phage clones were randomly selected and tested for SSEA-4 binding by ELISA

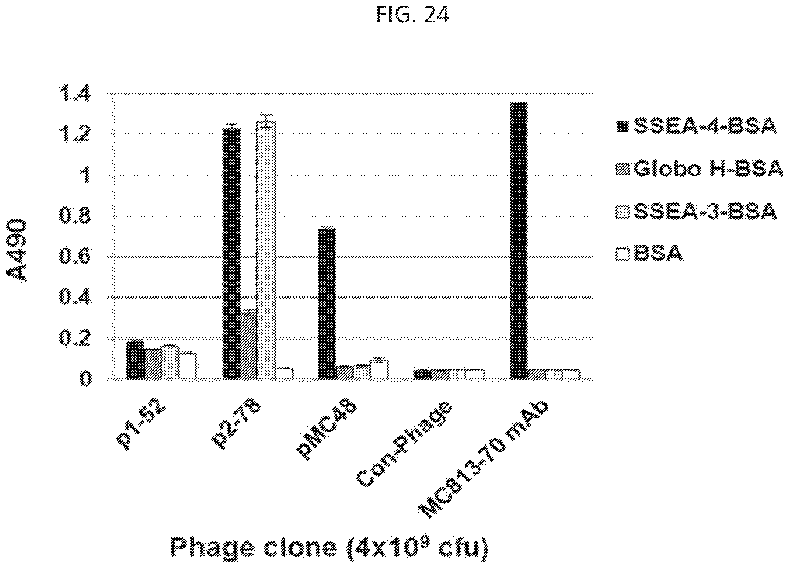

**Figure 24****.** To examine the specificity and binding affinity of the two phage clones, we performed a comparative ELISA using the same phage titer to Globo-series glycans including SSEA-4-BSA, Globo H-BSA and SSEA-3-BSA.

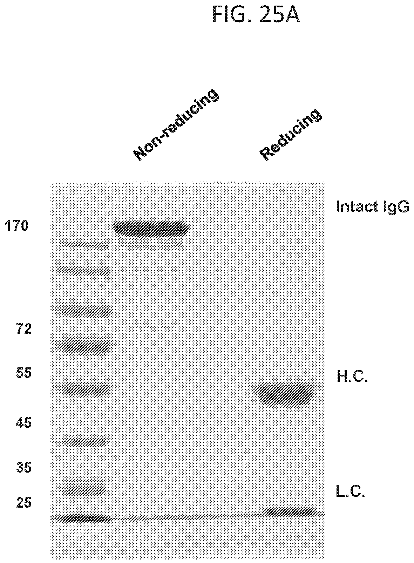

**Figure 25A****.** To establish the fully human antibody (hAb) against SSEA-4, we molecularly engineered the VH and VL coding sequences of p2-78 scFv into human IgG1 backbone, respectively. The anti-SSEA-4 p2-78 hAb was produced using FreeStyle 293 expression system and then purified through the protein G sepharose column. We examined the purity of antibody by SDS-PAGE analysis with coomassie blue staining

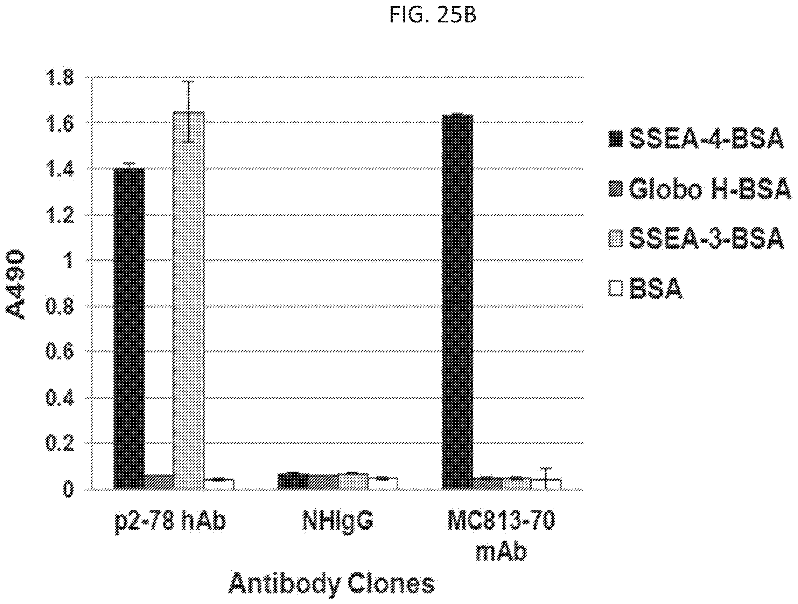

**Figure 25B****.** ELISA to investigate the binding activity of p2-78 hAb for Globo-series glycans.

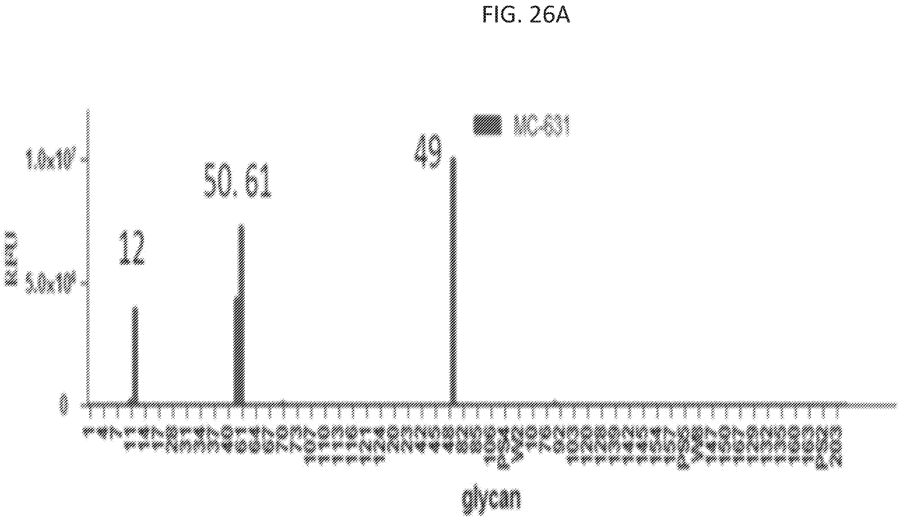

**Figure 26A****.** Positive control of commercially available IgM antibody, MC631. Glycan array containing 203 different glycans to further confirm the specificity of p2-78 hAb.

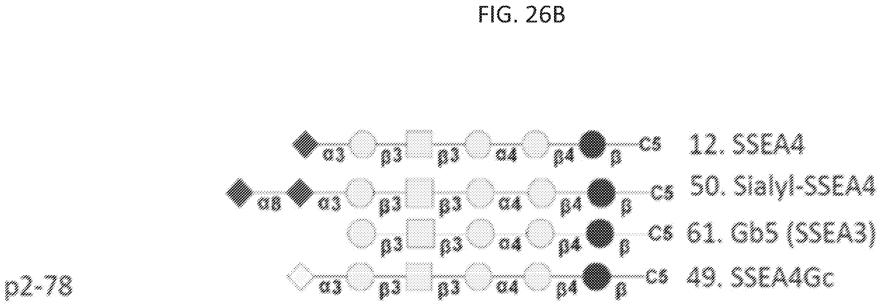

**Figure 26B****.** Glycans recognized by p2-78 hAb.

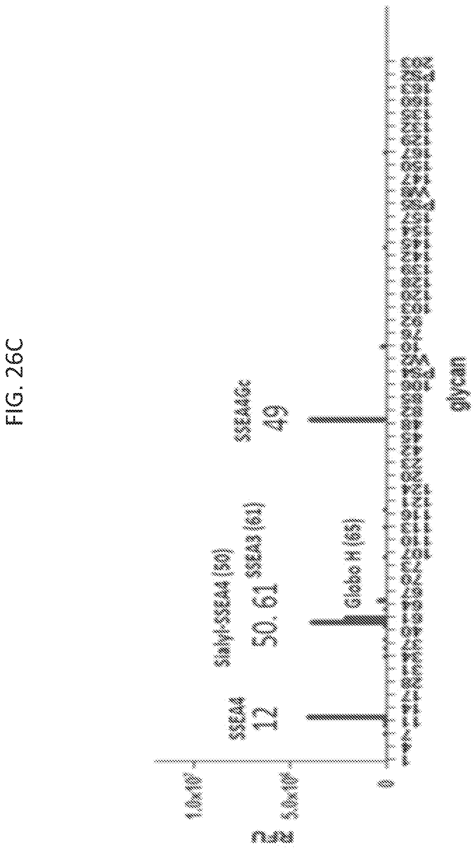

**Figure 26C****.** Glycan array containing 203 different glycans to further confirm the specificity of p2-78 hAb.

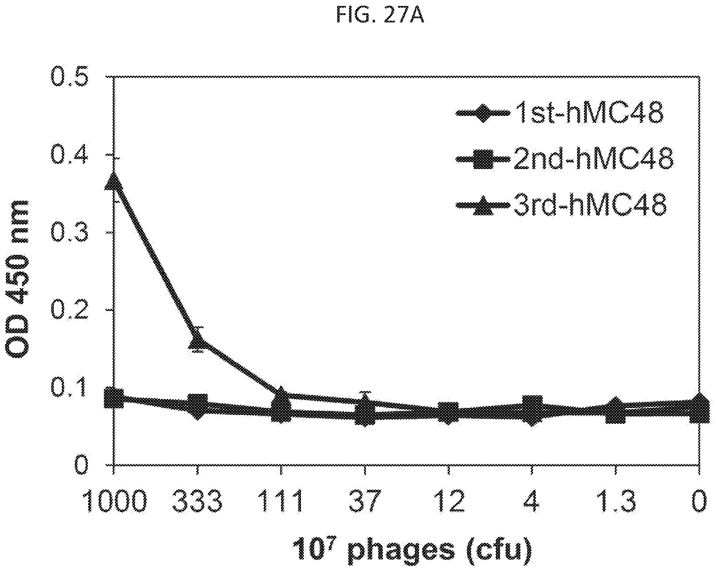

**Figure 27A****.** After alignment of VH and VL variable region of MC48 and MC41 with the NCBI IgBLAST or IMGT database, we generated 1st, 2nd, 3rd and 4th humanized MC48 sequences and 1st, 2nd and 3rd humanized MC41 sequences. We next constructed and generated the phage-displayed scFv formats according to these humanized MC48 and MC41 sequences. To determine the binding activity of the humanized MC48 and MC41 phage clones, we carried out solid-based ELISA coating SSEA-4-BSA. We found that the 3rd and 4th humanized MC48, and 2nd and 3rd humanized MC41 scFv phages could recognize SSEA-4 in a dose-dependent manner, whereas the 1st and 2nd humanized MC48 and 1st MC41 scFv lost the binding activity to SSEA-4. The data showed that the binding affinities of the 4th humanized MC48, and 3rd humanized MC41 scFv phage clones were maintained, compared to that of the murine mAbs MC48 or MC41.

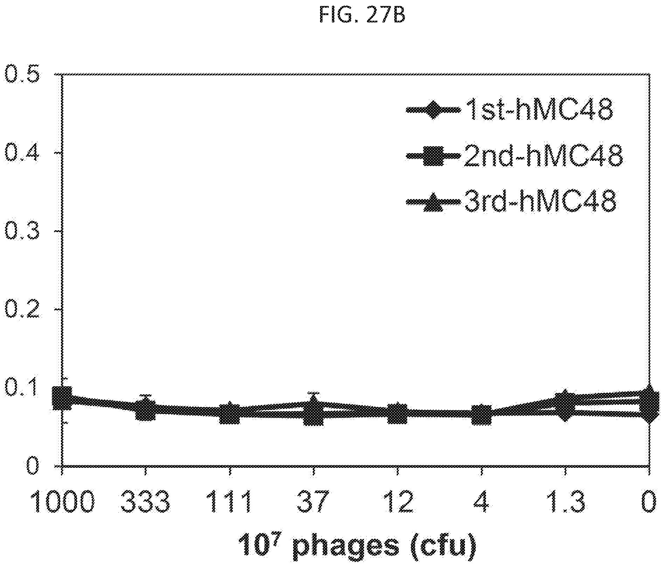

**Figure 27B****.** After alignment of VH and VL variable region of MC48 and MC41 with the NCBI IgBLAST or IMGT database, we generated 1st, 2nd, 3rd and 4th humanized MC48 sequences and 1st, 2nd and 3rd humanized MC41 sequences. We next constructed and generated the phage-displayed scFv formats according to these humanized MC48 and MC41 sequences. To determine the binding activity of the humanized MC48 and MC41 phage clones, we carried out solid-based ELISA coating SSEA-4-BSA. We found that the 3rd and 4th humanized MC48, and 2nd and 3rd humanized MC41 scFv phages could recognize SSEA-4 in a dose-dependent manner, whereas the 1st and 2nd humanized MC48 and 1st MC41 scFv lost the binding activity to SSEA-4. The data showed that the binding affinities of the 4th humanized MC48, and 3rd humanized MC41 scFv phage clones were maintained, compared to that of the murine mAbs MC48 or MC41.

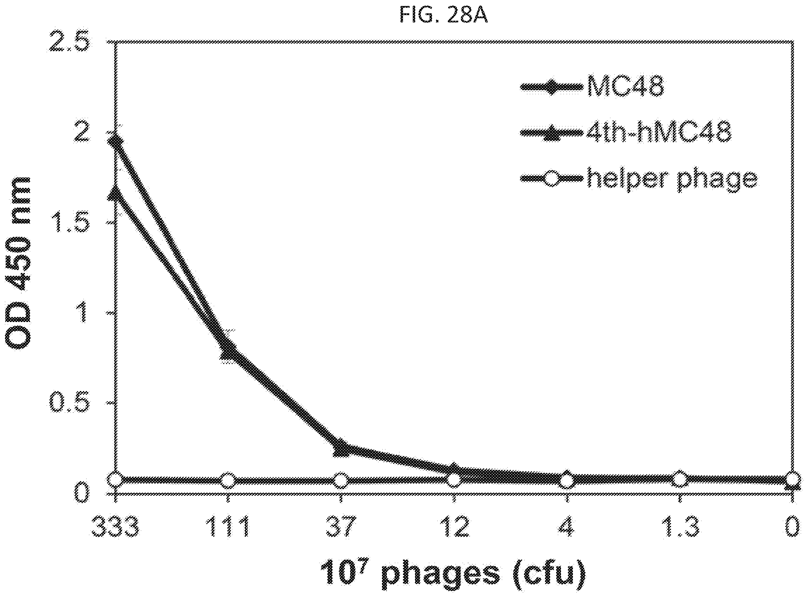

**Figure 28A****.** After alignment of VH and VL variable region of MC48 and MC41 with the NCBI IgBLAST or IMGT database, we generated 1st, 2nd, 3rd and 4th humanized MC48 sequences and 1st, 2nd and 3rd humanized MC41 sequences. We next constructed and generated the phage-displayed scFv formats according to these humanized MC48 and MC41 sequences. To determine the binding activity of the humanized MC48 and MC41 phage clones, we carried out solid-based ELISA coating SSEA-4-BSA. We found that the 3rd and 4th humanized MC48, and 2nd and 3rd humanized MC41 scFv phages could recognize SSEA-4 in a dose-dependent manner, whereas the 1st and 2nd humanized MC48 and 1st MC41 scFv lost the binding activity to SSEA-4. The data showed that the binding affinities of the 4th humanized MC48, and 3rd humanized MC41 scFv phage clones were maintained, compared to that of the murine mAbs MC48 or MC41.

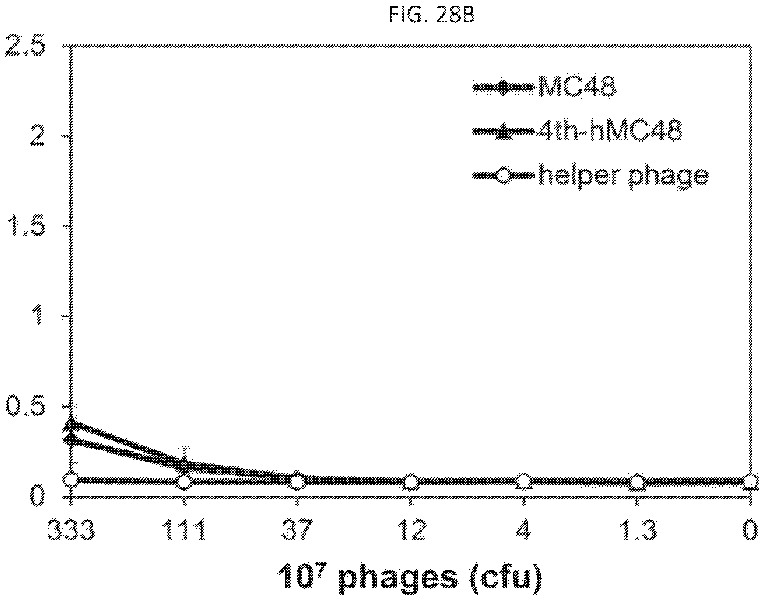

**Figure 28B****.** After alignment of VH and VL variable region of MC48 and MC41 with the NCBI IgBLAST or IMGT database, we generated 1st, 2nd, 3rd and 4th humanized MC48 sequences and 1st, 2nd and 3rd humanized MC41 sequences. We next constructed and generated the phage-displayed scFv formats according to these humanized MC48 and MC41 sequences. To determine the binding activity of the humanized MC48 and MC41 phage clones, we carried out solid-based ELISA coating SSEA-4-BSA. We found that the 3rd and 4th humanized MC48, and 2nd and 3rd humanized MC41 scFv phages could recognize SSEA-4 in a dose-dependent manner, whereas the 1st and 2nd humanized MC48 and 1st MC41 scFv lost the binding activity to SSEA-4. The data showed that the binding affinities of the 4th humanized MC48, and 3rd humanized MC41 scFv phage clones were maintained, compared to that of the murine mAbs MC48 or MC41.

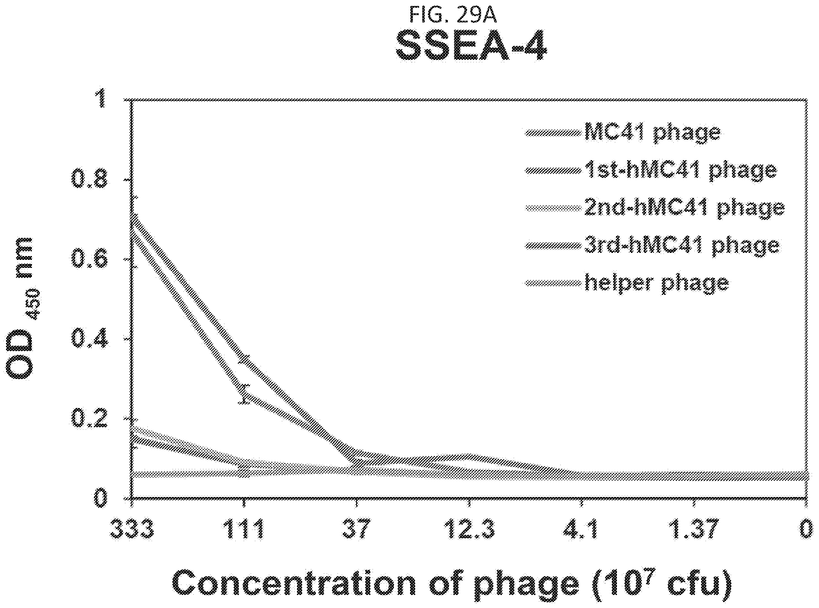

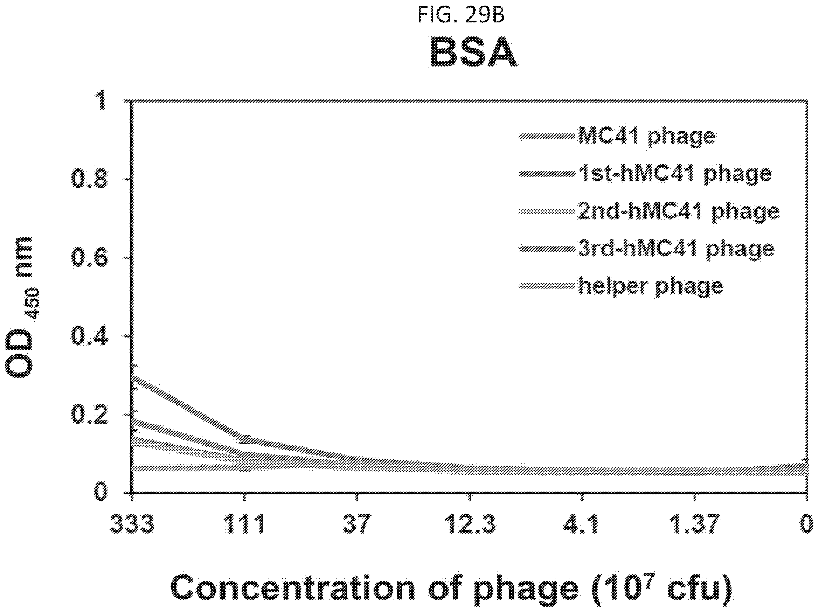

**Figure 29A** **and** **Figure 29B****.** After alignment of VH and VL variable region of MC48 and MC41 with the NCBI IgBLAST or IMGT database, we generated 1st, 2nd, 3rd and 4th humanized MC48 sequences and 1st, 2nd and 3rd humanized MC41 sequences. We next constructed and generated the phage-displayed scFv formats according to these humanized MC48 and MC41 sequences. To determine the binding activity of the humanized MC48 and MC41 phage clones, we carried out solid-based ELISA coating SSEA-4-BSA. We found that the 3rd and 4th humanized MC48, and 2nd and 3rd humanized MC41 scFv phages could recognize SSEA-4 in a dose-dependent manner, whereas the 1st and 2nd humanized MC48 and 1st MC41 scFv lost the binding activity to SSEA-4. The data showed that the binding affinities of the 4th humanized MC48, and 3rd humanized MC41 scFv phage clones were maintained, compared to that of the murine mAbs MC48 or MC41.
**Figure 29B****.** After alignment of VH and VL variable region of MC48 and MC41 with the NCBI IgBLAST or IMGT database, we generated 1st, 2nd, 3rd and 4th humanized MC48 sequences and 1st, 2nd and 3rd humanized MC41 sequences. We next constructed and generated the phage-displayed scFv formats according to these humanized MC48 and MC41 sequences. To determine the binding activity of the humanized MC48 and MC41 phage clones, we carried out solid-based ELISA coating SSEA-4-BSA. We found that the 3rd and 4th humanized MC48, and 2nd and 3rd humanized MC41 scFv phages could recognize SSEA-4 in a dose-dependent manner, whereas the 1st and 2nd humanized MC48 and 1st MC41 scFv lost the binding activity to SSEA-4. The data showed that the binding affinities of the 4th humanized MC48, and 3rd humanized MC41 scFv phage clones were maintained, compared to that of the murine mAbs MC48 or MC41.

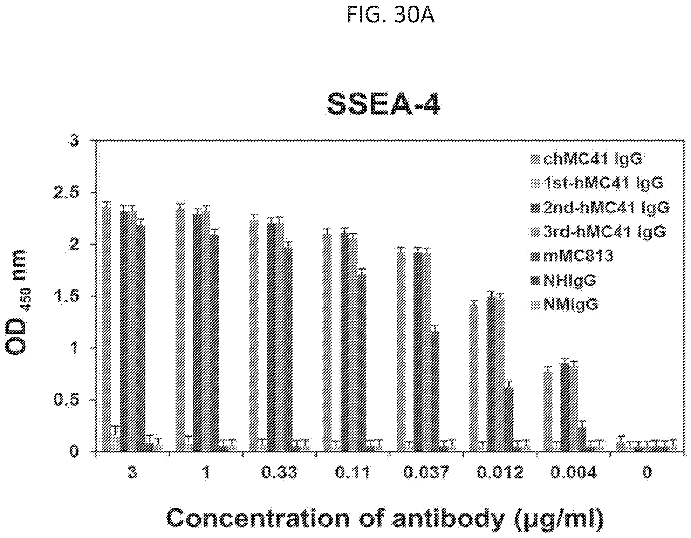

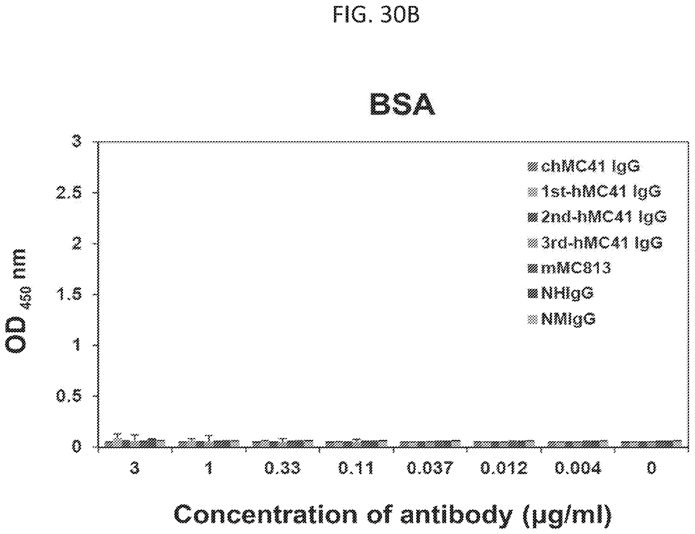

**Figure 30A** **and** **30B****.** To evaluate the binding activity by intact humanized MC41 IgG, we constructed intact IgGs of 1st, 2nd, 3rd humanized MC41 and chimeric MC41 (chMC41). The ELISA results showed that the humanized 2nd and 3rd MC41 could react to SSEA-4 (Fig. 30A) but not to BSA (Fig. 30B) in a dose-dependent pattern, same results were observed for chMC41.

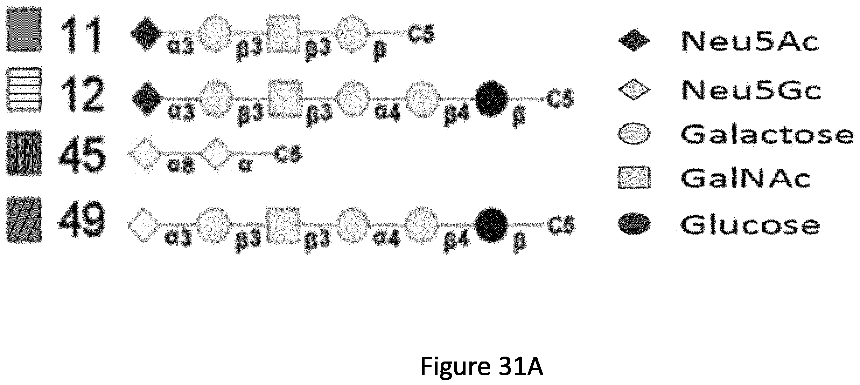

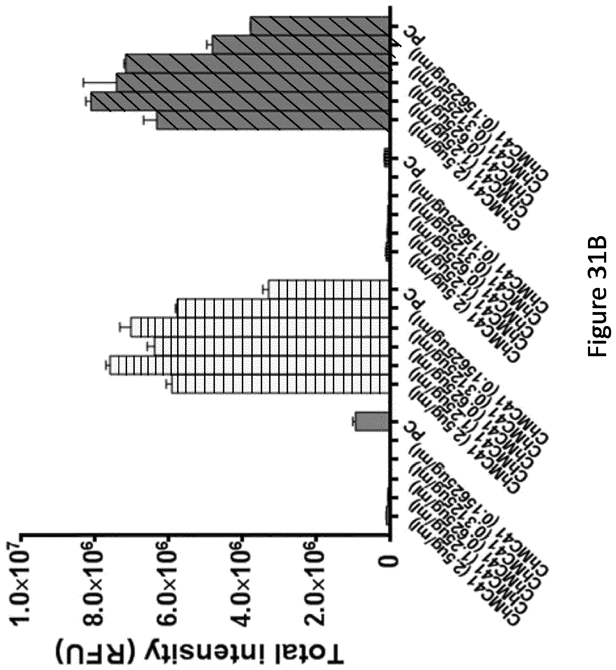

**Figure 31A** **and** **Figure 31B****.** In order to determine the binding specificity of chMC41 and hMC41, glycan array was performed. The chimeric and humanized MC41 showed more specific binding than commercial SSEA4 antibody (MC813). They only recognized SSEA4 or glycolyl modified SSEA4. Fig. 31A shows the glycans that were recognized and Fig. 31B shows the array results.

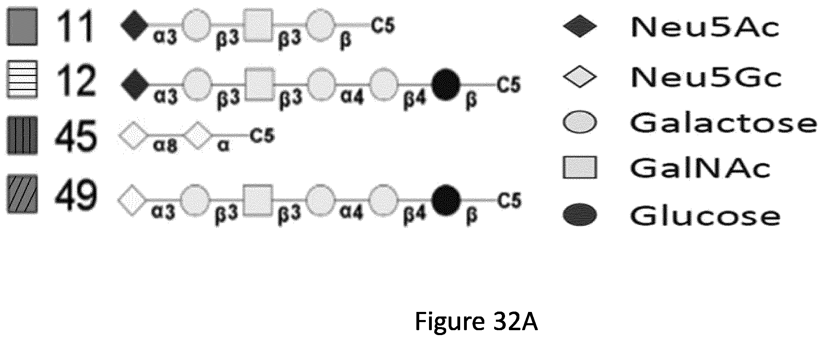

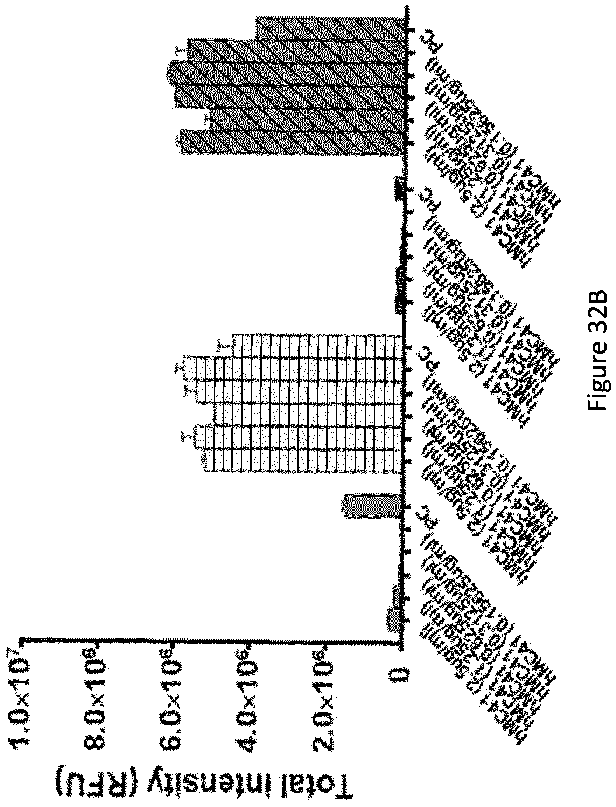

**Figure 32A** **and** **Figure 32B****.** In order to determine the binding specificity of chMC41 and hMC41, glycan array was performed. The chimeric and humanized MC41 showed more specific binding than commercial SSEA4 antibody (MC813). They only recognized SSEA4 or glycolyl modified SSEA4. Fig. 32A shows the glycans that were recognized and Fig. 32B shows the array results.

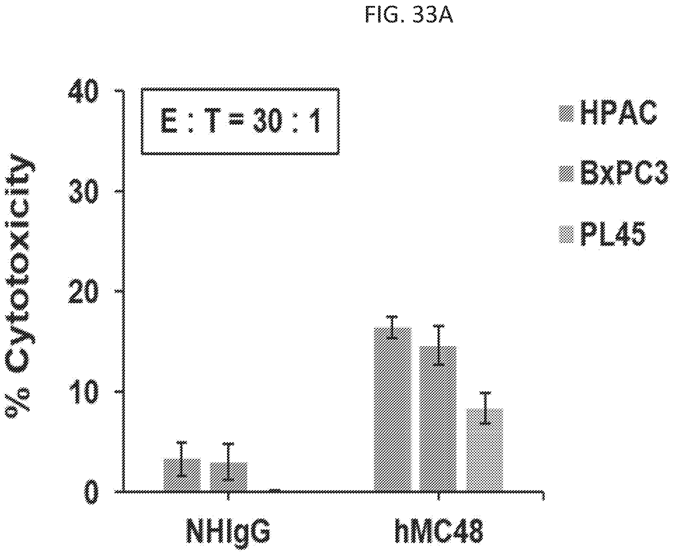

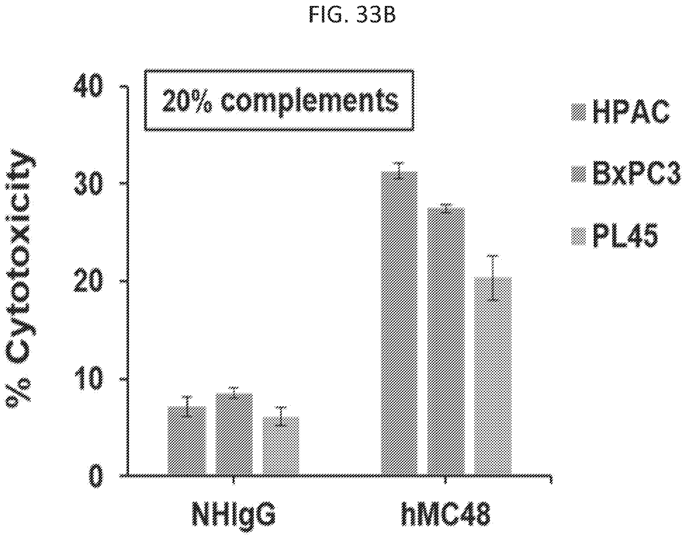

**Figure 33A** **and** **Figure 33B****.** To investigate the effector function of hMC48, chMC41 and hMC41, ADCC and CDC assays were performed. HPAC, BxPC3 and PL45 pancreatic cancer cell lines were used to evaluate the ADCC and CDC activities at the concentration of 10 µg/ml for hMC48 or NHIgG.

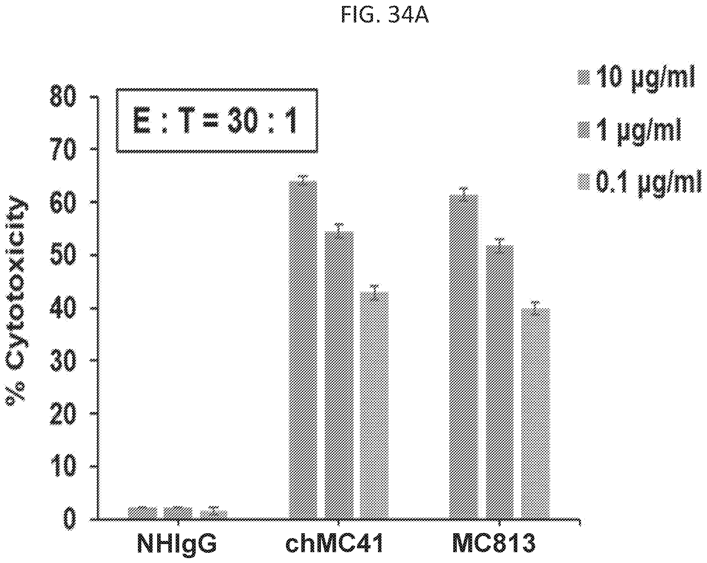

**Figure 34A****.** HPAC cells were treated with chMC41, hMC41, positive control MC813 or negative control NHIgG.

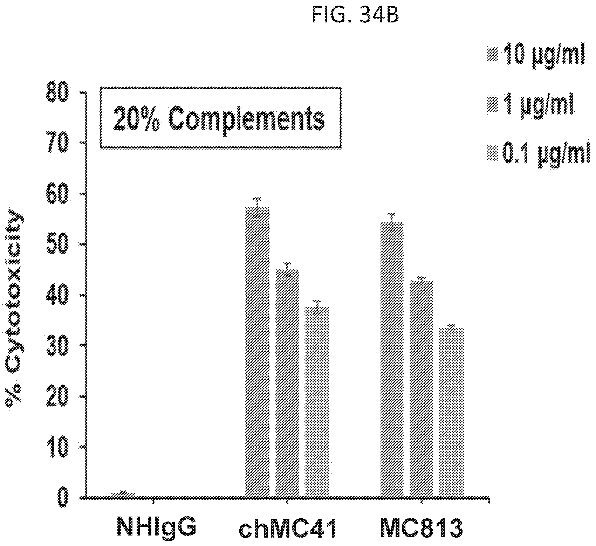

**Figure 34B****.** HPAC cells were treated with chMC41, hMC41, positive control MC813 or negative control NHIgG.

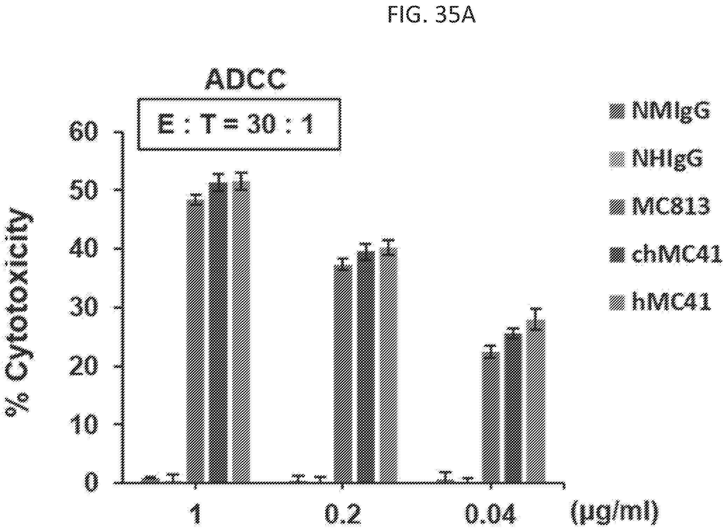

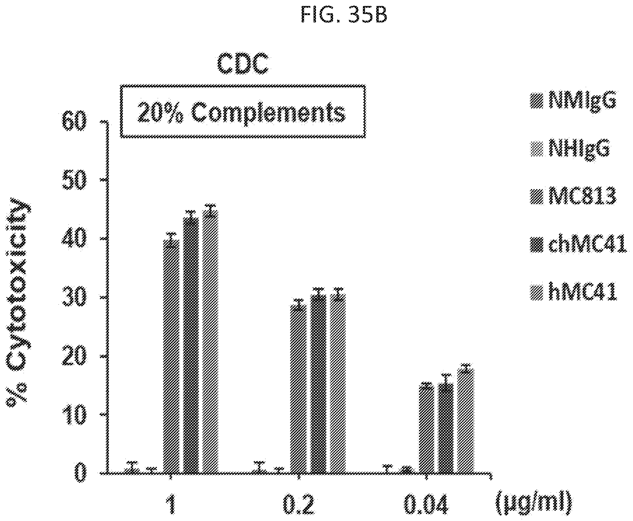

**Figure 35A** **and** **35B****.** The data showed that the effector function of hMC41 and chMC41 was superior to that of hMC48. Interestingly, the humanized MC41 not only maintain its original activity, but it also showed stronger cancer cell killing activity than MC813 through ADCC and CDC.

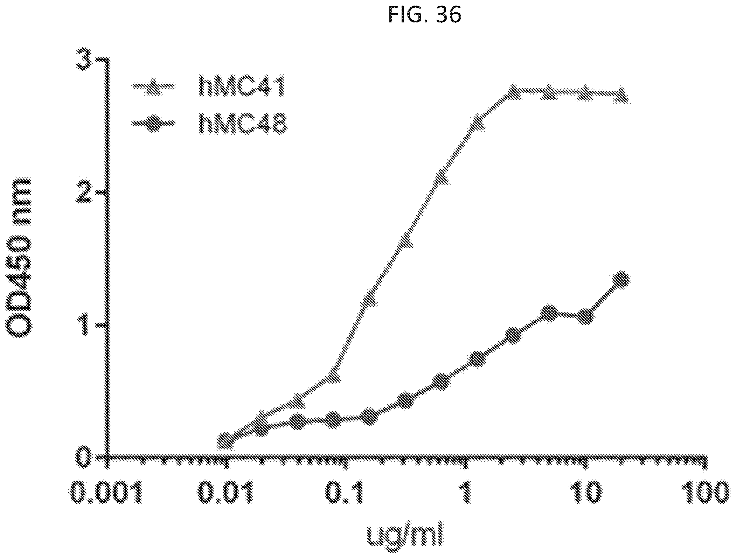

**Figure 36****.** The binding abilities of hMC41 and hMC48 to SSEA-4 were examined by ELISA. The result showed that the binding of hMC41 to SSEA-4 was much better than hMC48. The humanized MC41 has a higher binding maximum and a smaller Kd (0.2 µg/ml and 4.6 µg/ml for hMC41 and hMC48, respectively) value as compared to hMC48.

### DETAILED DESCRIPTIONS

The invention is as defined in the claims.

### Chemical definitions

**Definitions** of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987. Moreover, exemplary glycan and antibody methodologies are described in Wong et al, US20100136042, US20090317837, and US20140051127.

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The invention additionally encompasses compounds described herein as individual isomers substantially free of other isomers, and alternatively, as mixtures of various isomers.

When a range of values is listed, it is intended to encompass each value and sub-range within the range. For example "C₁₋₆" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

The practice of the present invention will employ, unless otherwise indicated, conventional techniques of molecular biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press, 1989); DNA Cloning, Volumes I and II (D. N. Glover ed., 1985); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Antibodies: A Laboratory Manual, by Harlow and Lane s (Cold Spring Harbor Laboratory Press, 1988); and Handbook Of Experimental Immunology, Volumes I-IV (D. M. Weir and C. C. Blackwell, eds., 1986).

As used herein, the term "glycan" refers to a polysaccharide, or oligosaccharide. Glycan is also used herein to refer to the carbohydrate portion of a glycoconjugate, such as a glycoprotein, glycolipid, glycopeptide, glycoproteome, peptidoglycan, lipopolysaccharide or a proteoglycan. Glycans usually consist solely of O-glycosidic linkages between monosaccharides. For example, cellulose is a glycan (or more specifically a glucan) composed of β-1,4-linked D-glucose, and chitin is a glycan composed of β-1,4-linked N-acetyl-D-glucosamine. Glycans can be homo or heteropolymers of monosaccharide residues, and can be linear or branched. Glycans can be found attached to proteins as in glycoproteins and proteoglycans. They are generally found on the exterior surface of cells. O- and N-linked glycans are very common in eukaryotes but may also be found, although less commonly, in prokaryotes. N-Linked glycans are found attached to the R-group nitrogen (N) of asparagine in the sequon. The sequon is a Asn-X-Ser or Asn-X-Thr sequence, where X is any amino acid except praline.

As used herein, the term "epitope" is defined as the parts of an antigen molecule which contact the antigen binding site of an antibody or a T cell receptor.

As used herein, the term "Flow cytometry" or "FACS" means a technique for examining the physical and chemical properties of particles or cells suspended in a stream of fluid, through optical and electronic detection devices.

A non-naturally occurring or an "isolated" antibody is one which has been identified and separated and/or recovered from a component of its native environment. Contaminant components of its native environment are materials which would interfere with research, diagnostic or therapeutic uses for the antibody, and may include enzymes, hormones, and other proteinaceous or nonproteinaceous solutes. In one embodiment, the antibody will be purified (1) to greater than 95% by weight of antibody as determined by, for example, the Lowry method, and in some embodiments more than 99% by weight, (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. Isolated antibody includes the antibody in situ within recombinant cells since at least one component of the antibody's natural environment will not be present. Ordinarily, however, isolated antibody will be prepared by at least one purification step.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (2000). An antibody may be part of a larger fusion molecule, formed by covalent or non-covalent association of the antibody with one or more other proteins or peptides.

"Antibody fragments" comprise only a portion of an intact antibody, wherein the portion retains at least one, and as many as most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an in vivo half life substantially similar to an intact antibody. For example, such an antibody fragment may comprise an antigen binding arm linked to an Fc sequence capable of conferring in vivo stability to the fragment.

Identity or homology with respect to a specified amino acid sequence of this invention is defined herein as the percentage of amino acid residues in a candidate sequence that are identical with the specified residues, after aligning the sequences and introducing gaps, if necessary, to achieve the maximum percent homology, and not considering any conservative substitutions as part of the sequence identity. None of N-terminal, C-terminal or internal extensions, deletions, or insertions into the specified sequence shall be construed as affecting homology. All sequence alignments called for in this invention are such maximal homology alignments. Generally, the nucleic acid sequence homology between the polynucleotides, oligonucleotides, and fragments of the invention and a nucleic acid sequence of interest will be at least 80% >, and more typically with preferably increasing homologies of at least 85%, 90%, 91%, 92%, 92%, 94%, 95%, 96%, 97%, 98%, 99%, and/or 100%. Two amino acid sequences are homologous if there is a partial or complete identity between their sequences.

The term "globoseries -related disorder" refers to or describes a disorder that is typically characterized by or contributed to by aberrant functioning or presentation of the pathway. Examples of such disorders include, but are not limited to, hyperproliferative diseases, including cancer.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing or decreasing inflammation and/or tissue/organ damage, decreasing the rate of disease progression, amelioration or palliation of the disease state, and remission or improved prognosis. In some embodiments, antibodies of the invention are used to delay development of a disease or disorder.

The practice of the present invention will employ, unless otherwise indicated, conventional techniques of molecular biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press, 1989); DNA Cloning, Volumes I and II (D. N. Glover ed., 1985); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Antibodies: A Laboratory Manual, by Harlow and Lane s (Cold Spring Harbor Laboratory Press, 1988); and Handbook Of Experimental Immunology, Volumes I-IV (D. M. Weir and C. C. Blackwell, eds., 1986).

As used herein, the term "glycan" refers to a polysaccharide, or oligosaccharide. Glycan is also used herein to refer to the carbohydrate portion of a glycoconjugate, such as a glycoprotein, glycolipid, glycopeptide, glycoproteome, peptidoglycan, lipopolysaccharide or a proteoglycan. Glycans usually consist solely of O-glycosidic linkages between monosaccharides. For example, cellulose is a glycan (or more specifically a glucan) composed of β-1,4-linked D-glucose, and chitin is a glycan composed of β-1,4-linked N-acetyl-D-glucosamine. Glycans can be homo or heteropolymers of monosaccharide residues, and can be linear or branched. Glycans can be found attached to proteins as in glycoproteins and proteoglycans. They are generally found on the exterior surface of cells. O- and N-linked glycans are very common in eukaryotes but may also be found, although less commonly, in prokaryotes. N-Linked glycans are found attached to the R-group nitrogen (N) of asparagine in the sequon. The sequon is a Asn-X-Ser or Asn-X-Thr sequence, where X is any amino acid except praline.

As used herein, the term "antigen" is defined as any substance capable of eliciting an immune response.

As used herein, the term "immunogenicity" refers to the ability of an immunogen, antigen, or vaccine to stimulate an immune response.

As used herein, the term "CD1d" refers to a member of the CD1 (cluster of differentiation 1) family of glycoproteins expressed on the surface of various human antigen-presenting cells. CD1d presented lipid antigens activate natural killer T cells. CD1d has a deep antigen-binding groove into which glycolipid antigens bind. CD Id molecules expressed on dendritic cells can bind and present glycolipids, including alpha-GalCer analogs such as C34.

As used herein, the term "epitope" is defined as the parts of an antigen molecule which contact the antigen binding site of an antibody or a T cell receptor.

As used herein, the term "vaccine" refers to a preparation that contains an antigen, consisting of whole disease-causing organisms (killed or weakened) or components of such organisms, such as proteins, peptides, or polysaccharides, that is used to confer immunity against the disease that the organisms cause. Vaccine preparations can be natural, synthetic or derived by recombinant DNA technology.

As used herein, the term "antigen specific" refers to a property of a cell population such that supply of a particular antigen, or a fragment of the antigen, results in specific cell proliferation.

As used herein, the term "specifically binding," refers to the interaction between binding pairs (e.g., an antibody and an antigen). In various instances, specifically binding can be embodied by an affinity constant of about 10-6 moles/liter, about 10-7 moles/liter, or about 10-8 moles/liter, or less.

An "isolated" antibody is one which has been identified and separated and/or recovered from a component of its natural environment. Contaminant components of its natural environment are materials which would interfere with research, diagnostic or therapeutic uses for the antibody, and may include enzymes, hormones, and other proteinaceous or nonproteinaceous solutes. In one embodiment, the antibody will be purified (1) to greater than 95% by weight of antibody as determined by, for example, the Lowry method, and in some embodiments more than 99% by weight, (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. Isolated antibody includes the antibody in situ within recombinant cells since at least one component of the antibody's natural environment will not be present. Ordinarily, however, isolated antibody will be prepared by at least one purification step.

The phrase "substantially similar," "substantially the same", "equivalent", or "substantially equivalent", as used herein, denotes a sufficiently high degree of similarity between two numeric values (for example, one associated with a molecule and the other associated with a reference/comparator molecule) such that one of skill in the art would consider the difference between the two values to be of little or no biological and/or statistical significance within the context of the biological characteristic measured by said values (e.g., Kd values, anti-viral effects, etc.). The difference between said two values is, for example, less than about 50%, less than about 40%, less than about 30%, less than about 20%, and/or less than about 10% as a function of the value for the reference/comparator molecule.

The phrase "substantially reduced," or "substantially different", as used herein, denotes a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (e.g., Kd values). The difference between said two values is, for example, greater than about 10%, greater than about 20%, greater than about 30%, greater than about 40%, and/or greater than about 50% as a function of the value for the reference/comparator molecule.

"Binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative embodiments are described in the following.

In one embodiment, the "Kd" or "Kd value" according to this invention is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (1251)-labeled antigen in the presence of a titration series of unlabeled antigen, then capturing bound antigen with an anti-Fab antibody-coated plate (Chen, et al., (1999) J. Mol Biol 293:865-881). To establish conditions for the assay, microtiter plates (Dynex) are coated overnight with 5 µ g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23° C.). In a non-adsorbent plate (Nunc #269620), 100 pM or 26 pM [125I]-antigen are mixed with serial dilutions of a Fab of interest (e.g., consistent with assessment of an anti-VEGF antibody, Fab-12, in Presta et al., (1997) Cancer Res. 57:4593-4599). The Fab of interest is then incubated overnight; however, the incubation may continue for a longer period (e.g., 65 hours) to insure that equilibrium is reached. Thereafter, the mixtures are transferred to the capture plate for incubation at room temperature (e.g., for one hour). The solution is then removed and the plate washed eight times with 0.1% Tween-20 in PBS. When the plates have dried, 150 µl/well of scintillant (MicroScint-20; Packard) is added, and the plates are counted on a Topcount gamma counter (Packard) for ten minutes. Concentrations of each Fab that give less than or equal to 20% of maximal binding are chosen for use in competitive binding assays. According to another embodiment the Kd or Kd value is measured by using surface plasmon resonance assays using a BIAcore™-2000 or a BIAcore™-3000 (BIAcore, Inc., Piscataway, N.J.) at 25° C. with immobilized antigen CM5 chips at ^{∼}10 response units (RU). Briefly, carboxymethylated dextran biosensor chips (CM5, BIAcore Inc.) are activated with N-ethyl-N'-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (^{∼}0.2 µM) before injection at a flow rate of 5 µl/minute to achieve approximately 10 response units (RU) of coupled protein. Following the injection of antigen, 1 M ethanolamine is injected to block unreacted groups. In each experiment, a spot was activated and ethanolamine blocked without immobilizing protein, to be used for reference subtraction. For kinetics measurements, two-fold serial dilutions of Fab (0.78 nM to 500 nM) are injected in PBS with 0.05% Tween 20 (PBST) at 25° C. at a flow rate of approximately 25 µl/min. Association rates (kon) and dissociation rates (koff) are calculated using a simple one-to-one Langmuir binding model (BIAcore Evaluation Software version 3.2) by simultaneously fitting the association and dissociation sensorgrams. The equilibrium dissociation constant (Kd) is calculated as the ratio koff/kon. See, e.g., Chen, Y., et al., (1999) J. Mol Biol 293:865-881. If the on-rate exceeds 106 M-1s-1 by the surface plasmon resonance assay above, then the on-rate can be determined by using a fluorescent quenching technique that measures the increase or decrease in fluorescence emission intensity (excitation=295 nm; emission=340 nm, 16 nm band-pass) at 25° C. of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

An "on-rate" or "rate of association" or "association rate" or "kon" according to this invention can also be determined with the same surface plasmon resonance technique described above using a BIAcore™-2000 or a BIAcore™-3000 (BIAcore, Inc., Piscataway, N.J.) at 25° C. with immobilized antigen CM5 chips at ^{∼}10 response units (RU). Briefly, carboxymethylated dextran biosensor chips (CM5, BIAcore Inc.) are activated with N-ethyl-N '-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (^{∼}0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% Tween 20 (PBST) at 25° C. at a flow rate of approximately 25 µl/min. Association rates (kon) and dissociation rates (koff) are calculated using a simple one-to-one Langmuir binding model (BIAcore Evaluation Software version 3.2) by simultaneously fitting the association and dissociation sensorgram. The equilibrium dissociation constant (Kd) was calculated as the ratio koff/kon. See, e.g., Chen, Y., et al., (1999)J. Mol Biol 293:865-881. However, if the on-rate exceeds 106 M-1s-1 by the surface plasmon resonance assay above, then the on-rate can be determined by using a fluorescent quenching technique that measures the increase or decrease in fluorescence emission intensity (excitation=295 nm; emission=340 nm, 16 nm band-pass) at 25° C. of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5 'and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), "(O)NR2 ("amidate"), P(O)R, P(O)OR', CO or CH2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single-stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (e.g., full length or intact monoclonal antibodies), polyclonal antibodies, monovalent, multivalent antibodies, multispecific antibodies (e.g., bispecific antibodies so long as they exhibit the desired biological activity) and may also include certain antibody fragments (as described in greater detail herein). An antibody can be chimeric, human, humanized and/or affinity matured.

The "variable region" or "variable domain" of an antibody refers to the amino-terminal domains of heavy or light chain of the antibody. These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a beta-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen-binding site of antibodies (see Kabat et al., Sequences of Proteins of Immunological Interest, Fifth Edition, National Institute of Health, Bethesda, Md. (1991)). The constant domains are not involved directly in binding an antibody to an antigen, but exhibit various effector functions, such as participation of the antibody in antibody-dependent cellular toxicity.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen.

"Fv" is the minimum antibody fragment which contains a complete antigen-recognition and -binding site. In a two-chain Fv species, this region consists of a dimer of one heavy- and one light-chain variable domain in tight, non-covalent association. In a single-chain Fv species, one heavy- and one light-chain variable domain can be covalently linked by a flexible peptide linker such that the light and heavy chains can associate in a "dimeric" structure analogous to that in a two-chain Fv species. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (2000). An antibody may be part of a larger fusion molecule, formed by covalent or non-covalent association of the antibody with one or more other proteins or peptides.

The terms "full length antibody," "intact antibody" and "whole antibody" are used herein interchangeably, to refer to an antibody in its substantially intact form, not antibody fragments as defined below. The terms particularly refer to an antibody with heavy chains that contain the Fc region.

"Antibody fragments" comprise only a portion of an intact antibody, wherein the portion retains at least one, and as many as most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an in vivo half life substantially similar to an intact antibody. For example, such an antibody fragment may comprise an antigen binding arm linked to an Fc sequence capable of conferring in vivo stability to the fragment.

The term "monoclonal antibody" as used herein refers to an antibody obtained from a population of substantially homogeneous antibodies, e.g., the individual antibodies comprising the population are identical except for possible naturally occurring mutations that may be present in minor amounts or comprising only homogeneous glycoform profile (having only a single glycan or single glycan profile on a glycoantibody in a population). Examples of homogeneous antibody composition to enhance the effector functions by using the 2,3- and 2,6-sialyl and defucosylated complex bi-antennary glycans at the Fc-297 position are described in US12/959, 351. Thus, the modifier "monoclonal" indicates the character of the antibody as not being a mixture of discrete antibodies. Such monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones or recombinant DNA clones. It should be understood that the selected target binding sequence can be further altered, for example, to improve affinity for the target, to humanize the target binding sequence, to improve its production in cell culture, to reduce its immunogenicity in vivo, to create a multispecific antibody, etc., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparation is directed against a single determinant on an antigen. In addition to their specificity, the monoclonal antibody preparations are advantageous in that they are typically uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler et al., Nature, 256: 495 (1975); Harlow et al., Antibodies: A Laboratory Manual, (Cold Spring Harbor Laboratory Press, 2nd ed. 1988); Hammerling et al., in: Monoclonal Antibodies and T-Cell hybridomas 563-681 (Elsevier, N.Y., 1981)), recombinant DNA methods (see, e.g., U.S. Pat. No. 4,816,567), phage display technologies (See, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132 (2004), and technologies for producing human or human-like antibodies in animals that have parts or all of the human immunoglobulin loci or genes encoding human immunoglobulin sequences (see, e.g., WO98/24893; WO96/34096; WO96/33735; WO91/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Pat. Nos. 5,545,807; 5,545,806; 5,569,825; 5,625,126; 5,633,425; 5,661,016; Marks et al., Bio. Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996) and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA 81:6851-6855 (1984)).

Humanized" forms of non-human (e.g., murine) antibodies are chimeric antibodies that contain minimal sequence derived from non-human immunoglobulin. In one embodiment, a humanized antibody is a human immunoglobulin (recipient antibody) in which residues from a hypervariable region of the recipient are replaced by residues from a hypervariable region of a non-human species (donor antibody) such as mouse, rat, rabbit or nonhuman primate having the desired specificity, affinity, and/or capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

The term "hypervariable region", "HVR", or "HV", when used herein refers to the regions of an antibody variable domain which are hypervariable in sequence and/or form structurally defined loops. Generally, antibodies comprise six hypervariable regions; three in the VH (H1, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these hypervariable regions are noted below.
Loop Kabat AbM Chothia Contact
L1 L24-L34 L24-L34 L26-L32 L30-L36
L2 L50-L56 L50-L56 L50-L52 L46-L55
L3 L89-L97 L89-L97 L91-L96 L89-L96
H1 H31-H35B H26-H35B H26-H32 H30-H35B

### (Kabat Numbering)

H1 H31-H35 H26-H35 H26-H32 H30-H35

### (Chothia Numbering)

H2 H50-H65 H50-H58 H53-H55 H47-H58
H3 H95-H102 H95-H102 H96-H101 H93-H101

Hypervariable regions may comprise "extended hypervariable regions" as follows: 24-36 or 24-34 (L1), 46-56 or 50-56 or 49-56 (L2) and 89-97 or 89-96 (L3) in the VL and 26-35 (HI), 50-65 or 49-65 (H2) and 93-102, 94-102, or 95-102 (H3) in the VH. The variable domain residues are numbered according to Kabat et al., supra, for each of these definitions.

"Framework" or "FR" residues are those variable domain residues other than the hypervariable region residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO93/1161; and Hollinger et al., Proc. Natl. Acad. Sci. USA 90: 6444-6448 (1993).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more HVRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking" antibody or an "antagonist" antibody is one which inhibits or reduces biological activity of the antigen it binds. Certain blocking antibodies or antagonist antibodies substantially or completely inhibit the biological activity of the antigen.

An "agonist antibody", as used herein, is an antibody which mimics at least one of the functional activities of a polypeptide of interest.

A "disorder" is any condition that would benefit from treatment with an antibody of the invention. This includes chronic and acute disorders or diseases including those pathological conditions which predispose the mammal to the disorder in question. Nonlimiting examples of disorders to be treated herein include cancer.

The terms "cell proliferative disorder" and "proliferative disorder" refer to disorders that are associated with some degree of abnormal cell proliferation. In one embodiment, the cell proliferative disorder is cancer.

"Tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

The terms "cancer" and "cancerous" refer to or describe the physiological condition in mammals that is typically characterized by unregulated cell growth/proliferation. Examples of cancer include, but are not limited to, carcinoma, lymphoma (e.g., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing or decreasing inflammation and/or tissue/organ damage, decreasing the rate of disease progression, amelioration or palliation of the disease state, and remission or improved prognosis. In some embodiments, antibodies of the invention are used to delay development of a disease or disorder.

An "individual" or a "subject" is a vertebrate. In certain embodiments, the vertebrate is a mammal. Mammals include, but are not limited to, farm animals (such as cows), sport animals, pets (such as cats, dogs, and horses), primates, mice and rats. In certain embodiments, the vertebrate is a human.

"Mammal" for purposes of treatment refers to any animal classified as a mammal, including humans, domestic and farm animals, and zoo, sports, or pet animals, such as dogs, horses, cats, cows, etc. In certain embodiments, the mammal is human.

An "effective amount" refers to an amount effective, at dosages and for periods of time necessary, to achieve the desired therapeutic or prophylactic result.

A "therapeutically effective amount" of a substance/molecule of the invention may vary according to factors such as the disease state, age, sex, and weight of the individual, and the ability of the substance/molecule, to elicit a desired response in the individual. A therapeutically effective amount is also one in which any toxic or detrimental effects of the substance/molecule are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount would be less than the therapeutically effective amount.

The term "cytotoxic agent" as used herein refers to a substance that inhibits or prevents the function of cells and/or causes destruction of cells. The term is intended to include radioactive isotopes (e.g., At211, 1131, 1125, Y90, Re186, Re188, Sm153, Bi212, P32, Pb212 and radioactive isotopes of Lu), chemotherapeutic agents (e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolyticenzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gamma1I and calicheamicin omegaI1 (see, e.g., Agnew, Chem. Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANE™ Cremophor-free, albumin-engineered nanoparticle formulation of paclitaxel (American Pharmaceutical Partners, Schaumberg, 111.), and TAXOTERE® doxetaxel (Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluoromethylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin.

### Pharmaceutical Formulations

The pharmaceutical composition is administered in a manner compatible with the dosage formulation, and in an amount that is therapeutically effective, protective and therapeutic. Precise amounts of active ingredient required to be administered depend on the judgment of the practitioner. However, suitable dosage ranges are readily determinable by one skilled in the art. Suitable regimes for initial administration and booster doses are also variable, but may include an initial administration followed by subsequent administrations. The dosage of the vaccine may also depend on the route of administration and varies according to the size of the host.

Methods of making monoclonal and polyclonal antibodies and fragments thereof in animals (e.g., mouse, rabbit, goat, sheep, or horse) are well known in the art. See, for example, Harlow and Lane, (1988) Antibodies: A Laboratory Manual, Cold Spring Harbor Laboratory, New York. The term "antibody" includes intact immunoglobulin molecules as well as fragments thereof, such as Fab, F(ab')₂, Fv, scFv (single chain antibody), and dAb (domain antibody; Ward, et. al. (1989) Nature, 341, 544).

The compositions disclosed herein can be included in a pharmaceutical composition together with additional active agents, carriers, vehicles, excipients, or auxiliary agents identifiable by a person skilled in the art upon reading of the present disclosure.

The pharmaceutical compositions preferably comprise at least one pharmaceutically acceptable carrier. In such pharmaceutical compositions, the compositions disclosed herein form the "active compound," also referred to as the "active agent." As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions. A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol, or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates, or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

### Clinical Applications

The present invention provides selected and directed optimized glycoantibodies useful for the treatment of a proliferative disease such as cancer (*e.g.* lung cancer, large bowel cancer, pancreas cancer, biliary tract cancer, or endometrial cancer), benign neoplasm, or angiogenesis in a subject.

The compositions described herein can be used in cancer treatment. Methods of making monoclonal and polyclonal antibodies and fragments thereof in human and/or animals (e.g., mouse, rabbit, goat, sheep, or horse) are well known in the art. See, for example, Harlow and Lane, (1988) Antibodies: A Laboratory Manual, Cold Spring Harbor Laboratory, New York. The term "antibody" includes intact immunoglobulin molecules as well as fragments thereof, such as Fab, F(ab').sub.2, Fv, scFv (single chain antibody), and dAb (domain antibody; Ward, et. al. (1989) Nature, 341, 544).

These compositions may further comprise suitable carriers, such as pharmaceutically acceptable excipients including buffers, which are well known in the art.

Non naturally occurring and or isolated antibodies and polynucleotides are also provided. In certain embodiments, the isolated antibodies and polynucleotides are substantially pure.

The antigen-binding domain of an antibody is formed from two variable (V) regions of about 110 amino acids, one each from the light (VL) and heavy (VH) chains, that both present three hypervariable loops or complementarity-determining regions (CDRs). Variable domains can be displayed functionally on phage, either as single-chain Fv (scFv) fragments, in which VH and VL are covalently linked through a short, flexible peptide, or as Fab fragments, in which they are each fused to a constant domain and interact non-covalently, as described in Winter et al., Ann. Rev. Immunol., 12: 433-455 (1994). As used herein, scFv encoding phage clones and Fab encoding phage clones are collectively referred to as "Fv phage clones" or "Fv clones".

Repertoires of VH and VL genes can be separately cloned by polymerase chain reaction (PCR) and recombined randomly in phage libraries, which can then be searched for antigen-binding clones as described in Winter et al., Ann. Rev. Immunol., 12: 433-455 (1994). Libraries from immunized sources provide high-affinity antibodies to the immunogen without the requirement of constructing hybridomas. Alternatively, the naive repertoire can be cloned to provide a single source of human antibodies to a wide range of non-self and also self antigens without any immunization as described by Griffiths et al., EMBO J, 12: 725-734 (1993). Finally, naive libraries can also be made synthetically by cloning the unrearranged V-gene segments from stem cells, and using PCR primers containing random sequence to encode the highly variable CDR3 regions and to accomplish rearrangement in vitro as described by Hoogenboom and Winter, J. Mol. Biol., 227: 381-388 (1992).

Filamentous phage is used to display antibody fragments by fusion to the minor coat protein pIII. The antibody fragments can be displayed as single chain Fv fragments, in which VH and VL domains are connected on the same polypeptide chain by a flexible polypeptide spacer, e.g. as described by Marks et al., J. Mol. Biol., 222: 581-597 (1991), or as Fab fragments, in which one chain is fused to pIII and the other is secreted into the bacterial host cell periplasm where assembly of a Fab-coat protein structure which becomes displayed on the phage surface by displacing some of the wild type coat proteins, e.g. as described in Hoogenboom et al., Nucl. Acids Res., 19: 4133-4137 (1991).

Nucleic acid encoding antibody variable gene segments (including VH and VL segments) are recovered from the cells of interest and amplified. In the case of rearranged VH and VL gene libraries, the desired DNA can be obtained by isolating genomic DNA or mRNA from lymphocytes followed by polymerase chain reaction (PCR) with primers matching the 5' and 3' ends of rearranged VH and VL genes as described in Orlandi et al., Proc. Natl. Acad. Sci. (USA), 86: 3833-3837 (1989), thereby making diverse V gene repertoires for expression. The V genes can be amplified from cDNA and genomic DNA, with back primers at the 5' end of the exon encoding the mature V-domain and forward primers based within the J-segment as described in Orlandi et al. (1989) and in Ward et al., Nature, 341: 544-546 (1989). However, for amplifying from cDNA, back primers can also be based in the leader exon as described in Jones et al., Biotechnol., 9: 88-89 (1991), and forward primers within the constant region as described in Sastry et al., Proc. Natl. Acad. Sci. (USA), 86: 5728-5732 (1989). To maximize complementarity, degeneracy can be incorporated in the primers as described in Orlandi et al. (1989) or Sastry et al. (1989). The library diversity is maximized by using PCR primers targeted to each V-gene family in order to amplify all available VH and VL arrangements present in the immune cell nucleic acid sample, e.g. as described in the method of Marks et al., J. Mol. Biol., 222: 581-597 (1991) or as described in the method of Orum et al., Nucleic Acids Res., 21: 4491-4498 (1993). For cloning of the amplified DNA into expression vectors, rare restriction sites can be introduced within the PCR primer as a tag at one end as described in Orlandi et al. (1989), or by further PCR amplification with a tagged primer as described in Clackson et al., Nature, 352: 624-628 (1991).

Repertoires of synthetically rearranged V genes can be derived in vitro from V gene segments. Most of the human VH-gene segments have been cloned and sequenced (reported in Tomlinson et al., J. Mol. Biol., 227: 776-798 (1992)), and mapped (reported in Matsuda et al., Nature Genet., 3: 88-94 (1993); these cloned segments (including all the major conformations of the H1 and H2 loop) can be used to generate diverse VH gene repertoires with PCR primers encoding H3 loops of diverse sequence and length as described in Hoogenboom and Winter, J. Mol. Biol., 227: 381-388 (1992). VH repertoires can also be made with all the sequence diversity focused in a long H3 loop of a single length as described in Barbas et al., Proc. Natl. Acad. Sci. USA, 89: 4457-4461 (1992). Human Vκ and Vλ segments have been cloned and sequenced (reported in Williams and Winter, Eur. J. Immunol., 23: 1456-1461 (1993)) and can be used to make synthetic light chain repertoires. Synthetic V gene repertoires, based on a range of VH and VL folds, and L3 and H3 lengths, will encode antibodies of considerable structural diversity. Following amplification of V-gene encoding DNAs, germline V-gene segments can be rearranged in vitro according to the methods of Hoogenboom and Winter, J. Mol. Biol., 227: 381-388 (1992).

Repertoires of antibody fragments can be constructed by combining VH and VL gene repertoires together in several ways. Each repertoire can be created in different vectors, and the vectors recombined in vitro, e.g., as described in Hogrefe et al., Gene, 128: 119-126 (1993), or in vivo by combinatorial infection, e.g., the loxP system described in Waterhouse et al., Nucl. Acids Res., 21: 2265-2266 (1993). The in vivo recombination approach exploits the two-chain nature of Fab fragments to overcome the limit on library size imposed by E. coli transformation efficiency. Naive VH and VL repertoires are cloned separately, one into a phagemid and the other into a phage vector. The two libraries are then combined by phage infection of phagemid-containing bacteria so that each cell contains a different combination and the library size is limited only by the number of cells present (about 1012 clones). Both vectors contain in vivo recombination signals so that the VH and VL genes are recombined onto a single replicon and are co-packaged into phage virions. These huge libraries provide large numbers of diverse antibodies of good affinity (Kd -1 of about 10-8 M).

Alternatively, the repertoires may be cloned sequentially into the same vector, e.g. as described in Barbas et al., Proc. Natl. Acad. Sci. USA, 88: 7978-7982 (1991), or assembled together by PCR and then cloned, e.g. as described in Clackson et al., Nature, 352: 624-628 (1991). PCR assembly can also be used to join VH and VL DNAs with DNA encoding a flexible peptide spacer to form single chain Fv (scFv) repertoires. In yet another technique, "in cell PCR assembly" is used to combine VH and VL genes within lymphocytes by PCR and then clone repertoires of linked genes as described in Embleton et al., Nucl. Acids Res., 20: 3831-3837 (1992).

Screening of the libraries can be accomplished by any art-known technique. Targets can be used to coat the wells of adsorption plates, expressed on host cells affixed to adsorption plates or used in cell sorting, or conjugated to biotin for capture with streptavidin-coated beads, or used in any other art-known method for panning phage display libraries.

The phages bound to the solid phase are washed and then eluted by acid, e.g. as described in Barbas et al., Proc. Natl. Acad. Sci. USA, 88: 7978-7982 (1991), or by alkali, e.g. as described in Marks et al., J. Mol. Biol., 222: 581-597 (1991), or by SSEA-3/SSEA-4/GLOBO H antigen competition, e.g. in a procedure similar to the antigen competition method of Clackson et al., Nature, 352: 624-628 (1991). Phages can be enriched 20-1,000-fold in a single round of selection. Moreover, the enriched phages can be grown in bacterial culture and subjected to further rounds of selection.

The efficiency of selection depends on many factors, including the kinetics of dissociation during washing, and whether multiple antibody fragments on a single phage can simultaneously engage with antigen. Antibodies with fast dissociation kinetics (and weak binding affinities) can be retained by use of short washes, multivalent phage display and high coating density of antigen in solid phase. The high density not only stabilizes the phage through multivalent interactions, but favors rebinding of phage that has dissociated. The selection of antibodies with slow dissociation kinetics (and good binding affinities) can be promoted by use of long washes and monovalent phage display as described in Bass et al., Proteins, 8: 309-314 (1990) and in WO 92/09690, and a low coating density of antigen as described in Marks et al., Biotechnol., 10: 779-783 (1992).

However, random mutation of a selected antibody (e.g. as performed in some of the affinity maturation techniques described above) is likely to give rise to many mutants, most binding to antigen, and a few with higher affinity. With limiting SSEA-4, rare high affinity phage could be competed out. To retain all the higher affinity mutants, phages can be incubated with excess biotinylated SSEA-4, but with the biotinylated SSEA-4 at a concentration of lower molarity than the target molar affinity constant for SSEA-4. The high affinity-binding phages can then be captured by streptavidin-coated paramagnetic beads. Such "equilibrium capture" allows the antibodies to be selected according to their affinities of binding, with sensitivity that permits isolation of mutant clones with as little as two-fold higher affinity from a great excess of phages with lower affinity. Conditions used in washing phages bound to a solid phase can also be manipulated to discriminate on the basis of dissociation kinetics.

DNA encoding the Fv clones of the invention is readily isolated and sequenced using conventional procedures (e.g. by using oligonucleotide primers designed to specifically amplify the heavy and light chain coding regions of interest from hybridoma or phage DNA template). Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of the desired monoclonal antibodies in the recombinant host cells. Review articles on recombinant expression in bacteria of antibody-encoding DNA include Skerra et al., Curr. Opinion in Immunol., 5: 256 (1993) and Pluckthun, Immunol. Revs, 130: 151 (1992).

DNA encoding the Fv clones of the invention can be combined with known DNA sequences encoding heavy chain and/or light chain constant regions (e.g. the appropriate DNA sequences can be obtained from Kabat et al., supra) to form clones encoding full or partial length heavy and/or light chains. It will be appreciated that constant regions of any isotype can be used for this purpose, including IgG, IgM, IgA, IgD, and IgE constant regions, and that such constant regions can be obtained from any human or animal species. A Fv clone derived from the variable domain DNA of one animal (such as human) species and then fused to constant region DNA of another animal species to form coding sequence(s) for "hybrid", full length heavy chain and/or light chain is included in the definition of "chimeric" and "hybrid" antibody as used herein. In one embodiment, a Fv clone derived from human variable DNA is fused to human constant region DNA to form coding sequence(s) for all human, full or partial length heavy and/or light chains.

The antibodies produced by naive libraries (either natural or synthetic) can be of moderate affinity (Kd -1 of about 106 to 107 M-1), but affinity maturation can also be mimicked in vitro by constructing and reselecting from secondary libraries as described in Winter et al. (1994), supra. For example, mutation can be introduced at random in vitro by using error-prone polymerase (reported in Leung et al., Technique, 1: 11-15 (1989)) in the method of Hawkins et al., J. Mol. Biol., 226: 889-896 (1992) or in the method of Gram et al., Proc. Natl. Acad. Sci. USA, 89: 3576-3580 (1992). Additionally, affinity maturation can be performed by randomly mutating one or more CDRs, e.g. using PCR with primers carrying random sequence spanning the CDR of interest, in selected individual Fv clones and screening for higher affinity clones. WO 9607754 (published 14 Mar. 1996) described a method for inducing mutagenesis in a complementarity determining region of an immunoglobulin light chain to create a library of light chain genes. Another effective approach is to recombine the VH or VL domains selected by phage display with repertoires of naturally occurring V domain variants obtained from unimmunized donors and screen for higher affinity in several rounds of chain reshuffling as described in Marks et al., Biotechnol., 10: 779-783 (1992). This technique allows the production of antibodies and antibody fragments with affinities in the 10-9 M range.

Other methods of generating and assessing the affinity of antibodies are well known in the art and are described, e.g., in Kohler et al., Nature 256: 495 (1975); U.S. Pat. No. 4,816,567; Goding, Monoclonal Antibodies: Principles and Practice, pp. 59-103 (Academic Press, 1986; Kozbor, J. Immunol., 133:3001 (1984); Brodeur et al., Monoclonal Antibody Production Techniques and Applications, pp. 51-63 (Marcel Dekker, Inc., New York, 1987; Munson et al., Anal. Biochem., 107:220 (1980); Engels et al., Agnew. Chem. Int. Ed. Engl., 28: 716-734 (1989); Abrahmsen et al., EMBO J., 4: 3901 (1985); Methods in Enzymology, vol. 44 (1976); Morrison et al., Proc. Natl. Acad. Sci. USA, 81: 6851-6855 (1984).

### General Methods

Generation of antibodies can be achieved using routine skills in the art, including those described herein, such as the hybridoma technique and screening of phage displayed libraries of binder molecules. These methods are well-established in the art.

Briefly, antibodies of the invention can be made by using combinatorial libraries to screen for synthetic antibody clones with the desired activity or activities. In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the antibodies of the invention can be obtained by designing a suitable antigen screening procedure to select for the phage clone of interest followed by construction of a full length antibody clone using the Fv sequences from the phage clone of interest and suitable constant region (Fc) sequences described in Kabat et al., Sequences of Proteins of Immunological Interest, Fifth Edition, NIH Publication 91-3242, Bethesda Md. (1991), vols. 1-3.

Monoclonal antibodies can be obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies.

The monoclonal antibodies of the invention can be made using a variety of methods known in the art, including the hybridoma method first described by Kohler et al., Nature, 256:495 (1975), or alternatively they may be made by recombinant DNA methods (e.g., U.S. Pat. No. 4,816,567).

### Vectors, Host Cells and Recombinant Methods

For recombinant production of an antibody of the invention, the nucleic acid encoding it is isolated and inserted into a replicable vector for further cloning (amplification of the DNA) or for expression. DNA encoding the antibody is readily isolated and sequenced using conventional procedures (e.g., by using oligonucleotide probes that are capable of binding specifically to genes encoding the heavy and light chains of the antibody). Many vectors are available. The choice of vector depends in part on the host cell to be used. Host cells include, but are not limited to, cells of either prokaryotic or eukaryotic (generally mammalian) origin. It will be appreciated that constant regions of any isotype can be used for this purpose, including IgG, IgM, IgA, IgD, and IgE constant regions, and that such constant regions can be obtained from any human or animal species.

### Generating Antibodies Using Prokaryotic Host Cells

### Vector Construction

Polynucleotide sequences encoding polypeptide components of the antibody of the invention can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR322, a plasmid derived from an E. coli species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Pat. No. 5,648,237.

In addition, phage vectors containing replicon and control sequences that are compatible with the host microorganism can be used as transforming vectors in connection with these hosts. For example, bacteriophage such as λGEM™-11 may be utilized in making a recombinant vector which can be used to transform susceptible host cells such as E. coli LE392.

The expression vector may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, e.g. the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector. Both the native promoter sequence and many heterologous promoters may be used to direct amplification and/or expression of the target genes. Heterologous promotersmay be utilized, as they generally permit greater transcription and higher yields of expressed target gene as compared to the native target polypeptide promoter.

Promoters suitable for use with prokaryotic hosts include the PhoA promoter, the β-galactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al. (1980) Cell 20: 269) using linkers or adaptors to supply any required restriction sites.

Each cistron within the recombinant vector may comprise a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, Ipp, or heat-stable enterotoxin II (STII) leaders, LamB, PhoE, PelB, OmpA and MBP. The signal sequences used in both cistrons of the expression system may be STII signal sequences or variants thereof.

The production of the immunoglobulins can occur in the cytoplasm of the host cell, and therefore does not require the presence of secretion signal sequences within each cistron. In that regard, immunoglobulin light and heavy chains are expressed, folded and assembled to form functional immunoglobulins within the cytoplasm. Certain host strains (e.g., the E. coli trxB- strains) provide cytoplasm conditions that are favorable for disulfide bond formation, thereby permitting proper folding and assembly of expressed protein subunits. Proba and Pluckthun Gene, 159:203 (1995).

Antibodies of the invention can also be produced by using an expression system in which the quantitative ratio of expressed polypeptide components can be modulated in order to maximize the yield of secreted and properly assembled antibodies of the invention. Such modulation is accomplished at least in part by simultaneously modulating translational strengths for the polypeptide components.

One technique for modulating translational strength is disclosed in Simmons et al., U.S. Pat. No. 5,840,523. It utilizes variants of the translational initiation region (TIR) within a cistron. For a given TIR, a series of amino acid or nucleic acid sequence variants can be created with a range of translational strengths, thereby providing a convenient means by which to adjust this factor for the desired expression level of the specific chain. TIR variants can be generated by conventional mutagenesis techniques that result in codon changes which can alter the amino acid sequence. Changes in the nucleotide sequencemay be silent. Alterations in the TIR can include, for example, alterations in the number or spacing of Shine-Dalgarno sequences, along with alterations in the signal sequence. One method for generating mutant signal sequences is the generation of a "codon bank" at the beginning of a coding sequence that does not change the amino acid sequence of the signal sequence (i.e., the changes are silent). This can be accomplished by changing the third nucleotide position of each codon; additionally, some amino acids, such as leucine, serine, and arginine, have multiple first and second positions that can add complexity in making the bank. This method of mutagenesis is described in detail in Yansura et al. (1992) METHODS: A Companion to Methods in Enzymol. 4:151-158.

A set set of vectors may be generated with a range of TIR strengths for each cistron therein. This limited set provides a comparison of expression levels of each chain as well as the yield of the desired antibody products under various TIR strength combinations. TIR strengths can be determined by quantifying the expression level of a reporter gene as described in detail in Simmons et al. U.S. Pat. No. 5,840,523. Based on the translational strength comparison, the desired individual TIRs are selected to be combined in the expression vector constructs.

Prokaryotic host cells suitable for expressing antibodies of the invention include Archaebacteria and Eubacteria, such as Gram-negative or Gram-positive organisms. Examples of useful bacteria include Escherichia (e.g., E. coli), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. Gram-negative cells may be used. E. coli cells may be used as hosts. Examples of E. coli strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ(nmpc-fepE) degP41 kanR (U.S. Pat. No. 5,639,635). Other strains and derivatives thereof, such as E. coli 294 (ATCC 31,446), E. coli B, E. coli λ1776 (ATCC 31,537) and E. coli RV308 (ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, E. coli, Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture.

### Antibody Production

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic cells used to produce the polypeptides of the invention are grown in media known in the art and suitable for culture of the selected host cells. Examples of suitable media include luria broth (LB) plus necessary nutrient supplementsThe media may also contain a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For E. coli growth, for example, growth occurs at a temperature range including, but not limited to, about 20° C. to about 39° C., about 25° C. to about 37° C., and at about 30° C. The pH of the medium may be any pH ranging from about 5 to about 9, depending mainly on the host organism. For E. coli, the pH can be from about 6.8 to about 7.4, or about 7.0.

If an inducible promoter is used in the expression, protein expression is induced under conditions suitable for the activation of the promoter. PhoA promoters may be used for controlling transcription of the polypeptides. Accordingly, the transformed host cells are cultured in a phosphate-limiting medium for induction. The phosphate-limiting medium may be the C.R.A.P medium (see, e.g., Simmons et al., J. Immunol. Methods (2002), 263:133-147). A variety of other inducers may be used, according to the vector construct employed, as is known in the art.

The expressed polypeptides of the present invention may be secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

Antibody production is conducted in large quantity by a fermentation process. Various large-scale fed-batch fermentation procedures are available for production of recombinant proteins. Large-scale fermentations have at least 1000 liters of capacity, for example about 1,000 to 100,000 liters of capacity. These fermentors use agitator impellers to distribute oxygen and nutrients, especially glucose (a common carbon/energy source). Small scale fermentation refers generally to fermentation in a fermentor that is no more than approximately 100 liters in volumetric capacity, and can range from about 1 liter to about 100 liters.

In a fermentation process, induction of protein expression is typically initiated after the cells have been grown under suitable conditions to a desired density, e.g., an OD550 of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides of the invention, various fermentation conditions can be modified. For example, to improve the proper assembly and folding of the secreted antibody polypeptides, additional vectors overexpressing chaperone proteins, such as Dsb proteins (DsbA, DsbB, DsbC, DsbD and or DsbG) or FkpA (a peptidylprolyl cis,trans-isomerase with chaperone activity) can be used to co-transform the host prokaryotic cells. The chaperone proteins have been demonstrated to facilitate the proper folding and solubility of heterologous proteins produced in bacterial host cells. Chen et al. (1999) J Bio Chem 274:19601-19605; Georgiou et al., U.S. Pat. No. 6,083,715; Georgiou et al., U.S. Pat. No. 6,027,888; Bothmann and Pluckthun (2000) J. Biol. Chem. 275:17100-17105; Ramm and Pluckthun (2000) J. Biol. Chem. 275:17106-17113; Arie et al. (2001) Mol. Microbiol. 39:199-210.

To minimize proteolysis of expressed heterologous proteins (especially those that are proteolytically sensitive), certain host strains deficient for proteolytic enzymes can be used. For example, host cell strains may be modified to effect genetic mutation(s) in the genes encoding known bacterial proteases such as Protease III, OmpT, DegP, Tsp, Protease I, Protease Mi, Protease V, Protease VI and combinations thereof. Some E. coli proteasedeficient strains are available and described in, for example, Joly et al. (1998), supra; Georgiou et al., U.S. Pat. No. 5,264,365; Georgiou et al., U.S. Pat. No. 5,508,192; Hara et al., Microbial Drug Resistance, 2:63-72 (1996).

E. coli strains deficient for proteolytic enzymes and transformed with plasmids overexpressing one or more chaperone proteins may be used as host cells in the expression system.

### Antibody Purification

The antibody protein produced herein may be further purified to obtain preparations that are substantially homogeneous for further assays and uses. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75.

Protein A immobilized on a solid phase is used for immunoaffinity purification of the antibody products of the invention. Protein A is a 41 kD cell wall protein from Staphylococcus aureas which binds with a high affinity to the Fc region of antibodies. Lindmark et al (1983) J. Immunol. Meth. 62:1-13. The solid phase to which Protein A is immobilized can be a column comprising a glass or silica surface, or a controlled pore glass column or a silicic acid column. In some applications, the column is coated with a reagent, such as glycerol, to possibly prevent nonspecific adherence of contaminants.

As the first step of purification, the preparation derived from the cell culture as described above can be applied onto a Protein A immobilized solid phase to allow specific binding of the antibody of interest to Protein A. The solid phase would then be washed to remove contaminants non-specifically bound to the solid phase. Finally the antibody of interest is recovered from the solid phase by elution.

### Generating Antibodies Using Eukaryotic Host Cells

The vector components generally include, but are not limited to, one or more of the following: a signal sequence, an origin of replication, one or more marker genes, an enhancer element, a promoter, and a transcription termination sequence.

### (i) Signal Sequence Component

A vector for use in a eukaryotic host cell may also contain a signal sequence or other polypeptide having a specific cleavage site at the N-terminus of the mature protein or polypeptide of interest. The heterologous signal sequence selected generally is one that is recognized and processed (i.e., cleaved by a signal peptidase) by the host cell. In mammalian cell expression, mammalian signal sequences as well as viral secretory leaders, for example, the herpes simplex gD signal, are available.

The DNA for such precursor region is ligated in reading frame to DNA encoding the antibody.

### (ii) Origin of Replication

Generally, an origin of replication component is not needed for mammalian expression vectors. For example, the SV40 origin may typically be used only because it contains the early promoter.

### (iii) Selection Gene Component

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, where relevant, or (c) supply critical nutrients not available from complex media.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II (e.g., primate metallothionein genes), adenosine deaminase, ornithine decarboxylase, etc.

For example, cells transformed with the DHFR selection gene may first be identified by culturing all of the transformants in a culture medium that contains methotrexate (Mtx), a competitive antagonist of DHFR. Appropriate host cells when wild-type DHFR is employed include, for example, the Chinese hamster ovary (CHO) cell line deficient in DHFR activity (e.g., ATCC CRL-9096).

Alternatively, host cells (particularly wild-type hosts that contain endogenous DHFR) transformed or co-transformed with DNA sequences encoding an antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Pat. No. 4,965,199.

### (iv) Promoter Component

Expression and cloning vectors usually contain a promoter that is recognized by the host organism and is operably linked to nucleic acid encoding a polypeptide of interest (e.g., an antibody). Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Antibody polypeptide transcription from vectors in mammalian host cells can be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, or from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., Nature 297:598-601 (1982) on expression of human β-interferon cDNA in mouse cells under the control of a thymidine kinase promoter from herpes simplex virus. Alternatively, the Rous Sarcoma Virus long terminal repeat can be used as the promoter.

### (v) Enhancer Element Component

Transcription of DNA encoding an antibody polypeptide of the invention by higher eukaryotes can often be increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the antibody polypeptide-encoding sequence, but is generally located at a site 5' from the promoter.

### (vi) Transcription Termination Component

Expression vectors used in eukaryotic host cells will typically also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding an antibody. One useful transcription termination component is the bovine growth hormone polyadenylation region. See WO94/11026 and the expression vector disclosed therein.

### (vii) Selection and Transformation of Host Cells

Suitable host cells for cloning or expressing the DNA in the vectors herein include higher eukaryote cells described herein, including vertebrate host cells. Propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

### (viii) Culturing the Host Cells

The host cells used to produce an antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Pat. Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Purification of Antibody

When using recombinant techniques, the antibody can be produced intracellularly, or directly secreted into the medium. If the antibody is produced intracellularly, as a first step, the particulate debris, either host cells or lysed fragments, are generally removed, for example, by centrifugation or ultrafiltration. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being a generally acceptable purification technique. The suitability of affinity reagents such as protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Meth. 62:1-13 (1983)). Protein G is recommended for all mouse isotypes and for human y3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX™ resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to further purification steps, as necessary, for example by low pH hydrophobic interaction chromatography using an elution buffer at a pH between about 2.5-4.5, generally performed at low salt concentrations (e.g., from about 0-0.25M salt).

It should be noted that, in general, techniques and methodologies for preparing antibodies for use in research, testing and clinical use are well-established in the art, consistent with the above and/or as deemed appropriate by one skilled in the art for the particular antibody of interest.

### Activity Assays

Antibodies of the invention can be characterized for their physical/chemical properties and biological functions by various assays known in the art.

Purified antibodies can be further characterized by a series of assays including, but not limited to, N-terminal sequencing, amino acid analysis, non-denaturing size exclusion high pressure liquid chromatography (HPLC), mass spectrometry, ion exchange chromatography and papain digestion.

Where necessary, antibodies are analyzed for their biological activity. Antibodies of the invention may be tested for their antigen binding activity. The antigen binding assays that are known in the art and can be used herein include without limitation any direct or competitive binding assays using techniques such as western blots, radioimmunoassays, ELISA (enzyme linked immunosorbent assay), "sandwich" immunoassays, immunoprecipitation assays, fluorescent immunoassays, and protein A immunoassays.

In one embodiment, the invention contemplates an altered antibody that possesses some but not all effector functions, which make it a desirable candidate for many applications in which the half life of the antibody in vivo is important yet certain effector functions (such as complement and ADCC) are unnecessary or deleterious. The Fc activities of the antibody may be measured to ensure that only the desired properties are maintained. In vitro and/or in vivo cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcyRIII only, whereas monocytes express FcyRI, FcyRII and FcyRIII. FcR expression on hematopoietic cells is summarized in Table 3 on page 464 of Ravetch and Kinet, Annu. Rev. Immunol 9:457-92 (1991). An example of an in vitro assay to assess ADCC activity of a molecule of interest is described in U.S. Pat. No. 5,500,362 or U.S. Pat. No. 5,821,337. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. PNAS (USA) 95:652-656 (1998). C1q binding assays may also be carried out to confirm that the antibody is unable to bind C1q and hence lacks CDC activity. To assess complement activation, a CDC assay, e.g. as described in Gazzano-Santoro et al., J. Immunol. Methods 202:163 (1996), may be performed. FcRn binding and in vivo clearance/half life determinations can also be performed using methods known in the art.

### Antibody Fragments

The present invention encompasses antibody fragments. In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from E. coli, thus allowing the facile production of large amounts of these fragments. Antibody fragments can be isolated from the antibody phage libraries discussed above. Alternatively, Fab'-SH fragments can be directly recovered from E. coli and chemically coupled to form F(ab')2 fragments (Carter et al., Bio/Technology 10: 163-167 (1992)). According to another approach, F(ab')2 fragments can be isolated directly from recombinant host cell culture. Fab and F(ab')2 fragment with increased in vivo half-life comprising salvage receptor binding epitope residues are described in U.S. Pat. No. 5,869,046. Other techniques for the production of antibody fragments will be apparent to the skilled practitioner. In other embodiments, the antibody of choice is a single chain Fv fragment (scFv). See WO 93/16185; U.S. Pat. Nos. 5,571,894; and 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Pat. No. 5,641,870 for example. Such linear antibody fragments may be monospecific or bispecific.

### Humanized Antibodies

Any of the antibodies described herein can be a full length antibody or an antigen-binding fragment thereof. In some examples, the antigen binding fragment is a Fab fragment, a F(ab')2 fragment, or a single-chain Fv fragment. In some examples, the antigen binding fragment is a Fab fragment, a F(ab')2 fragment, or a single-chain Fv fragment. In some examples, the isolated antibody is a human antibody, a humanized antibody, a chimeric antibody, or a single-chain antibody.

Any of the antibodies described herein has one or more characteristics of:
a) is a recombinant antibody, a monoclonal antibody, a chimeric antibody, a humanized antibody, a human antibody, an antibody fragment, a bispecific antibody, a monospecific antibody, a monovalent antibody, an IgG1 antibody, an IgG2 antibody, or derivative of an antibody; b) is a human, murine, humanized, or chimeric antibody, antigen-binding fragment, or derivative of an antibody; c) is a single-chain antibody fragment, a multibody, a Fab fragment, and/or an immunoglobulin of the IgG, IgM, IgA, IgE, IgD isotypes and/or subclasses thereof; d) has one or more of the following characteristics: (i) mediates ADCC and/or CDC of cancer cells; (ii) induces and/or promotes apoptosis of cancer cells; (iii) inhibits proliferation of target cells of cancer cells; (iv) induces and/or promotes phagocytosis of cancer cells; and/or (v) induces and/or promotes the release of cytotoxic agents; e) specifically binds the tumor-associated carbohydrate antigen, which is a tumorspecific carbohydrate antigen; f) does not bind an antigen expressed on non-cancer cells, nontumor cells, benign cancer cells and/or benign tumor cells; and/or g) specifically binds a tumor-associated carbohydrate antigen expressed on cancer stem cells and on normal cancer cells.

Preferably the binding of the antibodies to their respective antigens is specific. The term "specific" is generally used to refer to the situation in which one member of a binding pair will not show any significant binding to molecules other than its specific binding partner (s) and e.g. has less than about 30%, preferably 20%, 10%, or 1 % cross-reactivity with any other molecule other than those specified herein.

The antibodies are suitable bind to its target epitopes with a high affinity (low KD value), and preferably KD is in the nanomolar range or lower. Affinity can be measured by methods known in the art, such as, for example; surface plasmon resonance.

### Exemplary Antibody Preparation

Exemplary Antibodies capable of binding to the SSEA-4 epitopes described herein can be made by any method known in the art. See, for example, Harlow and Lane, (1988) Antibodies: A Laboratory Manual, Cold Spring Harbor Laboratory, New York.

### Immunization of Host Animals and Hybridoma Technology

Exemplary Polyclonal antibodies against the anti-SSEA-4 antibodies may be prepared by collecting blood from the immunized mammal examined for the increase of desired antibodies in the serum, and by separating serum from the blood by any conventional method. Polyclonal antibodies include serum containing the polyclonal antibodies, as well as the fraction containing the polyclonal antibodies may be isolated from the serum.

Polyclonal antibodies are generally raised in host animals (e.g., rabbit, mouse, horse, or goat) by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, etc.

Any mammalian animal may be immunized with the antigen for producing the desired antibodies. In general, animals of Rodentia, Lagomorpha, or Primates can be used. Animals of Rodentia include, for example, mouse, rat, and hamster. Animals of Lagomorpha include, for example, rabbit. Animals of Primates include, for example, a monkey of Catarrhini (old world monkey) such as Macaca fascicularis, rhesus monkey, baboon, and chimpanzees.

Methods for immunizing animals with antigens are known in the art. Intraperitoneal injection or subcutaneous injection of antigens is a standard method for immunization of mammals. More specifically, antigens may be diluted and suspended in an appropriate amount of phosphate buffered saline (PBS), physiological saline, etc. If desired, the antigen suspension may be mixed with an appropriate amount of a standard adjuvant, such as Freund's complete adjuvant, made into emulsion, and then administered to mammalian animals. Animals are immunized against the antigen, immunogenic conjugates, or derivatives by combining 1 mg or 1 µg of the peptide or conjugate (for rabbits or mice, respectively) with 3 volumes of Freund's incomplete adjuvant.

Animals can be boosted until the titer plateaus by several administrations of antigen mixed with an appropriately amount of Freund's incomplete adjuvant every 4 to 21 days. Animals are boosted with 1/5 to 1/10 the original amount of peptide or conjugate in Freund's complete adjuvant by subcutaneous injection at multiple sites. Seven to 14 days later the animals are bled and the serum is assayed for antibody titer. An appropriate carrier may also be used for immunization. After immunization as above, serum is examined by a standard method for an increase in the amount of desired antibodies. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Over the past two to three decades, a number of methodologies have been developed to prepare chimeric, humanized or human antibodies for human in-vivo therapeutic applications. The most used and proven methodology is to prepare mouse mAbs using hybridoma methodology and then to humanize the mAbs by converting the framework regions of the V_{H} and V_{L} domains and constant domains of the mAbs into most homologous human framework regions of human V_{H} and V_{L} domains and constant regions of a desirable human y immunoglobulin isotype and subclass. Many mAbs, such as Xolair, used clinically are humanized mAbs of human γ1, κ isotype and subclass and prepared using this methodology.

Aantibodies may be be made by the conventional hybridoma technology. Kohler et al., Nature, 256:495 (1975). In the hybridoma method, a mouse or other appropriate host animal, such as a hamster or rabbit, is immunized as hereinabove described to elicit lymphocytes that produce or are capable of producing antibodies that will specifically bind to the protein used for immunization. Alternatively, lymphocytes may be immunized in vitro.

To prepare monoclonal antibodies, immune cells are collected from the mammal immunized with the antigen and checked for the increased level of desired antibodies in the serum as described above, and are subjected to cell fusion. The immune cells used for cell fusion are preferably obtained from spleen. Other preferred parental cells to be fused with the above immunocyte include, for example, myeloma cells of mammalians, and more preferably myeloma cells having an acquired property for the selection of fused cells by drugs.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, Calif. USA, and SP-2 cells available from the American Type Culture Collection, Rockville, Md. USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

The above immunocyte and myeloma cells can be fused according to known methods, for example, the method of Milstein et al. (Galfre et al., Methods Enzymol. 73:3-46, 1981). Lymphocytes are fused with myeloma cells using a suitable fusing agent, such as polyethylene glycol, to form a hybridoma cell (Goding, Monoclonal Antibodies: Principles and Practice, pp.59-103 (Academic Press, 1986)). Resulting hybridomas obtained by the cell fusion may be selected by cultivating them in a standard selection medium, such as HAT medium (hypoxanthine, aminopterin, and thymidine containing medium). The cell culture is typically continued in the HAT medium for several days to several weeks, the time being sufficient to allow all the other cells, with the exception of the desired hybridoma (non-fused cells), to die. Then, the standard limiting dilution is performed to screen and clone a hybridoma cell producing the desired antibody.

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay. Measurement of absorbance in enzyme-linked immunosorbent assay (ELISA), enzyme immunoassay (EIA), radioimmunoassay (RIA), and/or immunofluorescence may be used to measure the antigen binding activity of the antibody of the invention. In ELISA, the antibody of the present invention is immobilized on a plate, protein of the invention is applied to the plate, and then a sample containing a desired antibody, such as culture supernatant of antibody producing cells or purified antibodies, is applied. Then, a secondary antibody that recognizes the primary antibody and is labeled with an enzyme, such as alkaline phosphatase, is applied, and the plate is incubated. Next, after washing, an enzyme substrate, such as p-nitrophenyl phosphate, is added to the plate, and the absorbance is measured to evaluate the antigen binding activity of the sample. A fragment of the protein, such as a C-terminal or N-terminal fragment may be used in this method. BIAcore (Pharmacia) may be used to evaluate the activity of the antibody according to the present invention. The binding affinity of the monoclonal antibody can, for example, be determined by the Scatchard analysis of Munson et al., Anal. Biochem., 107:220 (1980).

Applying any of the conventional methods, including those described above, hybridoma cells producing antibodies that bind to epitopes described herein can be identified and selected for further characterization.

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

In addition, the hybridoma cells may be grown in vivo as ascites tumors in an animal. For example, the obtained hybridomas can be subsequently transplanted into the abdominal cavity of a mouse and the ascites are harvested.

The obtained monoclonal antibodies can be purified by, for example, ammonium sulfate precipitation, a protein A or protein G column, DEAE ion exchange chromatography, or an affinity column to which the protein of the present invention is coupled. The antibody of the present invention can be used not only for purification and detection of the protein of the present invention, but also as a candidate for agonists and antagonists of the protein of the present invention. In addition, this antibody can be applied to the antibody treatment for diseases related to the protein of the present invention.

### Recombinant Technology

The monoclonal antibodies thus obtained can be also recombinantly prepared using genetic engineering techniques (see, for example, Borrebaeck C. A. K. and Larrick J. W. Therapeutic Monoclonal Antibodies, published in the United Kingdom by MacMillan Publishers LTD, 1990). A DNA encoding an antibody may be cloned from an immune cell, such as a hybridoma or an immunized lymphocyte producing the antibody, inserted into an appropriate vector, and introduced into host cells to prepare a recombinant antibody. The present invention also provides recombinant antibodies prepared as described above.

When the obtained antibody is to be administered to the human body (antibody treatment), a human antibody or a humanized antibody is preferable for reducing immunogenicity. For example, transgenic animals having a repertory of human antibody genes may be immunized with an antigen selected from a protein, protein expressing cells, or their lysates. Antibody producing cells are then collected from the animals and fused with myeloma cells to obtain hybridoma, from which human antibodies against the protein can be prepared. Alternatively, an immune cell, such as an immunized lymphocyte, producing antibodies may be immortalized by an oncogene and used for preparing monoclonal antibodies.

DNA encoding the monoclonal antibodies can be readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Pluckthun, Immunol. Rev., 130:151-188 (1992).

DNAs encoding the antibodies produced by the hybridoma cells described above can be genetically modified, via routine technology, to produce genetically engineered antibodies. Genetically engineered antibodies, such as humanized antibodies, chimeric antibodies, single-chain antibodies, and bi-specific antibodies, can be produced via, e.g., conventional recombinant technology. The DNA can then be modified, for example, by substituting the coding sequence for human heavy and light chain constant domains in place of the homologous murine sequences, Morrison et al., (1984) Proc. Nat. Acad. Sci. 81:6851, or by covalently joining to the immunoglobulin coding sequence all or part of the coding sequence for a non-immunoglobulin polypeptide. In that manner, genetically engineered antibodies, such as "chimeric" or "hybrid" antibodies; can be prepared that have the binding specificity of a target antigen.

Techniques developed for the production of "chimeric antibodies" are well known in the art. See, e.g., Morrison et al. (1984) Proc. Natl. Acad. Sci. USA 81, 6851; Neuberger et al. (1984) Nature 312, 604; and Takeda et al. (1984) Nature 314:452.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

Chimeric or hybrid antibodies also may be prepared in vitro using known methods in synthetic protein chemistry, including those involving crosslinking agents. For example, immunotoxins may be constructed using a disulfide-exchange reaction or by forming a thioether bond. Examples of suitable reagents for this purpose include iminothiolate and methyl-4-mercaptobutyrimidate.

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (Carter et al., Proc. Natl. Acad Sci. USA, 89:4285 (1992); Prestaetal., J. Immnol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i. e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggermann et al., Year in Immuno., 7:33 (1993). Human antibodies can also be derived from phage-display libraries (Hoogenboom et al., J. Mol. Biol., 227:381 (1991); Marks et al., J. Mol. Biol., 222:581-597 (1991)).

In some examples, a vector comprising a nucleic acid comprising a nucleotide sequence encoding either the heavy chain variable region or the light chain variable region of an anti-SSEA-4 antibody as described herein.In other examples, the vector comprises nucleotide sequences encoding both the heavy chain variable region and the light chain variable region, the expression of which can be controlled by a single promoter or two separate promoters. Also provided here are methods for producing any of anti-SSEA-4 antibodies as described herein, e.g., via the recombinant technology described in this section.

### Other Technology for Preparing Antibodies

Fully human antibodies can be obtained by using commercially available mice that have been engineered to express specific human immunoglobulin proteins. Transgenic animals that are designed to produce a more desirable (e.g., fully human antibodies) or more robust immune response may also be used for generation of humanized or human antibodies. Examples of such technology are Xenomouse^{RTM} from Amgen, Inc. (Fremont, Calif.) and HuMAb-Mouse^{RTM} and TC Mouse™ from Medarex, Inc. (Princeton, N.J.). In another alternative, antibodies may be made recombinantly by phage display technology. See, for example, U.S. Pat. Nos. 5,565,332; 5,580,717; 5,733,743; and 6,265,150; and Winter et al., (1994) Annu. Rev. Immunol. 12:433-455. Alternatively, the phage display technology (McCafferty et al., (1990) Nature 348:552-553) can be used to produce human antibodies and antibody fragments in vitro, from immunoglobulin variable (V) domain gene repertoires from unimmunized donors.

Antigen-binding fragments of an intact antibody (full-length antibody) can be prepared via routine methods. For example, F(ab')2 fragments can be produced by pepsin digestion of an antibody molecule, and Fab fragments that can be generated by reducing the disulfide bridges of F(ab')2 fragments.

Alternatively, the anti-SSEA-4 antibodies described herein can be isolated from antibody phage libraries (e.g., single-chain antibody phage libraries) generated using the techniques described in McCafferty et al., Nature, 348:552-554 (1990). Clackson et al., Nature, 352:624-628 (1991) and Marks et al., J. Mol Biol., 222:581-597 (1991). Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

Antibodies obtained as described herein may be purified to homogeneity. For example, the separation and purification of the antibody can be performed according to separation and purification methods used for general proteins. For example, the antibody may be separated and isolated by the appropriately selected and combined use of column chromatographies, such as affinity chromatography, filter, ultrafiltration, salting-out, dialysis, SDS polyacrylamide gel electrophoresis, isoelectric focusing, and others (Antibodies: A Laboratory Manual. Ed Harlow and David Lane, Cold Spring Harbor Laboratory, 1988), but are not limited thereto. The concentration of the antibodies obtained as above may be determined by the measurement of absorbance, Enzyme-linked immunosorbent assay (ELISA), or so on. Exemplary chromatography, with the exception of affinity includes, for example, ion-exchange chromatography, hydrophobic chromatography, gel filtration, reversephase chromatography, adsorption chromatography, and the like (Strategies for Protein Purification and Characterization: A Laboratory Course Manual. Ed Daniel R. Marshak et al., Cold Spring Harbor Laboratory Press, 1996). The chromatographic procedures can be carried out by liquid-phase chromatography, such as HPLC, FPLC.

The antibodies can be characterized using methods well known in the art. For example, one method is to identify the epitope to which the antigen binds, or "epitope mapping." There are many methods known in the art for mapping and characterizing the location of epitopes on proteins, including solving the crystal structure of an antibody-antigen complex, competition assays, gene fragment expression assays, and synthetic peptide-based assays, as described, for example, in Chapter 11 of Harlow and Lane, Using Antibodies, a Laboratory Manual, Cold Spring Harbor Laboratory Press, Cold Spring Harbor, N.Y., 1999. In an additional example, epitope mapping can be used to determine the sequence to which an antibody binds. The epitope can be a linear epitope, i.e., contained in a single stretch of amino acids, or a conformational epitope formed by a three-dimensional interaction of amino acids that may not necessarily be contained in a single stretch (primary structure linear sequence). Peptides of varying lengths (e.g., at least 4-6 amino acids long) can be isolated or synthesized (e.g., recombinantly) and used for binding assays with an antibody. In another example, the epitope to which the antibody binds can be determined in a systematic screening by using overlapping peptides derived from the target antigen sequence and determining binding by the antibody. According to the gene fragment expression assays, the open reading frame encoding the target antigen is fragmented either randomly or by specific genetic constructions and the reactivity of the expressed fragments of the antigen with the antibody to be tested is determined. The gene fragments may, for example, be produced by PCR and then transcribed and translated into protein in vitro, in the presence of radioactive amino acids. The binding of the antibody to the radioactively labeled antigen fragments is then determined by immunoprecipitation and gel electrophoresis. Certain epitopes can also be identified by using large libraries of random peptide sequences displayed on the surface of phage particles (phage libraries). Alternatively, a defined library of overlapping peptide fragments can be tested for binding to the test antibody in simple binding assays.

In an additional example, mutagenesis of an antigen binding domain, domain swapping experiments and alanine scanning mutagenesis can be performed to identify residues required, sufficient, and/or necessary for epitope binding. For example, domain swapping experiments can be performed using a mutant of a target antigen in which various residues in the binding epitope for the candidate antibody have been replaced (swapped) with sequences from a closely related, but antigenically distinct protein (such as another member of the neurotrophin protein family). By assessing binding of the antibody to the mutant target protein, the importance of the particular antigen fragment to antibody binding can be assessed.

Alternatively, competition assays can be performed using other antibodies known to bind to the same antigen to determine whether an antibody binds to the same epitope (e.g., the MC45 antibody described herein) as the other antibodies. Competition assays are well known to those of skill in the art.

### Additional Aspects of Exemplary suitable General Antibody Production Methods

Methods of making monoclonal and polyclonal antibodies and fragments thereof in animals (e.g., mouse, rabbit, goat, sheep, or horse) are well known in the art. See, for example, Harlow and Lane, (1988) Antibodies: A Laboratory Manual, Cold Spring Harbor Laboratory, New York. The term "antibody" includes intact immunoglobulin molecules as well as fragments thereof, such as Fab, F(ab')2, Fv, scFv (single chain antibody), and dAb (domain antibody; Ward, et. al. (1989) Nature, 341, 544).

The compositions disclosed herein can be included in a pharmaceutical composition together with additional active agents, carriers, vehicles, excipients, or auxiliary agents identifiable by a person skilled in the art upon reading of the present disclosure.

The pharmaceutical compositions preferably comprise at least one pharmaceutically acceptable carrier. In such pharmaceutical compositions, the compositions disclosed herein form the "active compound," also referred to as the "active agent." As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions. A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol, or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates, or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

Compositions comprising at least one /SSEA-4 antibody or at least one polynucleotide comprising sequences encoding an SSEA-4 antibody are provided. In certain embodiments, a composition may be a pharmaceutical composition. As used herein, compositions comprise one or more antibodies that bind to one or more SSEA-4 and/or one or more polynucleotides comprising sequences encoding one or more antibodies that bind to one or more SSEA-4. These compositions may further comprise suitable carriers, such as pharmaceutically acceptable excipients including buffers, which are well known in the art.

Isolated antibodies and polynucleotides are also provided. In certain embodiments, the isolated antibodies and polynucleotides are substantially pure.

In one embodiment, anti-SSEA-4/ antibodies are monoclonal. In another embodiment, fragments of the anti-SSEA-4 antibodies (e.g., Fab, Fab'-SH and F(ab')2 fragments) are provided. These antibody fragments can be created by traditional means, such as enzymatic digestion, or may be generated by recombinant techniques. Such antibody fragments may be chimeric, humanized, or human. These fragments are useful for the diagnostic and therapeutic purposes set forth below.

A variety of methods are known in the art for generating phage display libraries from which an antibody of interest can be obtained. One method of generating antibodies of interest is through the use of a phage antibody library as described in Lee et al., J. Mol. Biol. (2004), 340(5): 1073-93.

The anti-SSEA-4 antibodies of the invention can be made by using combinatorial libraries to screen for synthetic antibody clones with the desired activity or activities. In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the anti-SSEA-4 antibodies of the invention can be obtained by designing a suitable antigen screening procedure to select for the phage clone of interest followed by construction of a full length anti- SSEA-4 antibody clone using the Fv sequences from the phage clone of interest and suitable constant region (Fc) sequences described in Kabat et al., Sequences of Proteins of Immunological Interest, Fifth Edition, NIH Publication 91-3242, Bethesda Md. (1991), vols. 1-3.

The antigen-binding domain of an antibody is formed from two variable (V) regions of about 110 amino acids, one each from the light (VL) and heavy (VH) chains, that both present three hypervariable loops or complementarity-determining regions (CDRs). Variable domains can be displayed functionally on phage, either as single-chain Fv (scFv) fragments, in which VH and VL are covalently linked through a short, flexible peptide, or as Fab fragments, in which they are each fused to a constant domain and interact non-covalently, as described in Winter et al., Ann. Rev. Immunol., 12: 433-455 (1994). As used herein, scFv encoding phage clones and Fab encoding phage clones are collectively referred to as "Fv phage clones" or "Fv clones".

Repertoires of VH and VL genes can be separately cloned by polymerase chain reaction (PCR) and recombined randomly in phage libraries, which can then be searched for antigen-binding clones as described in Winter et al., Ann. Rev. Immunol., 12: 433-455 (1994). Libraries from immunized sources provide high-affinity antibodies to the immunogen without the requirement of constructing hybridomas. Alternatively, the naive repertoire can be cloned to provide a single source of human antibodies to a wide range of non-self and also self antigens without any immunization as described by Griffiths et al., EMBO J, 12: 725-734 (1993). Finally, naive libraries can also be made synthetically by cloning the unrearranged V-gene segments from stem cells, and using PCR primers containing random sequence to encode the highly variable CDR3 regions and to accomplish rearrangement in vitro as described by Hoogenboom and Winter, J. Mol. Biol., 227: 381-388 (1992).

Filamentous phage is used to display antibody fragments by fusion to the minor coat protein pill. The antibody fragments can be displayed as single chain Fv fragments, in which VH and VL domains are connected on the same polypeptide chain by a flexible polypeptide spacer, e.g. as described by Marks et al., J. Mol. Biol., 222: 581-597 (1991), or as Fab fragments, in which one chain is fused to pill and the other is secreted into the bacterial host cell periplasm where assembly of a Fab-coat protein structure which becomes displayed on the phage surface by displacing some of the wild type coat proteins, e.g. as described in Hoogenboom et al., Nucl. Acids Res., 19: 4133-4137 (1991).

In general, nucleic acids encoding antibody gene fragments are obtained from immune cells harvested from humans or animals. If a library biased in favor of anti- SSEA-4 clones is desired, the subject is immunized with SSEA-4 H to generate an antibody response, and spleen cells and/or circulating B cells or other peripheral blood lymphocytes (PBLs) are recovered for library construction. In one embodiment, a human antibody gene fragment library biased in favor of anti-human /SSEA-4 clones is obtained by generating an anti-human SSEA-4 antibody response in transgenic mice carrying a functional human immunoglobulin gene array (and lacking a functional endogenous antibody production system) such that SSEA-4 immunization gives rise to B cells producing human antibodies against SSEA-4. The generation of human antibody-producing transgenic mice is described below.

Additional enrichment for anti-SSEA-4 reactive cell populations can be obtained by using a suitable screening procedure to isolate B cells expressing SSEA-4 -specific antibody, e.g., by cell separation with SSEA-4 affinity chromatography or adsorption of cells to fluorochrome-labeled SSEA-4 followed by flow-activated cell sorting (FACS).

Alternatively, the use of spleen cells and/or B cells or other PBLs from an unimmunized donor provides a better representation of the possible antibody repertoire, and also permits the construction of an antibody library using any animal (human or non-human) species in which SSEA-4 is not antigenic. For libraries incorporating in vitro antibody gene construction, stem cells are harvested from the subject to provide nucleic acids encoding unrearranged antibody gene segments. The immune cells of interest can be obtained from a variety of animal species, such as human, mouse, rat, lagomorpha, luprine, canine, feline, porcine, bovine, equine, and avian species, etc.

Nucleic acid encoding antibody variable gene segments (including VH and VL segments) are recovered from the cells of interest and amplified. In the case of rearranged VH and VL gene libraries, the desired DNA can be obtained by isolating genomic DNA or mRNA from lymphocytes followed by polymerase chain reaction (PCR) with primers matching the 5' and 3' ends of rearranged VH and VL genes as described in Orlandi et al., Proc. Natl. Acad. Sci. (USA), 86: 3833-3837 (1989), thereby making diverse V gene repertoires for expression. The V genes can be amplified from cDNA and genomic DNA, with back primers at the 5' end of the exon encoding the mature V-domain and forward primers based within the J-segment as described in Orlandi et al. (1989) and in Ward et al., Nature, 341: 544-546 (1989). However, for amplifying from cDNA, back primers can also be based in the leader exon as described in Jones et al., Biotechnol., 9: 88-89 (1991), and forward primers within the constant region as described in Sastry et al., Proc. Natl. Acad. Sci. (USA), 86: 5728-5732 (1989). To maximize complementarity, degeneracy can be incorporated in the primers as described in Orlandi et al. (1989) or Sastry et al. (1989). Thehe library diversity may be maximized by using PCR primers targeted to each V-gene family in order to amplify all available VH and VL arrangements present in the immune cell nucleic acid sample, e.g. as described in the method of Marks et al., J. Mol. Biol., 222: 581-597 (1991) or as described in the method of Orum et al., Nucleic Acids Res., 21: 4491-4498 (1993). For cloning of the amplified DNA into expression vectors, rare restriction sites can be introduced within the PCR primer as a tag at one end as described in Orlandi et al. (1989), or by further PCR amplification with a tagged primer as described in Clackson et al., Nature, 352: 624-628 (1991).

Repertoires of synthetically rearranged V genes can be derived in vitro from V gene segments. Most of the human VH-gene segments have been cloned and sequenced (reported in Tomlinson et al., J. Mol. Biol., 227: 776-798 (1992)), and mapped (reported in Matsuda et al., Nature Genet., 3: 88-94 (1993); these cloned segments (including all the major conformations of the H1 and H2 loop) can be used to generate diverse VH gene repertoires with PCR primers encoding H3 loops of diverse sequence and length as described in Hoogenboom and Winter, J. Mol. Biol., 227: 381-388 (1992). VH repertoires can also be made with all the sequence diversity focused in a long H3 loop of a single length as described in Barbas et al., Proc. Natl. Acad. Sci. USA, 89: 4457-4461 (1992). Human VK and Vλ segments have been cloned and sequenced (reported in Williams and Winter, Eur. J. Immunol., 23: 1456-1461 (1993)) and can be used to make synthetic light chain repertoires. Synthetic V gene repertoires, based on a range of VH and VL folds, and L3 and H3 lengths, will encode antibodies of considerable structural diversity. Following amplification of V-gene encoding DNAs, germline V-gene segments can be rearranged in vitro according to the methods of Hoogenboom and Winter, J. Mol. Biol., 227: 381-388 (1992).

Repertoires of antibody fragments can be constructed by combining VH and VL gene repertoires together in several ways. Each repertoire can be created in different vectors, and the vectors recombined in vitro, e.g., as described in Hogrefe et al., Gene, 128: 119-126 (1993), or in vivo by combinatorial infection, e.g., the loxP system described in Waterhouse et al., Nucl. Acids Res., 21: 2265-2266 (1993). The in vivo recombination approach exploits the two-chain nature of Fab fragments to overcome the limit on library size imposed by E. coli transformation efficiency. Naive VH and VL repertoires are cloned separately, one into a phagemid and the other into a phage vector. The two libraries are then combined by phage infection of phagemid-containing bacteria so that each cell contains a different combination and the library size is limited only by the number of cells present (about 1012 clones). Both vectors contain in vivo recombination signals so that the VH and VL genes are recombined onto a single replicon and are co-packaged into phage virions. These huge libraries provide large numbers of diverse antibodies of good affinity (Kd-1 of about 10-8 M).

Alternatively, the repertoires may be cloned sequentially into the same vector, e.g. as described in Barbas et al., Proc. Natl. Acad. Sci. USA, 88: 7978-7982 (1991), or assembled together by PCR and then cloned, e.g. as described in Clackson et al., Nature, 352: 624-628 (1991). PCR assembly can also be used to join VH and VL DNAs with DNA encoding a flexible peptide spacer to form single chain Fv (scFv) repertoires. In yet another technique, "in cell PCR assembly" is used to combine VH and VL genes within lymphocytes by PCR and then clone repertoires of linked genes as described in Embleton et al., Nucl. Acids Res., 20: 3831-3837 (1992).

Screening of the libraries can be accomplished by any art-known technique. For example, SSEA-4 targets can be used to coat the wells of adsorption plates, expressed on host cells affixed to adsorption plates or used in cell sorting, or conjugated to biotin for capture with streptavidin-coated beads, or used in any other art-known method for panning phage display libraries.

The phage library samples are contacted with immobilized SSEA-4 H under conditions suitable for binding of at least a portion of the phage particles with the adsorbent. Normally, the conditions, including pH, ionic strength, temperature and the like are selected to mimic physiological conditions. The phages bound to the solid phase are washed and then eluted by acid, e.g. as described in Barbas et al., Proc. Natl. Acad. Sci. USA, 88: 7978-7982 (1991), or by alkali, e.g. as described in Marks et al., J. Mol. Biol., 222: 581-597 (1991), or by SSEA-4 antigen competition, e.g. in a procedure similar to the antigen competition method of Clackson et al., Nature, 352: 624-628 (1991). Phages can be enriched from about 20x to about 1,000-fold in a single round of selection. Moreover, the enriched phages can be grown in bacterial culture and subjected to further rounds of selection.

The efficiency of selection depends on many factors, including the kinetics of dissociation during washing, and whether multiple antibody fragments on a single phage can simultaneously engage with antigen. Antibodies with fast dissociation kinetics (and weak binding affinities) can be retained by use of short washes, multivalent phage display and high coating density of antigen in solid phase. The high density not only stabilizes the phage through multivalent interactions, but favors rebinding of phage that has dissociated. The selection of antibodies with slow dissociation kinetics (and good binding affinities) can be promoted by use of long washes and monovalent phage display as described in Bass et al., Proteins, 8: 309-314 (1990) and in WO 92/09690, and a low coating density of antigen as described in Marks et al., Biotechnol., 10: 779-783 (1992).

It is possible to select between phage antibodies of different affinities, even with affinities that differ slightly, for SSEA-4. However, random mutation of a selected antibody (e.g. as performed in some of the affinity maturation techniques described above) is likely to give rise to many mutants, most binding to antigen, and a few with higher affinity. With limiting SSEA-4, rare high affinity phage could be competed out. To retain all the higher affinity mutants, phages can be incubated with excess biotinylated SSEA-4, but with the biotinylated SSEA-4 at a concentration of lower molarity than the target molar affinity constant for /SSEA-4. The high affinity-binding phages can then be captured by streptavidin-coated paramagnetic beads. Such "equilibrium capture" allows the antibodies to be selected according to their affinities of binding, with sensitivity that permits isolation of mutant clones with as little as two-fold higher affinity from a great excess of phages with lower affinity. Conditions used in washing phages bound to a solid phase can also be manipulated to discriminate on the basis of dissociation kinetics.

DNA encoding the Fv clones of the invention is readily isolated and sequenced using conventional procedures (e.g. by using oligonucleotide primers designed to specifically amplify the heavy and light chain coding regions of interest from hybridoma or phage DNA template). Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of the desired monoclonal antibodies in the recombinant host cells. Review articles on recombinant expression in bacteria of antibody-encoding DNA include Skerra et al., Curr. Opinion in Immunol., 5: 256 (1993) and Pluckthun, Immunol. Revs, 130: 151 (1992).

DNA encoding the Fv clones of the invention can be combined with known DNA sequences encoding heavy chain and/or light chain constant regions (e.g. the appropriate DNA sequences can be obtained from Kabat et al., supra) to form clones encoding full or partial length heavy and/or light chains. It will be appreciated that constant regions of any isotype can be used for this purpose, including IgG, IgM, IgA, IgD, and IgE constant regions, and that such constant regions can be obtained from any human or animal species. A Fv clone derived from the variable domain DNA of one animal (such as human) species and then fused to constant region DNA of another animal species to form coding sequence(s) for "hybrid", full length heavy chain and/or light chain is included in the definition of "chimeric" and "hybrid" antibody as used herein. In one embodiment, a Fv clone derived from human variable DNA is fused to human constant region DNA to form coding sequence(s) for all human, full or partial length heavy and/or light chains.

The antibodies produced by naive libraries (either natural or synthetic) can be of moderate affinity (Kd -1 of about 106 to 107 M-1), but affinity maturation can also be mimicked in vitro by constructing and reselecting from secondary libraries as described in Winter et al. (1994), supra. For example, mutation can be introduced at random in vitro by using error-prone polymerase (reported in Leung et al., Technique, 1: 11-15 (1989)) in the method of Hawkins et al., J. Mol. Biol., 226: 889-896 (1992) or in the method of Gram et al., Proc. Natl. Acad. Sci. USA, 89: 3576-3580 (1992). Additionally, affinity maturation can be performed by randomly mutating one or more CDRs, e.g. using PCR with primers carrying random sequence spanning the CDR of interest, in selected individual Fv clones and screening for higher affinity clones. WO 9607754 (published 14 Mar. 1996) described a method for inducing mutagenesis in a complementarity determining region of an immunoglobulin light chain to create a library of light chain genes. Another effective approach is to recombine the VH or VL domains selected by phage display with repertoires of naturally occurring V domain variants obtained from unimmunized donors and screen for higher affinity in several rounds of chain reshuffling as described in Marks et al., Biotechnol., 10: 779-783 (1992). This technique allows the production of antibodies and antibody fragments with affinities in the 10-9 M range.

### Other Methods of Generating Anti-SSEA-4 Antibodies

Other methods of generating and assessing the affinity of antibodies are well known in the art and are described, e.g., in Kohler et al., Nature 256: 495 (1975); U.S. Pat. No. 4,816,567; Goding, Monoclonal Antibodies: Principles and Practice, pp. 59-103 (Academic Press, 1986; Kozbor, J. Immunol., 133:3001 (1984); Brodeur et al., Monoclonal Antibody Production Techniques and Applications, pp. 51-63 (Marcel Dekker, Inc., New York, 1987; Munson et al., Anal. Biochem., 107:220 (1980); Engels et al., Agnew. Chem. Int. Ed. Engl., 28: 716-734 (1989); Abrahmsen et al., EMBO J., 4: 3901 (1985); Methods in Enzymology, vol. 44 (1976); Morrison et al., Proc. Natl. Acad. Sci. USA, 81: 6851-6855 (1984).

### General Methods

Accordingly, one aspect of the present disclosure relates to a composition of a homogeneous population of monoclonal antibodies comprising a single, uniform N-glycan on Fc, wherein the structure is an optimized N-glycan structure for enhancing the efficacy of effector cell function.

In preferred embodiments, the N-glycan is attached to the Asn-297 of the Fc region.

The N-glycan consists of the structure of Sia₂(α2-6)Gal₂GlcNAc₂Man₃GlcNAc₂.

The glycoantibodies described herein may be produced *in vitro.* The glycoantibodies may be generated by Fc glycoengineering. In certain embodiments, the glycoantibodies are enzymatically or chemoenzymatically engineered from the monoclonal antibodies obtained by mammalian cell culturing.

In some embodiments, the Fc region of the glycoantibodies described herein exhibits an increased binding affinity for FcyRIIA or FcyRIIIA relative to a wild-type Fc region in the corresponding monoclonal antibodies.

In some embodiments, the glycoantibodies described herein exhibit an enhanced antibody-dependent cell mediated cytotoxicity (ADCC) activity relative to wild-type immunoglobulins.

In some embodiments, the glycoantibodies are selected from a group consisting of human IgG1, IgG2, IgG3, and IgG4. The monoclonal antibodies may be humanized, human or chimeric.

The glycoantibodies described herein may bind to an antigen associated with cancers, autoimmune disorders, inflammatory disorders or infectious diseases. Exemplary cancer associated antigens can include, for example, Globo-H, SSEA-3, SSEA-4.

Generation of antibodies can be achieved using routine skills in the art, including those described herein, such as the hybridoma technique and screening of phage displayed libraries of binder molecules. These methods are well-established in the art.

Briefly, the SSEA-4 antibodies of the invention can be made by using combinatorial libraries to screen for synthetic antibody clones with the desired activity or activities. In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the anti-SSEA-4/ antibodies of the invention can be obtained by designing a suitable antigen screening procedure to select for the phage clone of interest followed by construction of a full length anti-SSEA-4 antibody clone using the Fv sequences from the phage clone of interest and suitable constant region (Fc) sequences described in Kabat et al., Sequences of Proteins of Immunological Interest, Fifth Edition, NIH Publication 91-3242, Bethesda Md. (1991), vols. 1-3.

In one embodiment, anti-SSEA-4 antibodies of the invention are monoclonal. Also encompassed within the scope of the invention are antibody fragments such as Fab, Fab', Fab' -SH and F(ab')2 fragments, and variations thereof, of the anti-SSEA-4 antibodies provided herein. These antibody fragments can be created by traditional means, such as enzymatic digestion, or may be generated by recombinant techniques. Such antibody fragments may be chimeric, human or humanized. These fragments are useful for the therapeutic purposes set forth herein.

Monoclonal antibodies can be obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies.

The anti-SSEA-4 monoclonal antibodies of the invention can be made using a variety of methods known in the art, including the hybridoma method first described by Kohler et al., Nature, 256:495 (1975), or alternatively they may be made by recombinant DNA methods (e.g., U.S. Pat. No. 4,816,567).

### Vectors, Host Cells and Recombinant Methods

For recombinant production of an antibody of the invention, the nucleic acid encoding it is isolated and inserted into a replicable vector for further cloning (amplification of the DNA) or for expression. DNA encoding the antibody is readily isolated and sequenced using conventional procedures (e.g., by using oligonucleotide probes that are capable of binding specifically to genes encoding the heavy and light chains of the antibody). Many vectors are available. The choice of vector depends in part on the host cell to be used. Host cells include, but are not limited to, cells of either prokaryotic or eukaryotic (generally mammalian) origin. It will be appreciated that constant regions of any isotype can be used for this purpose, including IgG, IgM, IgA, IgD, and IgE constant regions, and that such constant regions can be obtained from any human or animal species.

### Generating Antibodies Using Prokaryotic Host Cells

### Vector Construction

Polynucleotide sequences encoding polypeptide components of the antibody of the invention can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR322, a plasmid derived from an E. coli species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Pat. No. 5,648,237.

In addition, phage vectors containing replicon and control sequences that are compatible with the host microorganism can be used as transforming vectors in connection with these hosts. For example, bacteriophage such as λGEM™-11 may be utilized in making a recombinant vector which can be used to transform susceptible host cells such as E. coli LE392.

The expression vector may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, e.g. the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector. Both the native promoter sequence and many heterologous promoters may be used to direct amplification and/or expression of the target genes. Heterologous promoters may be utilized, as they generally permit greater transcription and higher yields of expressed target gene as compared to the native target polypeptide promoter.

Promoters suitable for use with prokaryotic hosts include the PhoA promoter, the β-galactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al. (1980) Cell 20: 269) using linkers or adaptors to supply any required restriction sites.

Each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, Ipp, or heat-stable enterotoxin II (STII) leaders, LamB, PhoE, PelB, OmpA and MBP. The signal sequences used in both cistrons of the expression system may be STII signal sequences or variants thereof.

The production of the immunoglobulins according to the invention can occur in the cytoplasm of the host cell, and therefore does not require the presence of secretion signal sequences within each cistron. In that regard, immunoglobulin light and heavy chains are expressed, folded and assembled to form functional immunoglobulins within the cytoplasm. Certain host strains (e.g., the E. coli trxB- strains) provide cytoplasm conditions that are favorable for disulfide bond formation, thereby permitting proper folding and assembly of expressed protein subunits. Proba and Pluckthun Gene, 159:203 (1995).

Antibodies of the invention can also be produced by using an expression system in which the quantitative ratio of expressed polypeptide components can be modulated in order to maximize the yield of secreted and properly assembled antibodies of the invention. Such modulation is accomplished at least in part by simultaneously modulating translational strengths for the polypeptide components.

One technique for modulating translational strength is disclosed in Simmons et al., U.S. Pat. No. 5,840,523. It utilizes variants of the translational initiation region (TIR) within a cistron. For a given TIR, a series of amino acid or nucleic acid sequence variants can be created with a range of translational strengths, thereby providing a convenient means by which to adjust this factor for the desired expression level of the specific chain. TIR variants can be generated by conventional mutagenesis techniques that result in codon changes which can alter the amino acid sequence. Changes in the nucleotide sequence may be silent. Alterations in the TIR can include, for example, alterations in the number or spacing of Shine-Dalgarno sequences, along with alterations in the signal sequence. One method for generating mutant signal sequences is the generation of a "codon bank" at the beginning of a coding sequence that does not change the amino acid sequence of the signal sequence (i.e., the changes are silent). This can be accomplished by changing the third nucleotide position of each codon; additionally, some amino acids, such as leucine, serine, and arginine, have multiple first and second positions that can add complexity in making the bank. This method of mutagenesis is described in detail in Yansura et al. (1992) METHODS: A Companion to Methods in Enzymol. 4:151-158.

A set of vectors may be generated with a range of TIR strengths for each cistron therein. This limited set provides a comparison of expression levels of each chain as well as the yield of the desired antibody products under various TIR strength combinations. TIR strengths can be determined by quantifying the expression level of a reporter gene as described in detail in Simmons et al. U.S. Pat. No. 5,840,523. Based on the translational strength comparison, the desired individual TIRs are selected to be combined in the expression vector constructs.

Prokaryotic host cells suitable for expressing antibodies of the invention include Archaebacteria and Eubacteria, such as Gram-negative or Gram-positive organisms. Examples of useful bacteria include Escherichia (e.g., E. coli), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. Gram-negative cells may be used. E. coli cells may be used as hosts. Examples of E. coli strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ(nmpc-fepE) degP41 kanR (U.S. Pat. No. 5,639,635). Other strains and derivatives thereof, such as E. coli 294 (ATCC 31,446), E. coli B, E. coli A1776 (ATCC 31,537) and E. coli RV308 (ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, E. coli, Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture. Antibody Production

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic cells used to produce the polypeptides of the invention are grown in media known in the art and suitable for culture of the selected host cells. Examples of suitable media include luria broth (LB) plus necessary nutrient supplements. The media may also contain a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For E. coli growth, for example, growth occurs at a temperature range including, but not limited to, about 20° C. to about 39° C., about 25° C. to about 37° C., and at about 30° C. The pH of the medium may be any pH ranging from about 5 to about 9, depending mainly on the host organism. For E. coli, the pH can be from about 6.8 to about 7.4, or about 7.0.

If an inducible promoter is used in the expression vector, protein expression is induced under conditions suitable for the activation of the promoter. PhoA promoters may be used for controlling transcription of the polypeptides. Accordingly, the transformed host cells are cultured in a phosphate-limiting medium for induction. The phosphate-limiting medium may the C.R.A.P medium (see, e.g., Simmons et al., J. Immunol. Methods (2002), 263:133-147). A variety of other inducers may be used, according to the vector construct employed, as is known in the art.

The expressed polypeptides of the present invention may be secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

Antibody production may be conducted in large quantity by a fermentation process. Various large-scale fed-batch fermentation procedures are available for production of recombinant proteins. Large-scale fermentations have at least 1000 liters of capacity, for example about 1,000 to 100,000 liters of capacity. These fermentors use agitator impellers to distribute oxygen and nutrients, especially glucose (a common carbon/energy source). Small scale fermentation refers generally to fermentation in a fermentor that is no more than approximately 100 liters in volumetric capacity, and can range from about 1 liter to about 100 liters.

In a fermentation process, induction of protein expression is typically initiated after the cells have been grown under suitable conditions to a desired density, e.g., an OD550 of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides of the invention, various fermentation conditions can be modified. For example, to improve the proper assembly and folding of the secreted antibody polypeptides, additional vectors overexpressing chaperone proteins, such as Dsb proteins (DsbA, DsbB, DsbC, DsbD and or DsbG) or FkpA (a peptidylprolyl cis,trans-isomerase with chaperone activity) can be used to co-transform the host prokaryotic cells. The chaperone proteins have been demonstrated to facilitate the proper folding and solubility of heterologous proteins produced in bacterial host cells. Chen et al. (1999) J Bio Chem 274:19601-19605; Georgiou et al., U.S. Pat. No. 6,083,715; Georgiou et al., U.S. Pat. No. 6,027,888; Bothmann and Pluckthun (2000) J. Biol. Chem. 275:17100-17105; Ramm and Pluckthun (2000) J. Biol. Chem. 275:17106-17113; Arie et al. (2001) Mol. Microbiol. 39:199-210.

To minimize proteolysis of expressed heterologous proteins (especially those that are proteolytically sensitive), certain host strains deficient for proteolytic enzymes can be used for the present invention. For example, host cell strains may be modified to effect genetic mutation(s) in the genes encoding known bacterial proteases such as Protease III, OmpT, DegP, Tsp, Protease I, Protease Mi, Protease V, Protease VI and combinations thereof. Some E. coli protease-deficient strains are available and described in, for example, Joly et al. (1998), supra; Georgiou et al., U.S. Pat. No. 5,264,365; Georgiou et al., U.S. Pat. No. 5,508,192; Hara et al., Microbial Drug Resistance, 2:63-72 (1996).

E. coli strains deficient for proteolytic enzymes and transformed with plasmids overexpressing one or more chaperone proteins may be used as host cells in the expression system.

### Antibody Purification

The antibody protein produced herein is further purified to obtain preparations that are substantially homogeneous for further assays and uses. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75.

Protein A immobilized on a solid phasemay be used for immunoaffinity purification of the antibody products of the invention. Protein A is a 41 kD cell wall protein from Staphylococcus aureas which binds with a high affinity to the Fc region of antibodies. Lindmark et al (1983) J. Immunol. Meth. 62:1-13. The solid phase to which Protein A is immobilized can be a column comprising a glass or silica surface, or a controlled pore glass column or a silicic acid column. In some applications, the column is coated with a reagent, such as glycerol, to possibly prevent nonspecific adherence of contaminants.

As the first step of purification, the preparation derived from the cell culture as described above can be applied onto a Protein A immobilized solid phase to allow specific binding of the antibody of interest to Protein A. The solid phase would then be washed to remove contaminants non-specifically bound to the solid phase. Finally the antibody of interest is recovered from the solid phase by elution.

### Generating Antibodies Using Eukaryotic Host Cells

The vector components generally include, but are not limited to, one or more of the following: a signal sequence, an origin of replication, one or more marker genes, an enhancer element, a promoter, and a transcription termination sequence.

### (i) Signal Sequence Component

A vector for use in a eukaryotic host cell may also contain a signal sequence or other polypeptide having a specific cleavage site at the N-terminus of the mature protein or polypeptide of interest. The heterologous signal sequence selected generally is one that is recognized and processed (i.e., cleaved by a signal peptidase) by the host cell. In mammalian cell expression, mammalian signal sequences as well as viral secretory leaders, for example, the herpes simplex gD signal, are available.

The DNA for such precursor region is ligated in reading frame to DNA encoding the antibody.

### (ii) Origin of Replication

Generally, an origin of replication component is not needed for mammalian expression vectors. For example, the SV40 origin may typically be used only because it contains the early promoter.

### (iii) Selection Gene Component

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, where relevant, or (c) supply critical nutrients not available from complex media.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II (e.g., primate metallothionein genes), adenosine deaminase, ornithine decarboxylase, etc.

For example, cells transformed with the DHFR selection gene may first be identified by culturing all of the transformants in a culture medium that contains methotrexate (Mtx), a competitive antagonist of DHFR. Appropriate host cells when wild-type DHFR is employed include, for example, the Chinese hamster ovary (CHO) cell line deficient in DHFR activity (e.g., ATCC CRL-9096).

Alternatively, host cells (particularly wild-type hosts that contain endogenous DHFR) transformed or co-transformed with DNA sequences encoding an antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Pat. No. 4,965,199.

### (iv) Promoter Component

Expression and cloning vectors usually contain a promoter that is recognized by the host organism and is operably linked to nucleic acid encoding a polypeptide of interest (e.g., an antibody). Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Antibody polypeptide transcription from vectors in mammalian host cells can be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, or from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., Nature 297:598-601 (1982) on expression of human β-interferon cDNA in mouse cells under the control of a thymidine kinase promoter from herpes simplex virus. Alternatively, the Rous Sarcoma Virus long terminal repeat can be used as the promoter.

### (v) Enhancer Element Component

Transcription of DNA encoding an antibody polypeptide of the invention by higher eukaryotes can often be increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the antibody polypeptide-encoding sequence, but is generally located at a site 5' from the promoter.

### (vi) Transcription Termination Component

Expression vectors used in eukaryotic host cells will typically also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding an antibody. One useful transcription termination component is the bovine growth hormone polyadenylation region. See WO94/11026 and the expression vector disclosed therein.

### (vii) Selection and Transformation of Host Cells

Suitable host cells for cloning or expressing the DNA in the vectors herein include higher eukaryote cells described herein, including vertebrate host cells. Propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

### (viii) Culturing the Host Cells

The host cells used to produce an antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Pat. Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Purification of Antibody

When using recombinant techniques, the antibody can be produced intracellularly, or directly secreted into the medium. If the antibody is produced intracellularly, as a first step, the particulate debris, either host cells or lysed fragments, are generally removed, for example, by centrifugation or ultrafiltration. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being a generally acceptable purification technique. The suitability of affinity reagents such as protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX™ resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to further purification steps, as necessary, for example by low pH hydrophobic interaction chromatography using an elution buffer at a pH between about 2.5-4.5, generally performed at low salt concentrations (e.g., from about 0-0.25M salt).

It should be noted that, in general, techniques and methodologies for preparing antibodies for use in research, testing and clinical use are well-established in the art, consistent with the above and/or as deemed appropriate by one skilled in the art for the particular antibody of interest.

### Activity Assays

Antibodies of the invention can be characterized for their physical/chemical properties and biological functions by various assays known in the art.

Purified antibodies can be further characterized by a series of assays including, but not limited to, N-terminal sequencing, amino acid analysis, non-denaturing size exclusion high pressure liquid chromatography (HPLC), mass spectrometry, ion exchange chromatography and papain digestion.

Where necessary, antibodies are analyzed for their biological activity. In some embodiments, antibodies of the invention are tested for their antigen binding activity. The antigen binding assays that are known in the art and can be used herein include without limitation any direct or competitive binding assays using techniques such as western blots, radioimmunoassays, ELISA (enzyme linked immunosorbent assay), "sandwich" immunoassays, immunoprecipitation assays, fluorescent immunoassays, chemiluminescent immunoassays, nanoparticle immunoassays, aptamer immunoassays, and protein A immunoassays.

### Antibody Fragments

The present invention encompasses antibody fragments. In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from E. coli, thus allowing the facile production of large amounts of these fragments. Antibody fragments can be isolated from the antibody phage libraries discussed above. Alternatively, Fab'-SH fragments can be directly recovered from E. coli and chemically coupled to form F(ab')2 fragments (Carter et al., Bio/Technology 10: 163-167 (1992)). According to another approach, F(ab')2 fragments can be isolated directly from recombinant host cell culture. Fab and F(ab')2 fragment with increased in vivo half-life comprising salvage receptor binding epitope residues are described in U.S. Pat. No. 5,869,046. Other techniques for the production of antibody fragments will be apparent to the skilled practitioner. In other embodiments, the antibody of choice is a single chain Fv fragment (scFv). See WO 93/16185; U.S. Pat. Nos. 5,571,894; and 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Pat. No. 5,641,870 for example. Such linear antibody fragments may be monospecific or bispecific.

### Humanized Antibodies

The invention encompasses humanized antibodies. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting hypervariable region sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies can be important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody (Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies (Carter et al. (1992) Proc. Natl. Acad. Sci. USA, 89:4285; Presta et al. (1993) J. Immunol., 151:2623.

It is further generally desirable that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

### Human Antibodies

Human anti-SSEA-4 antibodies of the invention can be constructed by combining Fv clone variable domain sequence(s) selected from human-derived phage display libraries with known human constant domain sequences(s) as described above. Alternatively, human monoclonal anti-SSEA-4 antibodies of the invention can be made by the hybridoma method. Human myeloma and mouse-human heteromyeloma cell lines for the production of human monoclonal antibodies have been described, for example, by Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (e.g. mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90: 2551 (1993); Jakobovits et al., Nature, 362: 255 (1993); Bruggermann et al., Year in Immunol., 7: 33 (1993).

Gene shuffling can also be used to derive human antibodies from non-human, e.g. rodent, antibodies, where the human antibody has similar affinities and specificities to the starting non-human antibody. According to this method, which is also called "epitope imprinting", either the heavy or light chain variable region of a non-human antibody fragment obtained by phage display techniques as described above is replaced with a repertoire of human V domain genes, creating a population of non-human chain/human chain scFv or Fab chimeras. Selection with antigen results in isolation of a non-human chain/human chain chimeric scFv or Fab wherein the human chain restores the antigen binding site destroyed upon removal of the corresponding non-human chain in the primary phage display clone, i.e. the epitope governs (imprints) the choice of the human chain partner. When the process is repeated in order to replace the remaining non-human chain, a human antibody is obtained (see PCT WO 93/06213 published Apr. 1, 1993). Unlike traditional humanization of non-human antibodies by CDR grafting, this technique provides completely human antibodies, which have no FR or CDR residues of non-human origin.

### Bispecific Antibodies

Bispecific antibodies are monoclonal antibodies that have binding specificities for at least two different antigens. In certain embodiments, bispecific antibodies are human or humanized antibodies. In certain embodiments, one of the binding specificities is for SSEA-4 including a specific lysine linkage and the other is for any other antigen. In certain embodiments, bispecific antibodies may bind to two different SSEA-4 having two different lysine linkages. Bispecific antibodies can be prepared as full length antibodies or antibody fragments (e.g. F(ab')2 bispecific antibodies).

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

According to a different embodiment, antibody variable domains with the desired binding specificities (antibody-antigen combining sites) are fused to immunoglobulin constant domain sequences. The fusion, for example, is with an immunoglobulin heavy chain constant domain, comprising at least part of the hinge, CH2, and CH3 regions. In certain embodiments, the first heavy-chain constant region (CH1), containing the site necessary for light chain binding, is present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach, the interface between a pair of antibody molecules can be engineered to maximize the percentage of heterodimers which are recovered from recombinant cell culture. The interface comprises at least a part of the CH3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Pat. No. 4,676,980), and for treatment of HIV infection (WO 91/00360, WO 92/00373, and EP 03089). Heteroconjugate antibodies may be made using any convenient cross-linking methods. Suitable cross-linking agents are well known in the art, and are disclosed in U.S. Pat. No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')2 fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from E. coli, which can be chemically coupled to form bispecific antibodies. Shalaby et al., J. Exp. Med., 175: 217-225 (1992) describe the production of a fully humanized bispecific antibody F(ab')2 molecule. Each Fab' fragment was separately secreted from E. coli and subjected to directed chemical coupling in vitro to form the bispecific antibody. The bispecific antibody thus formed was able to bind to cells overexpressing the HER2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The dimerization domain comprises (or consists of), for example, an Fc region or a hinge region. In this scenario, the antibody will comprise an Fc region and three or more antigen binding sites amino-terminal to the Fc region. In one embodiment, a multivalent antibody comprises (or consists of), for example, three to about eight, or four antigen binding sites. The multivalent antibody comprises at least one polypeptide chain (for example, two polypeptide chains), wherein the polypeptide chain(s) comprise two or more variable domains. For instance, the polypeptide chain(s) may comprise VD1-(X1)n-VD2-(X2)n-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein may further comprise at least two (for example, four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain. Antibody Variants

In some embodiments, amino acid sequence modification(s) of the antibodies described herein are contemplated. For example, it may be desirable to improve the binding affinity and/or other biological properties of the antibody. Amino acid sequence variants of the antibody are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution can be made to arrive at the final construct, provided that the final construct possesses the desired characteristics. The amino acid alterations may be introduced in the subject antibody amino acid sequence at the time that sequence is made.

A useful method for identification of certain residues or regions of the antibody that are preferred locations for mutagenesis is called "alanine scanning mutagenesis" as described by Cunningham and Wells (1989) Science, 244:1081-1085. Here, a residue or group of target residues are identified (e.g., charged residues such as Arg, Asp, His, Lys, and Glu) and replaced by a neutral or negatively charged amino acid (e.g., alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed immunoglobulins are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme (e.g. for ADEPT) or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in Table A under the heading of "preferred substitutions". If such substitutions result in a change in biological activity, then more substantial changes, denominated "exemplary substitutions" in Table A, or as further described below in reference to amino acid classes, may be introduced and the products screened.

**TABLE A**

| |
|---|
| Original Exemplary Preferred |
| Residue Substitutions |
| Ala (A) Val; Leu; Ile Val |
| Arg (R) Lys; Gln; Asn Lys |
| Asn (N) Gln; His; Asp, Lys; Arg Gln |
| Asp (D) Glu; Asn Glu |
| Cys (C) Ser; Ala Ser |
| Gln (Q) Asn; Glu Asn |
| Glu (E) Asp; Gln Asp |
| Gly (G) Ala Ala |
| His (H) Asn; Gln; Lys; Arg Arg |
| Ile (I) Leu; Val; Met; Ala; Leu |
| Phe; Norleucine |
| Leu (L) Norleucine; Ile; Val; Ile |
| Met; Ala; Phe |
| Lys (K) Arg; Gln; Asn Arg |
| Met (M) Leu; Phe; Ile Leu |
| Phe (F) Trp; Leu; Val; Ile; Ala; Tyr Tyr |
| Pro (P) Ala Ala |
| Ser (S) Thr Thr |
| Thr (T) Val; Ser Ser |
| Trp (W) Tyr; Phe Tyr |
| Tyr (Y) Trp; Phe; Thr; Ser Phe |
| Val (V) Ile; Leu; Met; Phe; Leu |
| Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)):
•(1) non-polar: Ala (A), Val (V), Leu (L), Ile (I), Pro (P), Phe (F), Trp (W), Met (M)
•(2) uncharged polar: Gly (G), Ser (S), Thr (T), Cys (C), Tyr (Y), Asn (N), Gln (O)
•(3) acidic: Asp (D), Glu (E)
•(4) basic: Lys (K), Arg (R), His (H)

Alternatively, naturally occurring residues may be divided into groups based on common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class. Such substituted residues also may be introduced into the conservative substitution sites or, into the remaining (non-conserved) sites.

One type of substitutional variant involves substituting one or more hypervariable region residues of a parent antibody (e.g. a humanized or human antibody). Generally, the resulting variant(s) selected for further development will have modified (e.g., improved) biological properties relative to the parent antibody from which they are generated. A convenient way for generating such substitutional variants involves affinity maturation using phage display. Briefly, several hypervariable region sites (e.g. 6-7 sites) are mutated to generate all possible amino acid substitutions at each site. The antibodies thus generated are displayed from filamentous phage particles as fusions to at least part of a phage coat protein (e.g., the gene III product of M13) packaged within each particle. The phage-displayed variants are then screened for their biological activity (e.g. binding affinity) as herein disclosed. In order to identify candidate hypervariable region sites for modification, scanning mutagenesis (e.g., alanine scanning) can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to techniques known in the art, including those elaborated herein. Once such variants are generated, the panel of variants is subjected to screening using techniques known in the art, including those described herein, and antibodies with superior properties in one or more relevant assays may be selected for further development.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to introduce one or more amino acid modifications in an Fc region of antibodies of the invention, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions including that of a hinge cysteine.

### Immunoconjugates

In another aspect, the invention provides immunoconjugates, or antibody-drug conjugates (ADC), comprising an antibody conjugated to a cytotoxic agent such as a chemotherapeutic agent, a drug, a growth inhibitory agent, a toxin (e.g., an enzymatically active toxin of bacterial, fungal, plant, or animal origin, or fragments thereof), or a radioactive isotope (i.e., a radioconjugate).

The use of antibody-drug conjugates for the local delivery of cytotoxic or cytostatic agents, i.e. drugs to kill or inhibit tumor cells in the treatment of cancer (Syrigos and Epenetos (1999) Anticancer Research 19:605-614; Niculescu-Duvaz and Springer (1997) Adv. Drg Del. Rev. 26:151-172; U.S. Pat. No. 4,975,278) allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al. (ed.s), pp. 475-506). Maximal efficacy with minimal toxicity is sought thereby. Both polyclonal antibodies and monoclonal antibodies have been reported as useful in these strategies (Rowland et al., (1986) Cancer Immunol. Immunother., 21:183-87). Drugs used in these methods include daunomycin, doxorubicin, methotrexate, and vindesine (Rowland et al., (1986) supra). Toxins used in antibody-toxin conjugates include bacterial toxins such as diphtheria toxin, plant toxins such as ricin, small molecule toxins such as geldanamycin (Mandler et al (2000) Jour. of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may effect their cytotoxic and cytostatic effects by mechanisms including tubutin binding, DNA binding, or topoisomerase inhibition. Some cytotoxic drugs tend to be inactive or less active when conjugated to large antibodies or protein receptor ligands.

### Antibody Derivatives

Antibodies of the invention can be further modified to contain additional nonproteinaceous moieties that are known in the art and readily available. In one embodiment, the moieties suitable for derivatization of the antibody are water soluble polymers. Nonlimiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, the polymers can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### Pharmaceutical Formulations

Therapeutic formulations comprising an antibody of the invention are prepared for storage by mixing the antibody having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

The formulation herein may also contain more than one active compound as necessary for the particular indication being treated, including, but not limited to those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37° C., resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### Uses

Antibodies of the invention are used in the treatment of a hyperproliferative disease or are used for in the treatment of cancer, thereby enhances ADCC activity.

A blocking antibody of the invention is specific for a SSEA-4.

In certain embodiments, an immunoconjugate comprising an antibody of the invention conjugated with a cytotoxic agent is administered to the patient. In some embodiments, the immunoconjugate and/or antigen to which it is bound is/are internalized by cells expressing one or more proteins on their cell surface which are associated with SSEA-4, resulting in increased therapeutic efficacy of the immunoconjugate in killing the target cell with which it is associated. In one embodiment, the cytotoxic agent targets or interferes with nucleic acid in the target cell. Examples of such cytotoxic agents include any of the chemotherapeutic agents noted herein (such as a maytansinoid or a calicheamicin), a radioactive isotope, or a ribonuclease or a DNA endonuclease.

Antibodies of the invention can be used either alone or in combination with other compositions in a therapy. For instance, an antibody of the invention may be co-administered with another antibody, and/or adjuvant/therapeutic agents (e.g., steroids). For instance, an antibody of the invention may be combined with an anti-inflammatory and/or antiseptic in a treatment scheme, e.g. in treating any of the diseases described herein, including cancer, muscular disorders, ubiquitin-pathway-related genetic disorders, immune/inflammatory disorders, neurological disorders, and other ubiquitin pathway-related disorders. Such combined therapies noted above include combined administration (where the two or more agents are included in the same or separate formulations), and separate administration, in which case, administration of the antibody of the invention can occur prior to, and/or following, administration of the adjunct therapy or therapies.

An antibody of the invention (and adjunct therapeutic agent) can be administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. In addition, the antibody is suitably administered by pulse infusion, particularly with declining doses of the antibody. Dosing can be by any suitable route, e.g. by injections, such as intravenous or subcutaneous injections, depending in part on whether the administration is brief or chronic.

The location of the binding target of an antibody of the invention may be taken into consideration in preparation and administration of the antibody. When the binding target is an intracellular molecule, certain embodiments of the invention provide for the antibody or antigen-binding fragment thereof to be introduced into the cell where the binding target is located. In one embodiment, an antibody of the invention can be expressed intracellularly as an intrabody. The term "intrabody," as used herein, refers to an antibody or antigen-binding portion thereof that is expressed intracellularly and that is capable of selectively binding to a target molecule, as described in Marasco, Gene Therapy 4: 11-15 (1997); Kontermann, Methods 34: 163-170 (2004); U.S. Pat. Nos. 6,004,940 and 6,329,173; U.S. Patent Application Publication No. 2003/0104402, and PCT Publication No. WO2003/077945. Intracellular expression of an intrabody is effected by introducing a nucleic acid encoding the desired antibody or antigen-binding portion thereof (lacking the wild-type leader sequence and secretory signals normally associated with the gene encoding that antibody or antigen-binding fragment) into a target cell. Any standard method of introducing nucleic acids into a cell may be used, including, but not limited to, microinjection, ballistic injection, electroporation, calcium phosphate precipitation, liposomes, and transfection with retroviral, adenoviral, adeno-associated viral and vaccinia vectors carrying the nucleic acid of interest. One or more nucleic acids encoding all or a portion of an anti-SSEA-4 antibody of the invention can be delivered to a target cell, such that one or more intrabodies are expressed which are capable of intracellular binding to a SSEA-4 and modulation of one or more SSEA-4-mediated cellular pathways.

In another embodiment, internalizing antibodies are provided. Antibodies can possess certain characteristics that enhance delivery of antibodies into cells, or can be modified to possess such characteristics. Techniques for achieving this are known in the art. For example, cationization of an antibody is known to facilitate its uptake into cells (see, e.g., U.S. Pat. No. 6,703,019). Lipofections or liposomes can also be used to deliver the antibody into cells. Where antibody fragments are used, the smallest inhibitory fragment that specifically binds to the binding domain of the target protein is generally advantageous. For example, based upon the variable-region sequences of an antibody, peptide molecules can be designed that retain the ability to bind the target protein sequence. Such peptides can be synthesized chemically and/or produced by recombinant DNA technology. See, e.g., Marasco et al., Proc. Natl. Acad. Sci. USA, 90: 7889-7893 (1993).

Entry of modulator polypeptides into target cells can be enhanced by methods known in the art. For example, certain sequences, such as those derived from HIV Tat or the Antennapedia homeodomain protein are able to direct efficient uptake of heterologous proteins across cell membranes. See, e.g., Chen et al., Proc. Natl. Acad. Sci. USA (1999), 96:4325-4329.

When the binding target is located in the brain, certain embodiments of the invention provide for the antibody or antigen-binding fragment thereof to traverse the blood-brain barrier. Certain neurodegenerative diseases are associated with an increase in permeability of the blood-brain barrier, such that the antibody or antigen-binding fragment can be readily introduced to the brain. When the blood-brain barrier remains intact, several art-known approaches exist for transporting molecules across it, including, but not limited to, physical methods, lipid-based methods, and receptor and channel-based methods.

Physical methods of transporting the antibody or antigen-binding fragment across the blood-brain barrier include, but are not limited to, circumventing the blood-brain barrier entirely, or by creating openings in the blood-brain barrier. Circumvention methods include, but are not limited to, direct injection into the brain (see, e.g., Papanastassiou et al., Gene Therapy 9: 398-406 (2002)), interstitial infusion/convection-enhanced delivery (see, e.g., Bobo et al., Proc. Natl. Acad. Sci. USA 91: 2076-2080 (1994)), and implanting a delivery device in the brain (see, e.g., Gill et al., Nature Med. 9: 589-595 (2003); and Gliadel Wafers™, Guildford Pharmaceutical). Methods of creating openings in the barrier include, but are not limited to, ultrasound (see, e.g., U.S. Patent Publication No. 2002/0038086), osmotic pressure (e.g., by administration of hypertonic mannitol (Neuwelt, E. A., Implication of the Blood-Brain Barrier and its Manipulation, Vols 1 & 2, Plenum Press, N.Y. (1989))), permeabilization by, e.g., bradykinin or permeabilizer A-7 (see, e.g., U.S. Pat. Nos. 5,112,596, 5,268,164, 5,506,206, and 5,686,416), and transfection of neurons that straddle the blood-brain barrier with vectors containing genes encoding the antibody or antigen-binding fragment (see, e.g., U.S. Patent Publication No. 2003/0083299).

Lipid-based methods of transporting the antibody or antigen-binding fragment across the blood-brain barrier include, but are not limited to, encapsulating the antibody or antigen-binding fragment in liposomes that are coupled to antibody binding fragments that bind to receptors on the vascular endothelium of the blood-brain barrier (see, e.g., U.S. Patent Application Publication No. 20020025313), and coating the antibody or antigen-binding fragment in low-density lipoprotein particles (see, e.g., U.S. Patent Application Publication No. 20040204354) or apolipoprotein E (see, e.g., U.S. Patent Application Publication No. 20040131692).

Receptor and channel-based methods of transporting the antibody or antigen-binding fragment across the blood-brain barrier include, but are not limited to, using glucocorticoid blockers to increase permeability of the blood-brain barrier (see, e.g., U.S. Patent Application Publication Nos. 2002/0065259, 2003/0162695, and 2005/0124533); activating potassium channels (see, e.g., U.S. Patent Application Publication No. 2005/0089473), inhibiting ABC drug transporters (see, e.g., U.S. Patent Application Publication No. 2003/0073713); coating antibodies with a transferrin and modulating activity of the one or more transferrin receptors (see, e.g., U.S. Patent Application Publication No. 2003/0129186), and cationizing the antibodies (see, e.g., U.S. Pat. No. 5,004,697).

The antibody composition of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibodies of the invention present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with other agents such as chemotherapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (e.g. 0.1 mg/kg-10 mg/kg) of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. An exemplary dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays.

Articles of Manufacture (not part of the present invention)

An article of manufacture containing materials useful for the treatment of the disorders described above is provided. The article of manufacture comprises a container and a label or package insert on or associated with the container. Suitable containers include, for example, bottles, vials, syringes, etc. The containers may be formed from a variety of materials such as glass or plastic. The container holds a composition which is by itself or when combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Pharmaceutical Compositions and Formulations

After preparation of the antibodies as described herein, "pre-lyophilized formulation" can be produced. The antibody for preparing the formulation is preferably essentially pure and desirably essentially homogeneous (i.e. free from contaminating proteins etc). "Essentially pure" protein means a composition comprising at least about 90% by weight of the protein, based on total weight of the composition, preferably at least about 95% by weight. "Essentially homogeneous" protein means a composition comprising at least about 99% by weight of protein, based on total weight of the composition. The protein is an antibody.

The amount of antibody in the pre-lyophilized formulation is determined taking into account the desired dose volumes, mode(s) of administration etc. Where the protein of choice is an intact antibody (a full-length antibody), from about 2 mg/mL to about 50 mg/mL, preferably from about 5 mg/mL to about 40 mg/mL and most preferably from about 20-30 mg/mL is an exemplary starting protein concentration. The protein is generally present in solution. For example, the protein may be present in a pH-buffered solution at a pH from about 4-8, and preferably from about 5-7. Exemplary buffers include histidine, phosphate, Tris, citrate, succinate and other organic acids. The buffer concentration can be from about 1 mM to about 20 mM, or from about 3 mM to about 15 mM, depending, for example, on the buffer and the desired isotonicity of the formulation (e.g. of the reconstituted formulation). The preferred buffer is histidine in that, as demonstrated below, this can have lyoprotective properties. Succinate was shown to be another useful buffer.

The lyoprotectant is added to the pre-lyophilized formulation. In preferred embodiments, the lyoprotectant is a non-reducing sugar such as sucrose or trehalose. The amount of lyoprotectant in the pre-lyophilized formulation is generally such that, upon reconstitution, the resulting formulation will be isotonic. However, hypertonic reconstituted formulations may also be suitable. In addition, the amount of lyoprotectant must not be too low such that an unacceptable amount of degradation/aggregation of the protein occurs upon lyophilization. Where the lyoprotectant is a sugar (such as sucrose or trehalose) and the protein is an antibody, exemplary lyoprotectant concentrations in the pre-lyophilized formulation are from about 10 mM to about 400 mM, and preferably from about 30 mM to about 300 mM, and most preferably from about 50 mM to about 100 mM.

The ratio of protein to lyoprotectant is selected for each protein and lyoprotectant combination. In the case of an antibody as the protein of choice and a sugar (e.g., sucrose or trehalose) as the lyoprotectant for generating an isotonic reconstituted formulation with a high protein concentration, the molar ratio of lyoprotectant to antibody may be from about 100 to about 1500 moles lyoprotectant to 1 mole antibody, and preferably from about 200 to about 1000 moles of lyoprotectant to 1 mole antibody, for example from about 200 to about 600 moles of lyoprotectant to 1 mole antibody.

In preferred embodiments of the invention, it has been found to be desirable to add a surfactant to the pre-lyophilized formulation. Alternatively, or in addition, the surfactant may be added to the lyophilized formulation and/or the reconstituted formulation. Exemplary surfactants include nonionic surfactants such as polysorbates (e.g. polysorbates 20 or 80); poloxamers (e.g. poloxamer 188); Triton; sodium dodecyl sulfate (SDS); sodium laurel sulfate; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palnidopropyl-, or isostearamidopropyl-betaine (e.g lauroamidopropyl); myristamidopropyl-, palmidopropyl-, or isostearamidopropyl-dimethylamine; sodium methyl cocoyl-, or disodium methyl oleyl-taurate; and the MONAQUATTM series (Mona Industries, Inc., Paterson, N.J.), polyethyl glycol, polypropyl glycol, and copolymers of ethylene and propylene glycol (e.g. Pluronics, PF68 etc). The amount of surfactant added is such that it reduces aggregation of the reconstituted protein and minimizes the formation of particulates after reconstitution. For example, the surfactant may be present in the pre-lyophilized formulation in an amount from about 0.001-0.5%, and preferably from about 0.005-0.05%.

In certain embodiments of the invention, a mixture of the lyoprotectant (such as sucrose or trehalose) and a bulking agent (e.g. mannitol or glycine) is used in the preparation of the pre-lyophilization formulation. The bulking agent may allow for the production of a uniform lyophilized cake without excessive pockets therein etc.

Other pharmaceutically acceptable carriers, excipients or stabilizers such as those described in Remington's Pharmaceutical Sciences 16th edition, Osol, A. Ed. (1980) may be included in the pre-lyophilized formulation (and/or the lyophilized formulation and/or the reconstituted formulation) provided that they do not adversely affect the desired characteristics of the formulation. Acceptable carriers, excipients or stabilizers are nontoxic to recipients at the dosages and concentrations employed and include; additional buffering agents; preservatives; co-solvents; antioxidants including ascorbic acid and methionine; chelating agents such as EDTA; metal complexes (e.g. Zn-protein complexes); biodegradable polymers such as polyesters; and/or salt-forming counterions such as sodium.

The pharmaceutical compositions and formulations described herein are preferably stable. A "stable" formulation/composition is one in which the antibody therein essentially retains its physical and chemical stability and integrity upon storage. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993). Stability can be measured at a selected temperature for a selected time period.

The formulations to be used for in vivo administration must be sterile. This is readily accomplished by filtration through sterile filtration membranes, prior to, or following, lyophilization and reconstitution. Alternatively, sterility of the entire mixture may be accomplished by autoclaving the ingredients, except for protein, at about 120° C. for about 30 minutes, for example.

After the protein, lyoprotectant and other optional components are mixed together, the formulation is lyophilized. Many different freeze-dryers are available for this purpose such as Hull50® (Hull, USA) or GT20® (Leybold-Heraeus, Germany) freeze-dryers. Freeze-drying is accomplished by freezing the formulation and subsequently subliming ice from the frozen content at a temperature suitable for primary drying. Under this condition, the product temperature is below the eutectic point or the collapse temperature of the formulation. Typically, the shelf temperature for the primary drying will range from about -30 to 25° C. (provided the product remains frozen during primary drying) at a suitable pressure, ranging typically from about 50 to 250 mTorr. The formulation, size and type of the container holding the sample (e.g., glass vial) and the volume of liquid will mainly dictate the time required for drying, which can range from a few hours to several days (e.g. 40-60hrs). A secondary drying stage may be carried out at about 0-40° C., depending primarily on the type and size of container and the type of protein employed. However, it was found herein that a secondary drying step may not be necessary. For example, the shelf temperature throughout the entire water removal phase of lyophilization may be from about 15-30° C. (e.g., about 20° C.). The time and pressure required for secondary drying will be that which produces a suitable lyophilized cake, dependent, e.g., on the temperature and other parameters. The secondary drying time is dictated by the desired residual moisture level in the product and typically takes at least about 5 hours (e.g. 10-15 hours). The pressure may be the same as that employed during the primary drying step. Freeze-drying conditions can be varied depending on the formulation and vial size.

In some instances, it may be desirable to lyophilize the protein formulation in the container in which reconstitution of the protein is to be carried out in order to avoid a transfer step. The container in this instance may, for example, be a 3, 5, 10, 20, 50 or 100 cc vial. As a general proposition, lyophilization will result in a lyophilized formulation in which the moisture content thereof is less than about 5%, and preferably less than about 3%.

At the desired stage, typically when it is time to administer the protein to the patient, the lyophilized formulation may be reconstituted with a diluent such that the protein concentration in the reconstituted formulation is at least 50 mg/mL, for example from about 50 mg/mL to about 400 mg/mL, more preferably from about 80 mg/mL to about 300 mg/mL, and most preferably from about 90 mg/mL to about 150 mg/mL. Such high protein concentrations in the reconstituted formulation are considered to be particularly useful where subcutaneous delivery of the reconstituted formulation is intended. However, for other routes of administration, such as intravenous administration, lower concentrations of the protein in the reconstituted formulation may be desired (for example from about 5-50 mg/mL, or from about 10-40 mg/mL protein in the reconstituted formulation). In certain embodiments, the protein concentration in the reconstituted formulation is significantly higher than that in the pre-lyophilized formulation. For example, the protein concentration in the reconstituted formulation may be about 2-40 times, preferably 3-10 times and most preferably 3-6 times (e.g. at least three fold or at least four fold) that of the pre-lyophilized formulation.

Reconstitution generally takes place at a temperature of about 25° C. to ensure complete hydration, although other temperatures may be employed as desired. The time required for reconstitution will depend, e.g., on the type of diluent, amount of excipient(s) and protein. Exemplary diluents include sterile water, bacteriostatic water for injection (BWFI), a pH buffered solution (e.g. phosphate-buffered saline), sterile saline solution, Ringer's solution or dextrose solution. The diluent optionally contains a preservative. Exemplary preservatives have been described above, with aromatic alcohols such as benzyl or phenol alcohol being the preferred preservatives. The amount of preservative employed is determined by assessing different preservative concentrations for compatibility with the protein and preservative efficacy testing. For example, if the preservative is an aromatic alcohol (such as benzyl alcohol), it can be present in an amount from about 0.1-2.0% and preferably from about 0.5-1.5%, but most preferably about 1.0-1.2%. Preferably, the reconstituted formulation has less than 6000 particles per vial which are > 10 µm size.

### Therapeutic Applications

Described herein is administering to a subject in need of such treatment a therapeutically effective amount of a composition that includes one or more antibodies described herein.

In certain embodiments, the subject being treated is a mammal. In certain embodiments, the subject is a human. In certain embodiments, the subject is a domesticated animal, such as a dog, cat, cow, pig, horse, sheep, or goat. In certain embodiments, the subject is a companion animal such as a dog or cat. In certain embodiments, the subject is a livestock animal such as a cow, pig, horse, sheep, or goat. In certain embodiments, the subject is a zoo animal. In another embodiment, the subject is a research animal such as a rodent, dog, or non-human primate. In certain embodiments, the subject is a non-human transgenic animal such as a transgenic mouse or transgenic pig.

In some embodiments, the subject (e.g., a human patient) in need of the treatment is diagnosed with, suspected of having, or at risk for cancer. Examples of the cancer include, but are not limited to, brain cancer, lung cancer, breast cancer, oral cancer, esophagus cancer, stomach cancer, liver cancer, bile duct cancer, pancreas cancer, colon cancer, kidney cancer, cervix cancer, ovary cancer and prostate cancer. In some embodiments, the cancer is brain cancer, lung cancer, breast cancer, ovarian cancer, prostate cancer, colon cancer, or pancreas cancer. In some preferred embodiments, the cancer is brain cancer or glioblastoma multiforme (GBM) cancer.

In preferred embodiments, the antibody is capable of targeting Globo H, SSEA-3 and SSEA-4-expressing cancer cells. In some embodiments, the antibody is capable of targeting SSEA on cancer cells. The antibody is capable of targeting SSEA in cancers.

The treatment results in reduction of tumor size, elimination of malignant cells, prevention of metastasis, prevention of relapse, reduction or killing of disseminated cancer, prolongation of survival and/or prolongation of time to tumor cancer progression.

In some embodiments, the treatment further comprises administering an additional therapy to said subject prior to, during or subsequent to said administering of the antibodies. In some embodiments, the additional therapy is treatment with a chemotherapeutic agent. In some embodiments, the additional therapy is radiation therapy.

The antibodies of the invention are particularly advantageous in treating and preventing early stage tumors, thereby preventing progression to the more advanced stages resulting in a reduction in the morbidity and mortality associated with advanced cancer. The antibodies of the invention are also advantageous in preventing the recurrence of a tumor or the regrowth of a tumor, for example, a dormant tumor that persists after removal of the primary tumor, or in reducing or preventing the occurrence of a tumor.

In some embodiments, the methods as disclosed herein are useful for the treatment or prevention of a cancer, for example where a cancer is characterized by increased Globo H, SSEA-3 and/or SSEA-4 expression. In some embodiments the cancer comprises a cancer stem cell. In some embodiments, the cancer is a pre-cancer, and/or a malignant cancer and/or a therapy resistant cancer. In some embodiments, the cancer is a brain cancer.

For the antibodies for use in the methods of the invention, the cancer may be a solid tumor, e.g., such as, breast cancer, colorectal cancer, rectal cancer, lung cancer, renal cell cancer, a glioma (e.g., anaplastic astrocytoma, anaplastic oligoastrocytoma, anaplastic oligodendroglioma, glioblastoma multiforme (GBM)), kidney cancer, prostate cancer, liver cancer, pancreatic cancer, soft-tissue sarcoma, carcinoid carcinoma, head and neck cancer, melanoma, and ovarian cancer. In one embodiment, the cancer is a brain cancer or GBM. To practice the method disclosed herein, an effective amount of the pharmaceutical composition/formulation described above, containing at least one antibody described herein, can be administered to a subject (e.g., a human) in need of the treatment via a suitable route, such as intravenous administration, e.g., as a bolus or by continuous infusion over a period of time, by intramuscular, intraperitoneal, intracerebrospinal, subcutaneous, intra-articular, intrasynovial, intrathecal, oral, inhalation or topical routes. Commercially available nebulizers for liquid formulations, including jet nebulizers and ultrasonic nebulizers are useful for administration. Liquid formulations can be directly nebulized and lyophilized powder can be nebulized after reconstitution. Alternatively, the antibodies can be aerosolized using a fluorocarbon formulation and a metered dose inhaler, or inhaled as a lyophilized and milled powder.

The subject to be treated by the antibodies described herein can be a mammal, more preferably a human. Mammals include, but are not limited to, farm animals, sport animals, pets, primates, horses, dogs, cats, mice and rats. A human subject who needs the treatment may be a human patient having, at risk for, or suspected of having cancer, which include, but not limited to, brain cancer, lung cancer, breast cancer, oral cancer, esophagus cancer, stomach cancer, liver cancer, bile duct cancer, pancreas cancer, colon cancer, kidney cancer, cervix cancer, ovary cancer and prostate cancer. A subject having cancer can be identified by routine medical examination.

"An effective amount" as used herein refers to the amount of each active agent required to confer therapeutic effect on the subject, either alone or in combination with one or more other active agents. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

Empirical considerations, such as the half-life, generally will contribute to the determination of the dosage. For example, antibodies that are compatible with the human immune system, such as humanized antibodies or fully human antibodies, may be used to prolong half-life of the antibody and to prevent the antibody being attacked by the host's immune system. Frequency of administration may be determined and adjusted over the course of therapy, and is generally, but not necessarily, based on treatment and/or suppression and/or amelioration and/or delay of cancer. Alternatively, sustained continuous release formulations of the antibodies described herein may be appropriate. Various formulations and devices for achieving sustained release are known in the art.

In one example, dosages for an antibody as described herein may be determined empirically in individuals who have been given one or more administration(s) of the antibody. Individuals are given incremental dosages of the antibody. To assess efficacy of the antibody, an indicator of the disease (e.g., cancer) can be followed according to routine practice.

Generally, for administration of any of the antibodies described herein, an initial candidate dosage can be about 2 mg/kg. For the purpose of the present disclosure, a typical daily dosage might range from about any of 0.1 µg/kg to 3 µg/kg to 30 µg/kg to 300 µg/kg to 3 mg/kg, to 30 mg/kg to 100 mg/kg or more, depending on the factors mentioned above. For repeated administrations over several days or longer, depending on the condition, the treatment is sustained until a desired suppression of symptoms occurs or until sufficient therapeutic levels are achieved to alleviate cancer, or a symptom thereof. An exemplary dosing regimen comprises administering an initial dose of about 2 mg/kg, followed by a weekly maintenance dose of about 1 mg/kg of the antibody, or followed by a maintenance dose of about 1 mg/kg every other week. However, other dosage regimens may be useful, depending on the pattern of pharmacokinetic decay that the practitioner wishes to achieve. For example, dosing from one-four times a week is contemplated. In some embodiments, dosing ranging from about 3 µg/mg to about 2 mg/kg (such as about 3 µg/mg, about 10 µg/mg, about 30 µg/mg, about 100 µg/mg, about 300 µg/mg, about 1 mg/kg, and about 2 mg/kg) may be used. In some embodiments, dosing frequency is once every week, every 2 weeks, every 4 weeks, every 5 weeks, every 6 weeks, every 7 weeks, every 8 weeks, every 9 weeks, or every 10 weeks; or once every month, every 2 months, or every 3 months, or longer. The progress of this therapy is easily monitored by conventional techniques and assays. The dosing regimen (including the antibody used) can vary over time.

For the purpose of the present disclosure, the appropriate dosage of an antibody described herein will depend on the specific antibody (or compositions thereof) employed, the type and severity of the cancer, whether the antibody is administered for preventive or therapeutic purposes, previous therapy, the patient's clinical history and response to the antibody, and the discretion of the attending physician. The administration of the antibodies described herein may be essentially continuous over a preselected period of time or may be in a series of spaced dose, e.g., either before, during, or after developing cancer.

As used herein, the term "treating" refers to the application or administration of a composition including one or more active agents to a subject, who has cancer, a symptom of cancer, or a predisposition toward cancer, with the purpose to cure, heal, alleviate, relieve, alter, remedy, ameliorate, improve, or affect cancer, the symptom of cancer, or the predisposition toward cancer.

Alleviating cancer includes delaying the development or progression of cancer, or reducing cancer severity. Alleviating cancer does not necessarily require curative results. As used therein, "delaying" the development of cancer means to defer, hinder, slow, retard, stabilize, and/or postpone progression of cancer. This delay can be of varying lengths of time, depending on the history of cancer and/or individuals being treated. A method that "delays" or alleviates the development of cancer, or delays the onset of cancer, is a method that reduces probability (the risk) of developing one or more symptoms of cancer in a given time frame and/or reduces extent of the symptoms in a given time frame, when compared to not using the method. Such comparisons are typically based on clinical studies, using a number of subjects sufficient to give a statistically significant result.

"Development" or "progression" of cancer means initial manifestations and/or ensuing progression of cancer. Development of cancer can be detectable and assessed using standard clinical techniques as well known in the art. However, development also refers to progression that may be undetectable. For purpose of this disclosure, development or progression refers to the biological course of the symptoms. "Development" includes occurrence, recurrence, and onset. As used herein "onset" or "occurrence" of cancer includes initial onset and/or recurrence.

Conventional methods, known to those of ordinary skill in the art of medicine, can be used to administer the pharmaceutical composition to the subject, depending upon the type of disease to be treated or the site of the disease. This composition can also be administered via other conventional routes, e.g., administered orally, parenterally, by inhalation spray, topically, rectally, nasally, buccally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. In addition, it can be administered to the subject via injectable depot routes of administration such as using 1-, 3-, or 6-month depot injectable or biodegradable materials and methods.

Injectable compositions may contain various carriers such as vegetable oils, dimethylactamide, dimethyformamide, ethyl lactate, ethyl carbonate, isopropyl myristate, ethanol, and polyols (glycerol, propylene glycol, liquid polyethylene glycol, and the like). For intravenous injection, water soluble antibodies can be administered by the drip method, whereby a pharmaceutical formulation containing the antibody and a physiologically acceptable excipients is infused. Physiologically acceptable excipients may include, for example, 5% dextrose, 0.9% saline, Ringer's solution or other suitable excipients. Intramuscular preparations, e.g., a sterile formulation of a suitable soluble salt form of the antibody, can be dissolved and administered in a pharmaceutical excipient such as Water-for-Injection, 0.9% saline, or 5% glucose solution.

In some embodiments, the provided glycan conjugates, immunogenic compositions are useful in treating a cancer, including, but are not limited to, acoustic neuroma, adenocarcinoma, adrenal gland cancer, anal cancer, angiosarcoma (*e.g*., lymphangiosarcoma, lymphangioendotheliosarcoma, hemangiosarcoma), appendix cancer, benign monoclonal gammopathy, biliary cancer (*e.g.,* cholangiocarcinoma), bladder cancer, breast cancer (*e.g.,* adenocarcinoma of the breast, papillary carcinoma of the breast, mammary cancer, medullary carcinoma of the breast), brain cancer (*e.g.,* meningioma; glioma, *e.g.,* astrocytoma, oligodendroglioma; medulloblastoma), bronchus cancer, carcinoid tumor, cervical cancer (*e.g.,* cervical adenocarcinoma), choriocarcinoma, chordoma, craniopharyngioma, colorectal cancer (*e.g.,* colon cancer, rectal cancer, colorectal adenocarcinoma), epithelial carcinoma, ependymoma, endotheliosarcoma (*e.g*., Kaposi's sarcoma, multiple idiopathic hemorrhagic sarcoma), endometrial cancer (*e.g.,* uterine cancer, uterine sarcoma), esophageal cancer *(e.g.,* adenocarcinoma of the esophagus, Barrett's adenocarinoma), Ewing sarcoma, eye cancer (*e.g*., intraocular melanoma, retinoblastoma), familiar hypereosinophilia, gall bladder cancer, gastric cancer (*e.g.*, stomach adenocarcinoma), gastrointestinal stromal tumor (GIST), head and neck cancer (*e.g.,* head and neck squamous cell carcinoma, oral cancer (*e.g.,* oral squamous cell carcinoma (OSCC), throat cancer (*e.g.*, laryngeal cancer, pharyngeal cancer, nasopharyngeal cancer, oropharyngeal cancer)), hematopoietic cancers (*e.g.*, leukemia such as acute lymphocytic leukemia (ALL) (*e.g.,* B-cell ALL, T-cell ALL), acute myelocytic leukemia (AML) (*e.g.,* B-cell AML, T-cell AML), chronic myelocytic leukemia (CML) (*e.g.,* B-cell CML, T-cell CML), and chronic lymphocytic leukemia (CLL) (*e.g.,* B-cell CLL, T-cell CLL); lymphoma such as Hodgkin lymphoma (HL) (*e.g.,* B-cell HL, T-cell HL) and non-Hodgkin lymphoma (NHL) (*e.g.,* B-cell NHL such as diffuse large cell lymphoma (DLCL) (e.g., diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (*e.g*., mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (*i.e*., "Waldenström's macroglobulinemia"), hairy cell leukemia (HCL), immunoblastic large cell lymphoma, precursor B-lymphoblastic lymphoma and primary central nervous system (CNS) lymphoma; and T-cell NHL such as precursor T-lymphoblastic lymphoma/leukemia, peripheral T-cell lymphoma (PTCL) (*e.g.,* cutaneous T-cell lymphoma (CTCL) (*e.g.,* mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma); a mixture of one or more leukemia/lymphoma as described above; and multiple myeloma (MM)), heavy chain disease (*e.g*., alpha chain disease, gamma chain disease, mu chain disease), hemangioblastoma, inflammatory myofibroblastic tumors, immunocytic amyloidosis, kidney cancer (*e.g.,* nephroblastoma *a.k.a.* Wilms' tumor, renal cell carcinoma), liver cancer (*e.g*., hepatocellular cancer (HCC), malignant hepatoma), lung cancer (*e.g*., bronchogenic carcinoma, small cell lung cancer (SCLC), non-small cell lung cancer (NSCLC), adenocarcinoma of the lung), leiomyosarcoma (LMS), mastocytosis (*e.g*., systemic mastocytosis), myelodysplastic syndrome (MDS), mesothelioma, myeloproliferative disorder (MPD) (*e.g*., polycythemia Vera (PV), essential thrombocytosis (ET), agnogenic myeloid metaplasia (AMM), a.k.a. myelofibrosis (MF), chronic idiopathic myelofibrosis, chronic myelocytic leukemia (CML), chronic neutrophilic leukemia (CNL), hypereosinophilic syndrome (HES)), neuroblastoma, neurofibroma (*e.g*., neurofibromatosis (NF) type 1 or type 2, schwannomatosis), neuroendocrine cancer *(e.g.,* gastroenteropancreatic neuroendoctrine tumor (GEP-NET), carcinoid tumor), osteosarcoma, ovarian cancer (*e.g.*, cystadenocarcinoma, ovarian embryonal carcinoma, ovarian adenocarcinoma), papillary adenocarcinoma, pancreatic cancer (*e.g*., pancreatic andenocarcinoma, intraductal papillary mucinous neoplasm (IPMN), islet cell tumors), penile cancer (*e.g*., Paget's disease of the penis and scrotum), pinealoma, primitive neuroectodermal tumor (PNT), prostate cancer (*e.g*., prostate adenocarcinoma), rectal cancer, rhabdomyosarcoma, salivary gland cancer, skin cancer (*e.g*., squamous cell carcinoma (SCC), keratoacanthoma (KA), melanoma, basal cell carcinoma (BCC)), small bowel cancer *(e.g.,* appendix cancer), soft tissue sarcoma (*e.g*., malignant fibrous histiocytoma (MFH), liposarcoma, malignant peripheral nerve sheath tumor (MPNST), chondrosarcoma, fibrosarcoma, myxosarcoma), sebaceous gland carcinoma, sweat gland carcinoma, synovioma, testicular cancer (*e.g.,* seminoma, testicular embryonal carcinoma), thyroid cancer (*e.g.*, papillary carcinoma of the thyroid, papillary thyroid carcinoma (PTC), medullary thyroid cancer), urethral cancer, vaginal cancer and vulvar cancer (*e.g*., Paget's disease of the vulva). In certain embodiments, the provided glycan conjugates, immunogenic compositions or vaccines are useful for treating brain cancer, lung cancer, breast cancer, oral cancer, esophagus cancer, stomach cancer, liver cancer, bile duct cancer, pancreas cancer, colon cancer, kidney cancer, bone cancer, skin cancer, cervix cancer, ovary cancer, and prostate cancer.

To perform the treatment described herein, an effective amount of any of the glycan compositions described herein may be administered to a subject in need of the treatment via a suitable route, as described above. The subject, such as a human subject, can be a patient having cancer, suspected of having cancer, or susceptible to cancer. In some embodiments, the amount of the glycan conjugate or immunogenic composition is sufficient to elicit responses leading to the inhibition of cancer growth and/or reduction of tumor mass. In other embodiments, the amount of the glycan composition may be effective in delaying the onset of the target cancer or reducing the risk for developing the cancer. The exact amount of the provided glycan compositions required to achieve an effective amount will vary from subject to subject, depending, for example, on species, age, and general condition of a subject, severity of the side effects or disorder, identity of the particular compound(s), mode of administration, and the like. The desired dosage can be delivered three times a day, two times a day, once a day, every other day, every third day, every week, every two weeks, every three weeks, or every four weeks. In certain embodiments, the desired dosage can be delivered using multiple administrations (*e.g*., two, three, four, five, six, seven, eight, nine, ten, eleven, twelve, thirteen, fourteen, or more administrations).

In certain embodiments, an effective amount, of the provided glycan compositions for administration one or more times a day to a 70 kg adult human may comprise about 0.0001 mg to about 3000 mg, about 0.0001 mg to about 2000 mg, about 0.0001 mg to about 1000 mg, about 0.001 mg to about 1000 mg, about 0.01 mg to about 1000 mg, about 0.1 mg to about 1000 mg, about 1 mg to about 1000 mg, about 1 mg to about 100 mg, about 10 mg to about 1000 mg, or about 100 mg to about 1000 mg, of a compound per unit dosage form.

In certain embodiments, the provided glycan compositions may be administered orally or parenterally at dosage levels sufficient to deliver from about 0.001 mg/kg to about 100 mg/kg, from about 0.01 mg/kg to about 50 mg/kg, preferably from about 0.1 mg/kg to about 40 mg/kg, preferably from about 0.5 mg/kg to about 30 mg/kg, from about 0.01 mg/kg to about 10 mg/kg, from about 0.1 mg/kg to about 10 mg/kg, and more preferably from about 1 mg/kg to about 25 mg/kg, of subject body weight per day, one or more times a day, to obtain the desired therapeutic effect.

It will be appreciated that dose ranges as described herein provide guidance for the administration of the provided glycan conjugates, immunogenic compositions or vaccines to an adult. The amount to be administered to, for example, a child or an adolescent can be determined by a medical practitioner or person skilled in the art and can be lower or the same as that administered to an adult.

It will be also appreciated that the provided glycan compositions can be administered in combination with one or more additional therapeutically active agents. The provided glycan conjugates, immunogenic compositions or vaccines can be administered in combination with additional therapeutically active agents that improve their bioavailability, reduce and/or modify their metabolism, inhibit their excretion, and/or modify their distribution within the body. It will also be appreciated that the therapy employed may achieve a desired effect for the same disorder, and/or it may achieve different effects.

The provided glycan compositions can be administered concurrently with, prior to, or subsequent to, one or more additional therapeutically active agents. In general, each agent will be administered at a dose and/or on a time schedule determined for that agent. In will further be appreciated that the additional therapeutically active agent utilized in this combination can be administered together in a single composition or administered separately in different compositions. The particular combination to employ in a regimen will take into account compatibility of the inventive compound with the additional therapeutically active agent and/or the desired therapeutic effect to be achieved. In general, it is expected that additional therapeutically active agents utilized in combination be utilized at levels that do not exceed the levels at which they are utilized individually. In some embodiments, the levels utilized in combination will be lower than those utilized individually.

In certain embodiments, the provided glycan composition is administered in combination with one or more additional pharmaceutical agents described herein. In certain embodiments, the additional pharmaceutical agent is an anti-cancer agent. Anti-cancer agents encompass biotherapeutic anti-cancer agents as well as chemotherapeutic agents.

Exemplary biotherapeutic anti-cancer agents include, but are not limited to, interferons, cytokines (*e.g*., tumor necrosis factor, interferon α, interferon γ), vaccines, hematopoietic growth factors, monoclonal serotherapy, immunostimulants and/or immunodulatory agents (e.g., IL-1, 2, 4, 6, or 12), immune cell growth factors *(e.g.,* GM-CSF) and antibodies (*e.g*. HERCEPTIN (trastuzumab), T-DM1, AVASTIN (bevacizumab), ERBITUX (cetuximab), VECTIBIX (panitumumab), RITUXAN (rituximab), BEXXAR (tositumomab)).

Exemplary chemotherapeutic agents include, but are not limited to, anti-estrogens (*e.g.* tamoxifen, raloxifene, and megestrol), LHRH agonists (*e.g.* goscrclin and leuprolide), anti-androgens (*e.g.* flutamide and bicalutamide), photodynamic therapies (*e.g.* vertoporfin (BPD-MA), phthalocyanine, photosensitizer Pc4, and demethoxy-hypocrellin A (2BA-2-DMHA)), nitrogen mustards (*e.g*. cyclophosphamide, ifosfamide, trofosfamide, chlorambucil, estramustine, and melphalan), nitrosoureas (*e.g*. carmustine (BCNU) and lomustine (CCNU)), alkylsulphonates (*e.g.* busulfan and treosulfan), triazenes (*e.g.* dacarbazine, temozolomide), platinum containing compounds (*e.g*. cisplatin, carboplatin, oxaliplatin), vinca alkaloids (e.g. vincristine, vinblastine, vindesine, and vinorelbine), taxoids (*e.g.* paclitaxel or a paclitaxel equivalent such as nanoparticle albumin-bound paclitaxel (Abraxane), docosahexaenoic acid bound-paclitaxel (DHA-paclitaxel, Taxoprexin), polyglutamate bound-paclitaxel (PG-paclitaxel, paclitaxel poliglumex, CT-2103, XYOTAX), the tumor-activated prodrug (TAP) ANG1005 (Angiopep-2 bound to three molecules of paclitaxel), paclitaxel-EC-1 (paclitaxel bound to the erbB2-recognizing peptide EC-1), and glucose-conjugated paclitaxel, e.g., 2'-paclitaxel methyl 2-glucopyranosyl succinate; docetaxel, taxol), epipodophyllins (*e.g*. etoposide, etoposide phosphate, teniposide, topotecan, 9-aminocamptothecin, camptoirinotecan, irinotecan, crisnatol, mytomycin C), anti-metabolites, DHFR inhibitors (*e.g.* methotrexate, dichloromethotrexate, trimetrexate, edatrexate), IMP dehydrogenase inhibitors (e.g. mycophenolic acid, tiazofurin, ribavirin, and EICAR), ribonuclotide reductase inhibitors (e.g. hydroxyurea and deferoxamine), uracil analogs (e.g. 5-fluorouracil (5-FU), floxuridine, doxifluridine, ratitrexed, tegafur-uracil, capecitabine), cytosine analogs (e.g. cytarabine (ara C), cytosine arabinoside, and fludarabine), purine analogs (e.g. mercaptopurine and Thioguanine), Vitamin D3 analogs (e.g. EB 1089, CB 1093, and KH 1060), isoprenylation inhibitors (*e.g*. lovastatin), dopaminergic neurotoxins (*e.g.* 1-methyl-4-phenylpyridinium ion), cell cycle inhibitors *(e.g.* staurosporine), actinomycin (*e.g.* actinomycin D, dactinomycin), bleomycin (*e.g*. bleomycin A2, bleomycin B2, peplomycin), anthracycline (*e.g.* daunorubicin, doxorubicin, pegylated liposomal doxorubicin, idarubicin, epirubicin, pirarubicin, zorubicin, mitoxantrone), MDR inhibitors (*e.g.* verapamil), Ca²⁺ ATPase inhibitors (e.g. thapsigargin), imatinib, thalidomide, lenalidomide, tyrosine kinase inhibitors (*e.g.*, axitinib (AG013736), bosutinib (SKI-606), cediranib (RECENTIN™, AZD2171), dasatinib (SPRYCEL®, BMS-354825), erlotinib (TARCEVA®), gefitinib (IRESSA®), imatinib (Gleevec®, CGP57148B, STI-571), lapatinib (TYKERB®, TYVERB®), lestaurtinib (CEP-701), neratinib (HKI-272), nilotinib (TASIGNA®), semaxanib (semaxinib, SU5416), sunitinib (SUTENT®, SU11248), toceranib (PALLADIA®), vandetanib (ZACTIMA®, ZD6474), vatalanib (PTK787, PTK/ZK), trastuzumab (HERCEPTIN®), bevacizumab (AVASTIN®), rituximab (RITUXAN®), cetuximab (ERBITUX®), panitumumab (VECTIBIX®), ranibizumab (Lucentis®), nilotinib (TASIGNA®), sorafenib (NEXAVAR®), everolimus (AFINITOR®), alemtuzumab (CAMPATH®), gemtuzumab ozogamicin (MYLOTARG®), temsirolimus (TORISEL®), ENMD-2076, PCI-32765, AC220, dovitinib lactate (TKI258, CHIR-258), BIBW 2992 (TOVOK™), SGX523, PF-04217903, PF-02341066, PF-299804, BMS-777607, ABT-869, MP470, BIBF 1120 (VARGATEF®), AP24534, JNJ-26483327, MGCD265, DCC-2036, BMS-690154, CEP-11981, tivozanib (AV-951), OSI-930, MM-121, XL-184, XL-647, and/or XL228), proteasome inhibitors (*e.g.,* bortezomib (VELCADE)), mTOR inhibitors (*e.g.,* rapamycin, temsirolimus (CCI-779), everolimus (RAD-001), ridaforolimus, AP23573 (Ariad), AZD8055 (AstraZeneca), BEZ235 (Novartis), BGT226 (Norvartis), XL765 (Sanofi Aventis), PF-4691502 (Pfizer), GDC0980 (Genetech), SF1126 (Semafoe) and OSI-027 (OSI)), oblimersen, gemcitabine, carminomycin, leucovorin, pemetrexed, cyclophosphamide, dacarbazine, procarbizine, prednisolone, dexamethasone, campathecin, plicamycin, asparaginase, aminopterin, methopterin, porfiromycin, melphalan, leurosidine, leurosine, chlorambucil, trabectedin, procarbazine, discodermolide, carminomycin,, aminopterin, and hexamethyl melamine.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are now described. All references cited in this specification are to be taken as indicative of the level of skill in the art.

### EXAMPLES

The following examples are included to represent techniques discovered by the inventor to function well in the practice of the invention, which is as defined in the claims.

### EXAMPLE 1: Exemplary Structure of optimized universal Fc glycan

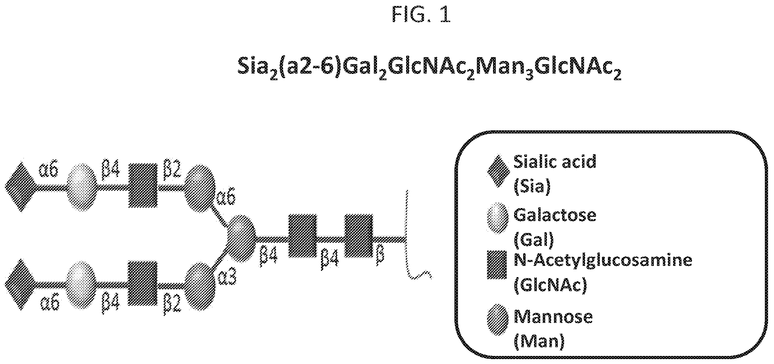

The glycan structure of an optimized universal Fc glycan for therapeutic antibodies is Sia₂(α2-6)Gal₂GlcNAc₂Man₃GlcNAc₂ (Figure 1).

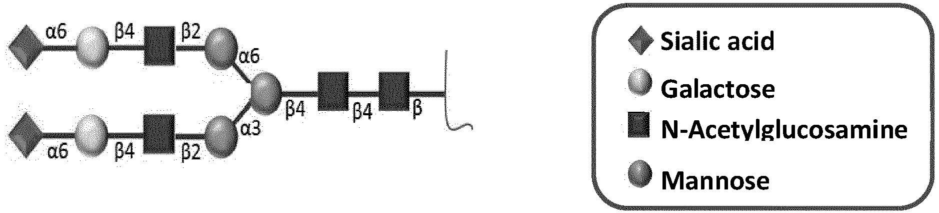

**Figure 1****.** Structure of optimized universal Fc glycan of therapeutic antibodies.

EXAMPLE 2: Exemplary General procedure for the preparation of homogeneous antibodies with the optimized universal glycan at the Fc region.

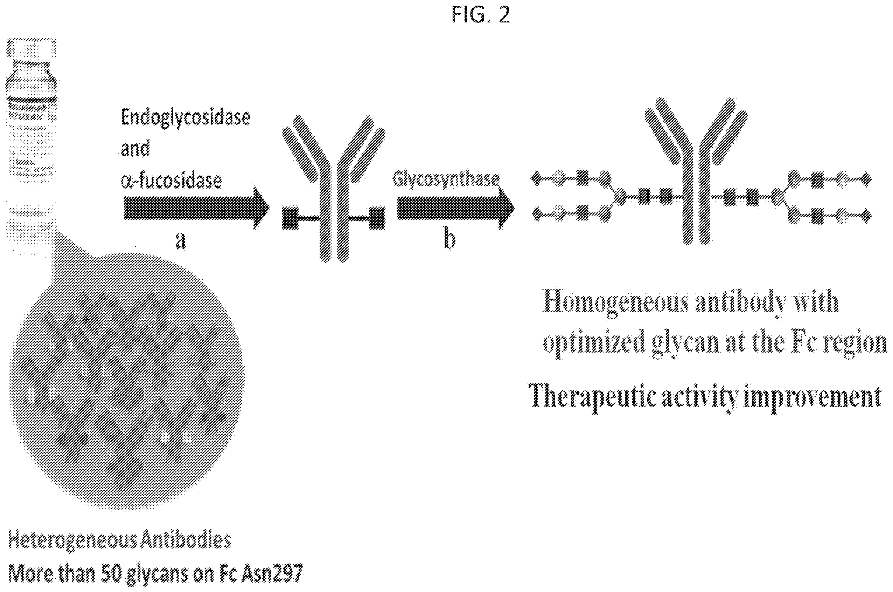

The present disclosure provides exemplary improved method for making a population of homogeneous antibodies with the optimized universal glycan at the Fc region comprising the steps of (a) contacting a monoclonal antibody with an α-fucosidase and at least one endoglycosidase, thereby yielding a defucosylated antibody having a single N-acetylglucosamine (GlcNAc), and (b) adding the universal glycan to GlcNAc of Fc region of antibody to form the homogeneous antibody with the Figure 1 showed optimized glycan form (Figure 2).

See Figure 2. General strategy for the preparation of homogeneous antibody with optimized universal glycan at the Fc region for the improvement of its therapeutic activity.

Endoglycosidase is used to trim off the variable portions of an oligosaccharide in N-glycan. Examples of endoglycosidases used herein include, but not limited to, EndoA, EndoF, EndoF1, EndoF2, EndoH, EndoM, EndoS, and variants thereof.

EXAMPLE 3: Preparation of homogeneous antibody with universal glycan at the Fc region toward enhancing monoclonal antibody mediated antiviral therapeutics.

Exemplary method for the preparation of homogeneous anti-influenza virus antibody with universal glycan at the Fc region to increase the its ADCC effect.

Broadly neutralizing monoclonal antibodies targeting the conserved stalk region of hemagglutinin (HA) can be facilitated by the interactions between the antibody Fc and Fc receptors to trigger its function. The anti-influenza virus antibody FI6 was chosen based on its demonstrated ADCC effects, and the other anti-influenza virus antibody F10 that target stalk region of (HA) for the preparation of homogeneous antibody with optimized universal glycan by using the general strategy and methods disclosed herein. In brief, FI6 and F10 antibodies were prepared by the literature reported methods (ref). The home-made heterogeneous monoclonal antibodies FI6 or F10 was used as the starting material and modified with endoglycosidase endo S to yield a mixture of di-sugar mAb of GlcNAc-Fuc, and mono-sugar mAb of GlcNAc. Subsequently a homogeneous mono-sugar mAb was obtained with application of fucosidase; or the mono-sugar species was obtained with combination of Endo S and fucosidase in one step.

FI6/F10 (0.25 mg) in a sodium phosphate buffer (50 mM, pH 7.0, 0.125 mL) was incubated with Endo S (12.5 µg) and BfFucH (0.25 mg) at 37 °C for 22 h. LC-MS and SDS-PAGE analyses indicated the complete cleavage of the N-glycans on the heavy chain. The reaction mixture was subject to affinity chromatography on a column of protein A-agarose resin (0.1 mL) that was pre-equilibrated with a sodium phosphate buffer (20 mM, pH 7.0). The column was washed with a sodium phosphate buffer (20 mM, pH 7.0, 1.0 mL). The bound IgG was released with glycine-HCl (50 mM, pH 3.0, 1.0 mL), and the elution fractions were immediately neutralized with Tris-Cl buffer (1.0 M, pH 8.3). The fractions containing the Fc fragments were combined and concentrated by centrifugal filtration (Amicon Ultra centrifugal filter, Millipore, Billerica, MA) to give mono-GlcNAc homogeneous antibody (0.193 mg). The product was trypsinized, and the glycopeptides, TKPREEQYNSTYR and EEQYNSTYR, were analyzed using nanospray LC/MS to confirm the glycosylation pattern of glycan engineering FI6/F10.

Isolation of the sialylglycan (SCT) from hen's egg yolk was according to the published method with some modification. Briefly, the ethanol extraction of hen's egg yolk was centrifuged, filtrated, and the treated with endo M, after reaction complete, the SCT was purified by gel filtration and ion exchange chromatography, the purified SCT was lyophilized to give pure SCT product as a white powder (82%).

A solution of SCT (Sia₂Gal₂GlcNAc₂Man₃GlcNAc) (3.0 mg), 2-chloro-1,3-dimethylimidazolinium chloride (DMC) (6.3 mg) and Et₃N (9.0 µL) in water (60.0 µL) was stirred at 4 °C for 1 h. The reaction mixture was subjected to gel filtration chromatography on a Sephadex G-25 column eluted by 0.05% aqueous Et₃N. The fractions containing the product (SCT oxazoline) were combined and lyophilized to give a white powder (2.6 mg, yield 87.4%).

SCT oxazoline was added to a mixture of glycosynthase and mono-GlcNAc Fi6 or F10 in 50 mM Tris buffer (pH 7.8) and incubated for an hour at room temperature. The reaction mixture was purified with protein A affinity column, followed by amanion exchange column capto Q to collect the desired product, optimized anti-influenza virus homogeneous antibody FI6-M or F10-M . The product was trypsinized, and the glycopeptides, TKPREEQYNSTYR and EEQYNSTYR, were analyzed using nanospray LC/MS to confirm the glycosylation pattern of FI6-M or F10-M.

### EXAMPLE 4:

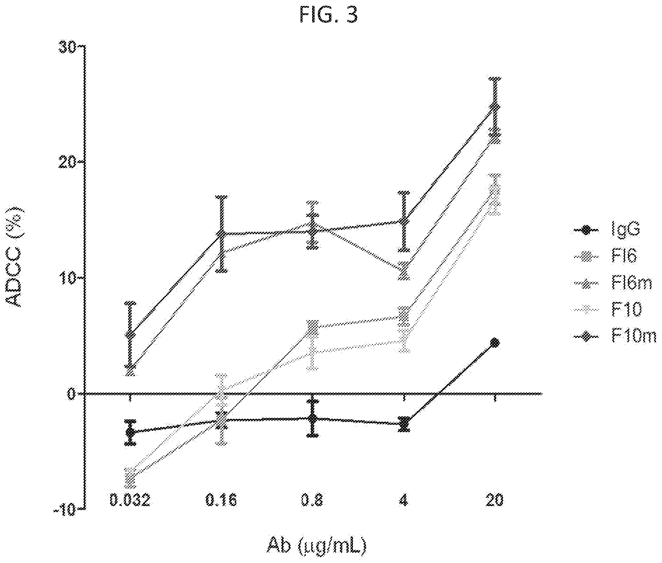

ADCC assay of FI6/F10 and glycoengineering FI6-M/F10-M. See Figure 3 demonstrated the enhanced ADCC results of anti-influenza virus antibodies.

Anti-viral antibody-dependent cell-mediated cytotoxicity (ADCC) enhancement is demonstrated with anti-influenza monoclonal antibodies FI6 and F10 with glycan modification. Human HEK293T cells were transiently transfected with plasmid to express full-length Cal/09 HA on the cell surface to mimic influenza virus infected cells. These cells were mixed with freshly prepared human peripheral blood mononuclear cells (PBMC) isolated from health donors with ratio of infected cells to PBMC of 1:20 or 1:50. Antibodies FI6 and F10 in different concentrations with and without glycan modification are then added into the mixtures. After 5 hours, the result of FI6 and F10 induced ADCC was monitored by HEK293T cell lysis (LDH release). The results show that the ADCC induced by antibodies FI6 and F10 with glycan modification is enhanced 1.5-3 folds.

### EXAMPLE 5: Exemplary Methods and Materials for ADCC assay

### EXAMPLE: Anti-stem monoclonal antibodies FI6 and F10

The F10 and FI6 antibody expression plasmids were transfected to HEK293F cell by using polyethyleneimine and cultured in Freestyle 293 expression medium (Invitrogen). After 7 days incubation, the supernatants were collected by centrifugation and the antibodies were purified by protein A beads (Roche Diagnostics). The antibody was further purified by gel filtration chromatography on Superdex 200 (GE Healthcare) in PBS buffer.

### EXAMPLE 6:

In vitro antibody-dependent cellular cytotoxicity (ADCC) assay

HEK293T cells were transfected with pVax-Cal/09 hemagglutinin (HA) expression plasmid for 48 hour. The HA-expressing HEK293T cells were trypsinized and seeded in 96-well U-bottom plates, 5,000 cells per well in 50 ul DMEM medium (Gibco).

Peripheral blood mononuclear cells (PBMCs) were prepared by Ficoll-Paque separation of whole blood obtained from healthy volunteers and used as effector cells in the ADCC assay. Briefly, whole blood was diluted with an equal volume of HBSS, layered over Ficoll-Paque plus (GE Healthcare) and centrifuged at 400 g for 40 min. The PBMC cells were harvested, washed twice with HBSS and mixed with HA-expressing HEK293T cells using an effector-to-target ratio of 50/1.

Mixture of PBMCs and HA-expressing HEK293T cells were treated with different concentrations of antibodies FI6 and F10 and incubated at 37°C for 5 hours.

After 5 hour incubation, ADCC was monitored by measuring the lactate dehydrogenase (LDH) released using cytoTox96 Non-Radioactive Cytotoxicity Assay kit (Promega).

EXAMPLE 7: Preparation of homogeneous antibody with universal glycan at the Fc region toward enhancing monoclonal antibody mediated anti-cancer therapeutics

Representative examples:
Commercial available Rituxan® and Herceptin® were used as starting material, after the same methods described previously for the preparation of homogeneous anti-influenza virus antibody with universal glycan at the Fc region. The homogeneous Rituxan® and Herceptin® with the optimized universal glycan Sia₂((α2-6)Gal₂GlcNAc₂Man₃GlcNAc₂ at the Fc region can be obtained. Using the same methods, we have also prepared different homogeneous Rituxan® and Herceptin® antibodies with different glycanform at their Fc region for the comparison of antibodies activities with different glycans.

Biological Characteristic of Anti-CD20 homogeneous antibody

Glycosylation on Fc can affect a variety of immunoglobulin effector-mediated functions, including ADCC, CDC and circulating half-life. ADCC enhancement is a key strategy for improving therapeutic antibody drug efficacy. It can lowering effective drug dosage for benefits of lower drug cost. The anti-CD20 homogeneous antibodies described herein can be characterized by functional properties. The anti-CD20 GAb has cell growth inhibitory activities including apoptosis against human CD20 expressing cells. In some embodiments, the anti-CD20 GAb exhibits more potent cell growth inhibitory activities as compared to its patent antibody.

### EXAMPLE 8:

### ADCC Activities of anti-CD20 glycoantibodies

The ADCC activity of the homogeneous antibody according to the invention is at least 8 fold increased, preferably at least 15 fold, more preferably at least 35 fold increased ADCC activity, preferably at least 50 fold increased ADCC activity, preferably at least 60 fold increased ADCC activity, most preferred at least 80 fold increased ADCC activity compared to the ADCC activity of the parental antibody.

The ADCC lysis activity of the inventive homogeneous antibody can be measured in comparison to the parental antibody using target cancer cell lines such as, for example, SKBR5, SKBR3, LoVo, MCF7, OVCAR3 and/or Kato III.

A number of anti-CD20 GAbs described herein, in particular GAb101, and GAb104, exhibited enhanced ADCC activity compared to it parental antibody, Rituximab. The homogeneous antibodies of the invention can exhibit superior effect as therapeutic agents for B cell-mediated malignant tumors and immunological diseases in which B cells or antibodies produced by B cells are involved, and an object of the present invention is to use the anti-CD20 GAb in development of therapeutic agents.

### Example 9: CDC Activities of anti-CD20 glycoantibodies

The homogeneous antibodies described herein are surprisingly able to provide improved ADCC without affecting CDC. Exemplary CDC assays are described in the examples. In exemplary embodiments, ADCC of the glycoantibody is increased but other immunoglobulin-type effector functions such as complement-dependent cytoxicity (CDC) remain similar or are not significantly affected.

### Binding between FcγRIII and anti-CD20 glycoantibodies

FcyRIIIA was transfected into HEK-293 cell line to express recombinant protein. The secreted FcyRIIIA recombinant protein was purified and then diluted to serial concentration in HBS-EP buffer (200 nM,100 nM, 50 nM, 25 nM, and 12.5 nM). Each of anti-CD20 GAbs101, 102, 104, 105, 106, 107, 108, 109, 110 and 111 was diluted in HBS-EP buffer to the concentration of 10 mg/ml, and then captured to the CM5 chip in which anti-human Fab domain antibodies were pre-immobilized. A serial titration of FcyRIIIA was injected and bound at the flow rate of 30 ml/min. Single cycle kinetics data was fitted into 1:1 binding model using Biacore T200 evaluation software to measure the equilibrium constant (Ka/Kd). The results were shown in Table 2.

Table 2 lists exemplary FcγRIIIA binding of anti-CD20 GAbs and Rituximab. FcyRIIIA binding may be measured using assays known in the art. Exemplary assays are described in the examples. The Fc receptor binding may be determined as the relative ratio of anti-CD20 GAb vs Rituximab. Fc receptor binding in exemplary embodiments is increased by at least 1.2-fold, 2-fold, 3-fold, 4-fold, 5-fold, 6-fold, 7-fold, 8-fold, 9-fold, 10-fold, 15-fold or 20-fold, 30-fold, 40-fold, 50-fold, 100-fold or higher.

### Table 2.

As compared to Rituximab, the binding data showed that the anti-CD20 GAbs, in particular GAb101 and GAb104, exhibit stronger binding affinity for the target molecule CD20.

Taken together, anti-CD20 GAbs, in particular GAb101, and GAb104, exhibited enhanced ADCC activity and stronger FcγRIIIA binding affinity as compared to Rituximab. The homogeneous antibodies of the invention can provide a superior clinical response either alone or, preferably, in a composition comprising two or more such antibodies, and optionally in combination with other treatments such as chemotherapy. The ADCC-enhanced anti-CD20 glycoantibody can provide an alternative therapeutic for B-cell lymphoma and other diseases. The glycoantibodies of the present invention can be used to alter current routes of administration and current therapeutic regimens, as their increased effector function means they can be dosed at lower concentrations and with less frequency, thereby reducing the potential for antibody toxicity and/or development of antibody tolerance. Furthermore, their improved effector function yields new approaches to treating clinical indications that have previously been resistant or refractory to treatment with the corresponding anti-CD20 monoclonal antibody produced in recombinant host systems.

### EXAMPLE 10: Binding to B-lymphoma cells

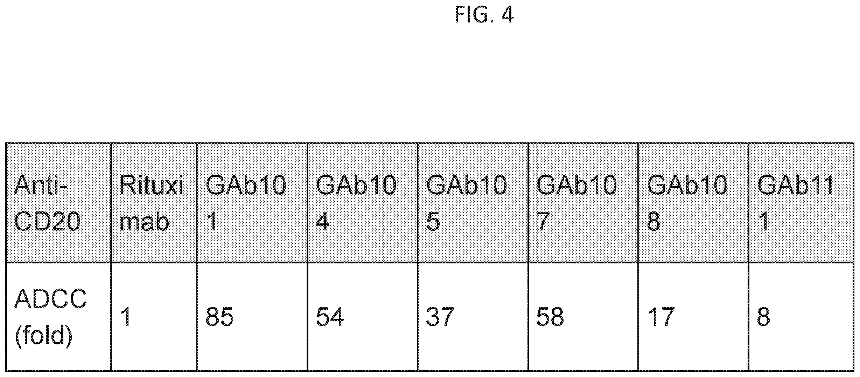

The binding activities of Rituxan-SCT (GAb101) and Rituxan mono-GlcNAc to Ramos cells, Raji and SU-DHL-4 cells were examined, and the results showed both have similar binding activities as Rituximab (Fig. 4).

Figure 4. Binding activities of different homogeneous antibodies with different cells with CD20.

### EXAMPLE 11: CDC to B-lymphoma cells

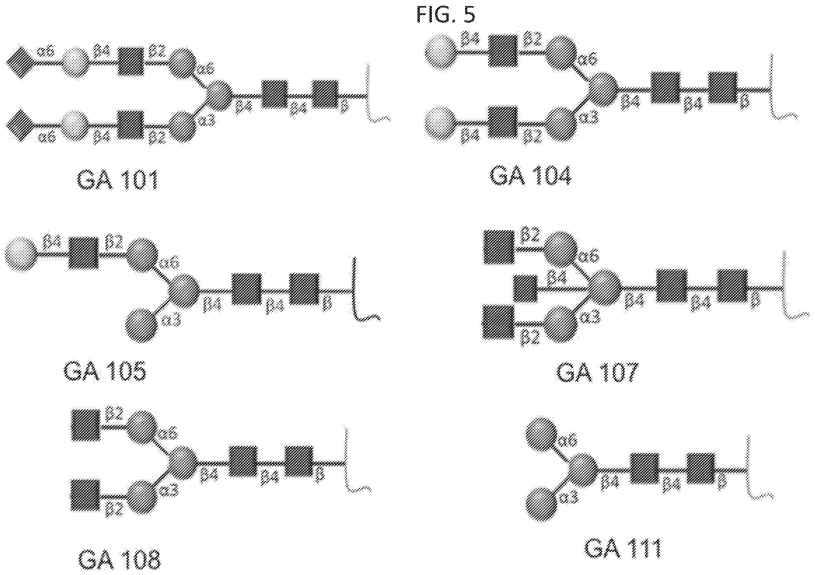

The CDC effects of Rituxan-SCT (GAb101) and Rituxan mono-GlcNAc to Ramos cells, Raji and SU-DHL-4 cells were tested. The comparative CDC profiles seen with Ramos cells (enhanced by GAb101 and reduced by Riruxan-GlcNAc) were confirmed in the other B-lymphoma cell line SU-DHL-4 (Fig. 5 right panel). Reproducible results were obtained when conducted on a second occasion using different cell passages.

### EXAMPLE 11:

### See Figure 5. Depletion of human B cells

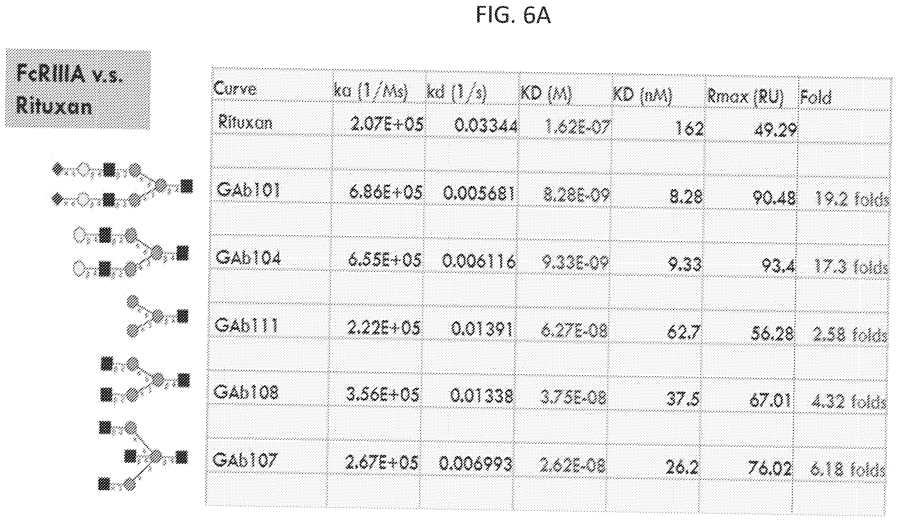

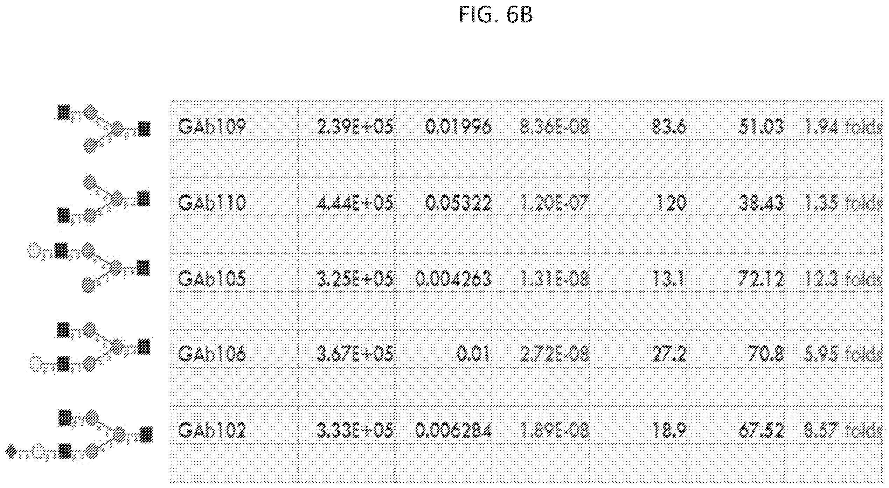

The depletion of human B cells was conducted using human PBMC cells freshly prepared from human blood. The cells at 2x10⁶ in RPMI 1640-5%FBS cultured on microplates were incubated, in the absence or presence of 15% autologous plasma, at 37 °C for 4 hr with the anti-CD20 GAbs Rituxan-SCT, Rituxan-GlcNAc and Rituximab at different concentrations. The cells after wash were stained with anti-CD2-PE and anti-CD 19-FITC on ice for 5 min. B cells depletion was analyzed on FACS, based on the CD19⁺ CD2⁻ B cells. (Figure 6) See Figure 6. Depletion of human B cells by different homogeneous antibodies.

### EXAMPLE 12: Binding to B-lymphoma cells.

The binding of the antibodies was investigated in CD20⁺ B lymphoma cell lines (Ramos, Raji, and) and analyzed on flow cytometry. The cells in PBS containing 1% fetal bovine serum at 2x10⁵/well on microplate were incubated on ice for 1 hr with antibodies of interest at different concentrations. The cells are washed, re-suspended in the PBS buffer, and incubated with the detecting goat anti-hIgG-Fcγ-PE on ice for 30 min. The cells are washed and subjected to analysis on FACS.

### Example 13: Binding to FcRIIIa-expressing CHO cells.

The binding of the antibodies to the FcRIIIa receptors (CD 16a), which is a precursor event known to be correlative with the induction of antibody-dependent cellular cytotoxicity (ADCC), was investigated in CHO cells transfected with the high-affinity CD16a (158Val) and analyzed on flow cytometry. The cells in PBS containing 1% fetal bovine serum at 1x10⁵/well on microplate were incubated on ice for 1 hr with antibodies of interest at different concentrations. The cells are washed, re-suspended in the PBS buffer, and incubated with the detecting goat anti-hIgG-Fcγ-PE on ice for 30 min. The cells are washed and subjected to analysis on FACS.

Complement-dependent cytotoxicity (CDC) to B-lymphoma cells. The CDC effect induced by the antibodies were investigated in CD20+ B lymphoma cell lines (Ramos and SKW6.4) and analyzed on flow cytometry. The cells in RPMI 1640 culture medium at 2.0x105/well on microplates were incubated on ice for 30 min with antibodies of interest at different concentrations. The cells were washed and incubated at 37 oC for 30 min with 10% human serum in RPMI 1640. The cells were washed and incubated in the dark for 5 min with the PI reagent. The cell deaths by CDC were analyzed on FACS.

Antibody-dependent cellular cytotoxicity (ADCC) to B-lymphoma cells. The ADCC effect induced by the glyco-antibodies were investigated in CD20-containing B lymphoma cell lines (Ramos and SKW6.4), using freshly prepared human PBMC as effector cells, and the results analyzed on flow cytometry. The target B cells in PBS-0.1%BSA were first labeled with CFSE at 37 oC for 5 min. After wash the CFSE-labeled cells in RPMI 1640 medium were incubated at 37 oC for 4 hr on microplates with the glyco-antibodies of interest at different concentrations and PBMC effector cells. The ratio of target cells to effector cells was set at 25:1. The resultant mixtures were stained in the dark for 5 min with the PI reagent. The cell deaths by ADDC were analyzed on FACS.

Depletion of human B cells. The depletion of human B cells was conducted using human PBMC cells freshly prepared from human blood. The cells at 2x106 in RPMI 1640-5%FBS cultured on microplates were incubated, in the absence or presence of 15% autologous plasma, at 37 oC for 4 hr with the antibodies of interest at different concentrations. The cells after wash were stained with anti-CD2-PE and anti-CD 19-FITC on ice for 5 min. B cells depletion was analyzed on FACS, based on the CD19+ CD2- B cells.

Preparation of homogeneous Herceptin® by the strategy of glycan engineering.

Methods to prepare different glycan modified homogeneous Herceptin®.

### Biological Characteristic of Anti-HER2 homogeneous antibodies

Glycosylation on Fc can affect a variety of immunoglobulin effector-mediated functions, including ADCC, CDC and circulating half-life. ADCC enhancement is a key strategy for improving therapeutic antibody drug efficacy. It has the potential of lowering effective drug dosage for benefits of lower drug cost. The anti-HER2 glycoantibodies described herein can be characterized by functional properties. The anti-HER2 GAb has cell growth inhibitory activities including apoptosis against human HER2 expressing cells. In some embodiments, the anti-HER2 GAb exhibits more potent cell growth inhibitory activities as compared to its patent antibody.

### ADCC Activities of anti-HER2 glycoantibodies

The ADCC activity of the glycoantibody according to the invention is at least 3 fold increased, preferably at least 9 fold, more preferably at least 10 fold increased ADCC activity, preferably at least 12 fold increased ADCC activity, preferably at least 20 fold increased ADCC activity, most preferred at least 30 fold increased ADCC activity compared to the ADCC activity of the parental antibody.

The ADCC lysis activity of the inventive glycoantibody can be measured in comparison to the parental antibody using target cancer cell lines such as SKBR5, SKBR3, LoVo, MCF7, OVCAR3 and/or Kato III.

Table 3 lists exemplary enhanced ADCC activities of anti-HER2 GAbs as compared to Trastuzumab. Exemplary assays are described in the examples.

**Table 3.**

| Anti-HER2 | Trastuzumab | GAb101 | GAb104 | GAb105 | GAb107 | GAb108 | GAb111 |
|---|---|---|---|---|---|---|---|
| ADCC (fold) | 1 | 30 | 14.3 | 9.5 | 10 | 6.5 | 3 |

A number of anti-HER2 GAbs described herein, in particular GAb101, and GAb104, exhibit enhanced ADCC activity compared to it parental antibody, Rituximab. It is contemplated that the glycoantibodies of the invention may exhibit superior effect as therapeutic agents for HER2-positive diseases, and an object of the present invention is to use the anti-HER2 GAb in development of therapeutic agents.

### CDC Activities of anti-HER2 glycoantibodies

The glycoantibody described herein is surprisingly able to provide improved ADCC without affecting CDC. Exemplary CDC assays are described in the examples. In exemplary embodiments, ADCC of the glycoantibody is increased but other immunoglobulin-type effector functions such as complement-dependent cytoxicity (CDC) remain similar or are not significantly affected.

### Binding between FcyRIII and anti-HER2 glycoantibodies

Table 4 lists exemplary FcγRIIIA binding of anti-HER2 GAbs and Rituximab. Table 4.

FcγRIIIA binding may be measured using assays known in the art. Exemplary assays are described in the examples. The Fc receptor binding may be determined as the relative ratio of anti-HER2 GAb vs Trastuzumab. Fc receptor binding in exemplary embodiments is increased by at least 2.5-fold, 3-fold, 4-fold, 5-fold, 6-fold, 7-fold, 8-fold, 9-fold, 10-fold, 15-fold or 20-fold, 30-fold, 40-fold, 50-fold or higher.

As compared to Trastuzumab, the binding data showed that the anti-HER2 GAbs, in particular GAb101 and GAb104, exhibit stronger binding affinity for the target molecule HER2.

Taken together, anti-HER2 GAbs, in particular GAb101, and GAb104, exhibit enhanced ADCC activity and stronger FcγRIIIA binding affinity as compared to Trastuzumab. It is contemplated that the glycoantibodies of the invention may provide a superior clinical response either alone or, preferably, in a composition comprising two or more such antibodies, and optionally in combination with other treatments such as chemotherapy. It is contemplated that the ADCC-enhanced anti-HER2 glycoantibody may provide an alternative therapeutic for HER2-positive diseases. The glycoantibodies of the present invention advantageously can be used to alter current routes of administration and current therapeutic regimens, as their increased effector function means they can be dosed at lower concentrations and with less frequency, thereby reducing the potential for antibody toxicity and/or development of antibody tolerance. Furthermore, their improved effector function yields new approaches to treating clinical indications that have previously been resistant or refractory to treatment with the corresponding anti-HER2 monoclonal antibody produced in recombinant host systems.

Preparation of homogeneous antibody with universal glycan (SCT) at the Fc region toward enhancing monoclonal antibody mediated anti-inflammation therapeutics

The Fc region with siaa2,6Gal structure can increase the activities of anti-inflammation. Here we prepare the homogeneous Humira with SCT glycan at the Fc region to improve its anti-inflammation activities.

General procedure for analysis of N-glycosylation of anti-TNFα,

We developed a mass spectrometric method to monitor the yield of oligosaccharide-derived fragment ions (oxonium ions) over a collision induced dissociation (CID) energy applied to a glycopeptides precursor. Multiple Reaction Monitoring (MRM) of oxonium ions method could fulfill the regulatory requirement on the routine quality control analysis of forthcoming biosimilar therapeutics.

5 ug of Adalimumab(Humira®) (purchased from Abbvie) was dissolved in 25 ul of 2M Guanidine-HCl, and dithiothreitol (DTT) were added to a final concentration of 5 mM. After 10 minutes incubation in 110 °C, reduced cysteine residues were alkylated in 10 mM Iodoacetamide (IAA) at 37 °C for 1 hour. Add 5 mM DTT to quench excess IAA at RT for 10 minutes. The product was diluted 15 times in 50 mM ammonium bicarbonate before microcentrifugation with spin column (10kDa protein MW cut-off). The trypsin digestion was performed for 4 hours at 37 °C using an enzyme: protein ratio of 1: 25 (w/w). Sample was frozen at -20 °C for LC-MS/MS analysis.

### Instrumentation

The glycopeptide quantification by m/z 204 oxonium ion (HexNAc) monitoring was performed using a 4000 QTrap triple quadrupole mass spectrometer (AB Sciex) with Aglient 1200 HPLC system. For relative quantification of glycopeptide microheterogeneity, precursor ion m/z was derived in-silico, covering all possible glycan compositions, and a single quantitative transition was monitored for each precursor ion (Q3 m/z= 204).

### MS data analysis

The acquired raw data was processed with Analyst 1.5 (AB Sciex). The mass chromatogram of each transition was integrated and quantified by peak area. The percentage composition of each component was calculated with respect to the sum of all components combined.

### Preparation of Anti-TNFα Antibody Humira-SCT

Isolation of the sialylglycopeptide (SGP) from hen's egg yolk was according to the published method. Briefly, the phenol extraction of hen's egg yolk was centrifuged, filtrated, and purified by the chromatographic columns, including Sephadex G-50, Sephadex G-25, DEAE-Toyoperarl 650M, CM-Sephadex C-25 and Sephadex G-25. A solution of sialylglycopeptide (SGP) (52 mg) in a sodium phosphate buffer (50 mM, pH 6.0, 5mM) was incubated with the Endo M (53 µg) at 37 °C. After 7 hour, the reaction mixture was subjected to gel filtration chromatography on a Sephadex G-25 column eluted by water. The fractions containing the product were combined and lyophilized to give the product (glycan-101) as a white powder (30 mg, yield 82%).

A solution of glycan-101 (Sia₂(α2-6)Gal₂GlcNAc₂Man₃GlcNAc) (30 mg), 2-chloro-1,3-dimethylimidazolinium chloride (DMC) (62.7 mg) and Et₃N (89 µL) in water was stirred at 4°C for 1 h. The reaction mixture was subjected to gel filtration chromatography on a Sephadex G-25 column and eluted by 0.05% aqueous Et₃N. The fractions containing the product (SCT oxazoline) were combined and lyophilized to give a white powder.

SCT oxazoline was added to a mixture of endoglycosidase and GAb Humira-GlcNAc in 50 mM Tris buffer (pH 7.8) and incubated for an hour at room temperature. The reaction mixture was purified with protein A affinity column, followed by amanion exchange column capto Q to collect the desired product, anti-TNFα, GAb101. The product was trypsinized, and the glycopeptides, TKPREEQYNSTYR and EEQYNSTYR, were analyzed using nanospray LC/MS to confirm the glycosylation pattern of Humira-SCT.

### Binding Affinity of anti-TNFα

Human recombinant TNF-α, containingg 158 amino acids (MW=17.5 kDa) was produced in *E. coli* (PROSPEC) and purified. Recombinant human TNF-α protein was titrated and a serial dilution of 50 nM, 25 nM, 12.5 nM, 6.25 nM, and 3.125 nM was prepared in HBS-EP buffer. Adalimumab and anti-TNFα, GAb 200 and 401 were diluted in HBS-EP buffer to a concentration of 10 µg/ml, and then captured to the CM5 chip where anti-human Fc domain antibodies were pre-immobilized. Serial concentration of recombinant human TNF-alpha as the analyte and then injected and bound to the captured antibody on chip at the flow rate of 30 µl/min. After binding, the antibody-analyte complex were washed by regeneration buffer, 10 mM glycine-HCl pH1.5 at the flow rate of 50 µl/min. CM5 chip was maintained in PBS pH7.4 at 4 °C for further use. Single cycle kinetics data was fitted into 1:1 binding model using Biacore T200 evaluation software to measure the equilibrium constant (Ka/Kd).

### EXAMPLE 13: Generation of Anti-SSEA-4 Monoclonal Antibodies

Hybridoma methodology was employed for the development of mAbs specific to SSEA-4. Female BALB/c mice, aged 6-8 weeks old, were immunized three times subcutaneously with the SSEA-4 vaccine. Three immunizations were given at 2-wk intervals. Each vaccination contained 2µg of SSEA-4. All of the sera were obtained by centrifugation at 4,000 × g for 10 min. The serologic responses were analyzed by glycan microarray. A final boost was given intraperitoneally with 2 µg of SSEA-4, and 3 days later, the spleen cells from immunized mice were used for generating hybridomas.

Hybridoma cells secreting antibodies with the desired antigen-binding activities were screened as follows. Microtiter plates were coated by incubating with 4 µg/mL of neutravidin in carbonate buffer, 0.1M, pH 9.6, overnight at 4°C. The wells were blocked with 1% BSA in PBS, pH=7.3 for 1 hour and incubated with 4 µg/mL SSEA-4-biotin for 1hour. The antisera were at various dilutions for 1 hour at 37°C. After washing, the ligand-bound antibodies were detected by HRP-conjugated goat anti-mouse IgG or IgM antibody (Jackson ImmunoResearch) at 1: 10,000 and incubated for 1 hour at 37°C, followed by incubation with TMB substrate. The OD was determined at 450 nm. Positive clones were selected for further characterization. Three exemplary clones 45, 46 and 48, were identified in this study as specifically binding to SSEA-4. For mouse monoclonal isotyping, the IsoQuick Strips and Kits was used (sigma, I9535). Add hybridoma medium to the reaction vial. Insert the strip into the sample making sure the strips are upright. The sample will travel up the strip. Allow the strip to develop for 5 minutes before making final interpretations.

The V_{H} and V_{L} gene segments of the mAbs 45, 46 and 48 were amplified by PCR from the hybridoma clone secreting the antibody. The gene segments thus obtained were sequenced to determine the V_{H} and V_{L} sequences of mAbs 45, 46 and 48, which are shown in Tables 3-5.

### EXAMPLE 14: Generations of Chimeric Antibodies

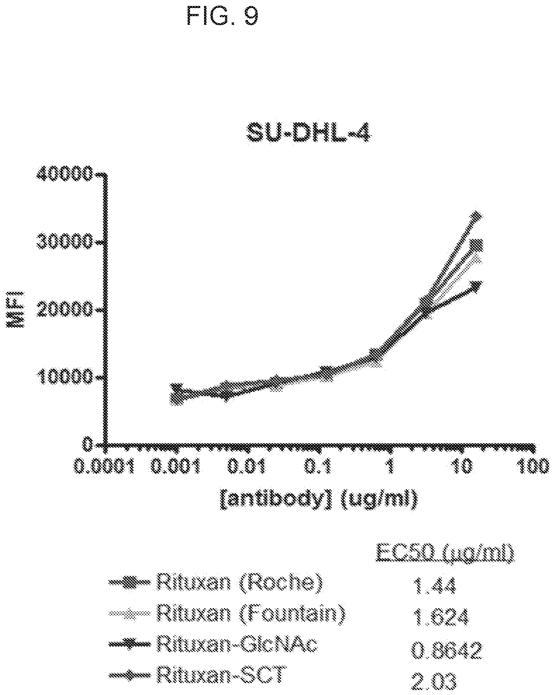

The V_{H} and V_{L} gene segments of the mAb 273 and 651 were amplified by PCR from the hybridoma clone secreting the antibody. The gene segments thus obtained were sequenced to determine the V_{H} and V_{L} sequences of mAb 273 and 651, which are shown in Tables 1 and 2. The heavy chain and light chain variable region were cloned to human IgG1 antibody expression vector show as Fig.9. VH was using enzyme site *BsiWI* and *ApaI,* and VL was using enzyme site *BsPEI* and *NheI.* Vectors were transiently transfected into either 293F or CHO-S cells. Recombinant chimeric Ab was purified and further study for binding assay and complement-dependent tumor cell lysis assay.

The V_{H} and V_{L} gene segments of the mAb 46 and 48 were amplified by PCR from the hybridoma clone secreting the antibody. The gene segments thus obtained were sequenced to determine the V_{H} and V_{L} sequences of mAb 46 and 48, which are shown in Tables 5 and 4. The heavy chain and light chain variable region were cloned to human IgG1 antibody expression vector show as Fig.9. VH was using enzyme site *BsiWI* and *ApaI,* and VL was using enzyme site *BsPEI* and *NheI.* Vectors were transiently transfected into either 293F or CHO-S cells. Recombinant chimeric Ab was purified and further study for binding assay and complement-dependent tumor cell lysis assay.

### EXAMPLE 15: Binding Analysis of Antibodiesto Cancer CellsBy Flow Cytometry

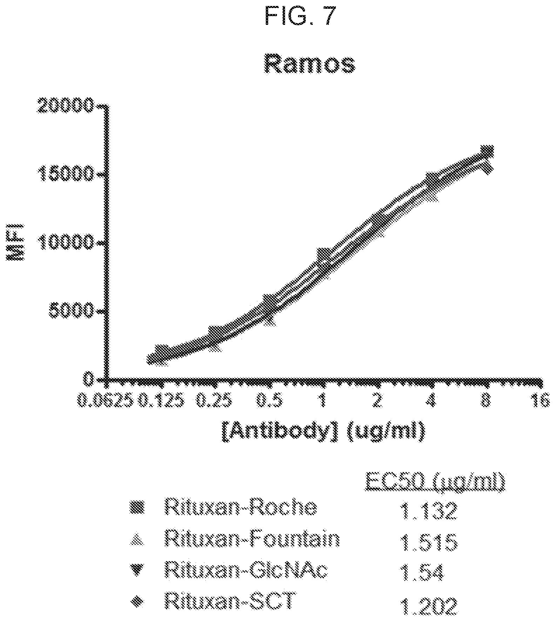

Binding of mAb 273 and anti-SSEA-4 (mAbs 45, 46 and 48) to cancer cell lines were examined. Cells (1×10⁵) were resuspended in 100µL FACS buffer (1% BSA/PBS solution) containing various concentration antibody and incubated on ice for 30 min. After being washed twice with FACS buffer, cells were incubated with 649-labeled goat anti-mouse antibody (1:100; Jackson ImmunoResearch) for 30 min on ice before analysis on a FACSCalibur system (BD Biosciences). The results are shown in Figures 7A-D. Breast cancer cells MCF-7 were stained with mAb 273 (Fig. 7A). Pancreatic cancer cells (HPAC and BxPC3) and breast cancer cells MCF-7were stained with mAb 45 (Fig. 7B). Pancreatic cancer cells (HPAC and BxPC3) and breast cancer cells MCF-7 were stained with mAb 46 (Fig. 7C). Pancreatic cancer cells (HPAC and BxPC3) and breast cancer cells MCF-7were stained with mAb 48 (Fig. 7D).

We also used the glycan array to determine the dissociation constants of MC45, MC48 and MC813-70 with SSEA-4 hexasaccharide on surface, and the Kd values for MC45, 48 and 813 are shown below. These results showed that these mAbs are highly specific for SSEA4.

| | **Kd (nM) ± SD(nM)** |
|---|---|
| **MC45** | 0.37 ± 0.08 |
| **MC48** | 0.46 ± 0.1 |
| **MC813-70** | 4.21 ± 0.26 |

Example 16: The ability of exemplary mAbs 46 and 48 to mediate CDC of SSEA-4 expressing cells was examined. Homo sapiens pancreas adenocarcinoma cell (BxPC3) in the presence of rabbit serum as a source of complement. Cell death was assessed by the addition of the viability probe 7-AAD. Based on the results of the 7-AAD measurement, percentage-specific lysis was calculated using a FACScan flow cytometer. The antibodies showed about 20% killing activity at 40µg/mL. As shown in Figure 5(C), mAbs 46 and 48 successfully mediated CDC of SSEA-4 expressing cells.

### EXAMPLE 15 Exemplary Phage display biopanning procedures

The phage-displayed human naive scFv library contained 2.5×10¹⁰ clones (Lu et al., 2011) was subtracted with non-specific binding in PEG-conjugated carboxyl Dynabeads (Invitrogen) at room temperature (RT) for 1 hour, and subsequently incubated with SSEA-4-PEG immobilized Dynabeads at 4°C for 1 hour. After washing with PBS or PBS containing 0.01% Tween 20 (PBST0.01), the phages that bound to SSEA-4-PEG-Dynabeads were recovered by infection with E-coli TG1 cells at 37°C for 0.5 hour. Some of the infected cells were serially diluted to determine titer, and the others were rescued by M13KO7 phage and amplified. After determination of rescued phages titer, the next round of biopanning was performed. In the fourth and fifth round of biopanning, the phage clones were randomly selected to culture for ELISA screening.

### ELISA screening of selected phage clones

For detection of antigen recognition, microwell plates (Nunc) were coated with 0.2 µ g/ml of SSEA-4-BSA, Globo H-BSA, SSEA-3-BSA and BSA, respectively. The selected phage clones were diluted 1:2 in PBS containing 3% BSA and added to each well. The plates were incubated at RT for 1 hour, washed with PBST0.1, and incubated with horseradish peroxidase (HRP)-conjugated mouse anti-M13 phage antibody (GE Healthcare). The plates were washed again, and OPD and H2O2 were added. After termination of reaction by 3 N HCl, the absorbance was measured using a 490 nm using microplate reader (Model 680, BioRad). We extracted phagemids from ELISA-positive phage clones to identity scFv coding regions by auto-sequencing.

### Construction and expression of anti-SSEA-4 human IgG

The VH region of selected scFv was cloned with AgeI and NheI site into modified expression vector pcDNA5-FRT-Gamma1 containing a signal peptide and the constant region of human immunoglobulin gamma 1 heavy chain. The VL region of selected scFv was cloned with AgeI and EcoRV site into modified expression vector p-Kappa-HuGs containing a signal peptide and constant region of human immunoglobulin kappa light chain. Both plasmids were transfected into FreeStyle293 cells (Invitrogen) and continuously incubated in serum-free medium at 37°C for 1 week to produce human antibody.

### Purification of anti-SSEA-4 human IgG

The culture medium was collected, centrifuged and filtrated with 0.45 µm pore-size membrane. The supernatant then was subjected to protein G column chromatography (GE healthcare) for purification of anti-SSEA-4 human IgG. After dialysis of eluents with PBS, the antibody was examined by SDS-PAGE analysis with coomassie blue staining as usual. The concentration of antibody was assessed by Bradford reagent (Thermo Scientific) and spectrophotometer.

### Humanization of MC48

Two human genes, GenBank accession Q9UL73 and AY577298, were the most similar to MC48 VH and VL, respectively. We humanized three sequences of MC48, including the 1st humanized MC48 (hMC48) VH consisted of modified framework (FR) 1 to FR4 of Q9UL73 gene and the 1st hMC48 VL consisted of four FRs from the accession AY577298, the 2nd hMC48 FRs of VH followed 1YY8 from PDB, while the 2nd hMC48 VL same as 1st sequence, and the 3rd hMC48 VH sequence modified FR1, 2 and 4 of Q9UL73 gene and the 3rd hMC48 VL changed FR2 and FR4 to human AY577298 gene. All of these humanized sequences were conserved CDR1 to CDR3 of VH and VL of MC48.

### Construction of single chain fragments variable (scFv) of humanized MC48 variants

The scFv form of humanized MC48 sequences (VH-GGGGSGGGGSGGGGS-VL (SEQ ID NO: 115)) were gene synthesized (Genomics) and cut by Sfi I and Not I (Fermentas). After gel extraction, the digested products were cloned to pCANTAB-5E phagemid (GE Healthcare).

Generation of humanized MC48 (hMC48) scFv phage clones.

hMC48 variant phagemids were transformed to TG1 E-coli and recovered in 2 × YT medium (BD Pharmingen) containing 100 µg/ml ampicillin and 2% glucose and rescued by M13KO7 helper phage (NEB) for 1 hour at 37°C. After centrifugation by 1,500 × g for 10 min, these pellets were resuspended in 2 × YT medium containing 100 µg/ml ampicillin and 50 µg/ml kanamycin overnight to generate scFv-phages.

### Binding assay of hMC48 scFv phage clones by ELISA

SSEA-4-BSA was coated on an ELISA plate at the concentration of 0.2 µg/ml. After washing and blocking, the serial diluted phages were incubated at RT for 1.5 hour. After washing, 1:1000 diluted HRP-conjugated anti-M13 antibody (GE Healthcare) was added at RT for 1 hour. Then, liquid substrate 3,3',5,5'-tetramethylbenzidine (TMB) developed and was terminated with 3N HCl. Optical density was measured at 450 nm.

### Results

### Identification of phage-displayed scFv that binds to SSEA-4

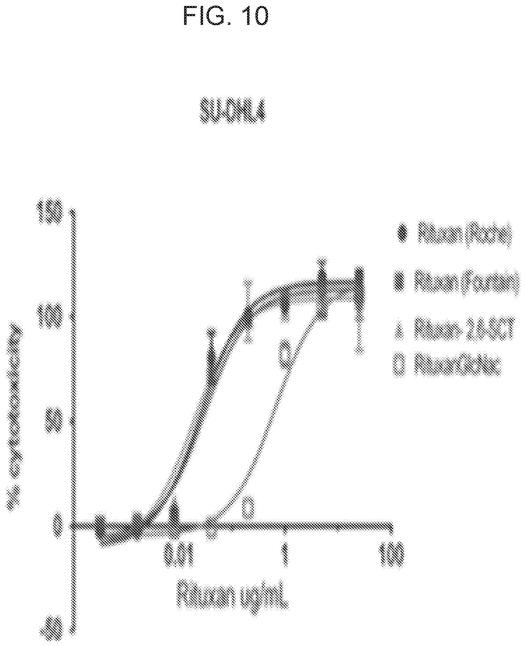

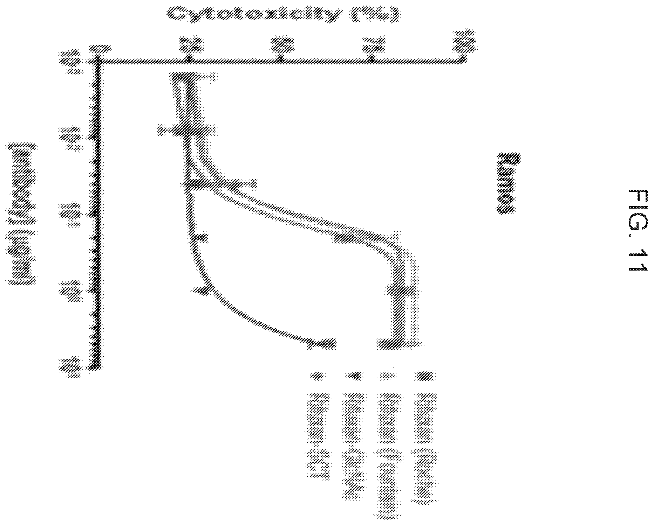

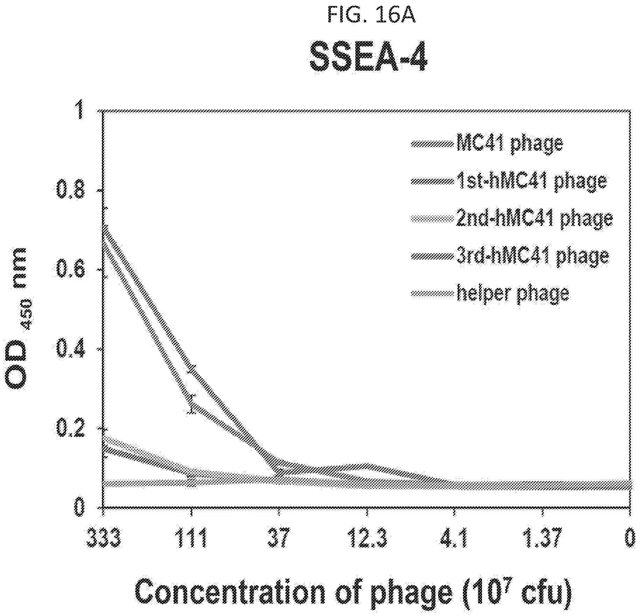

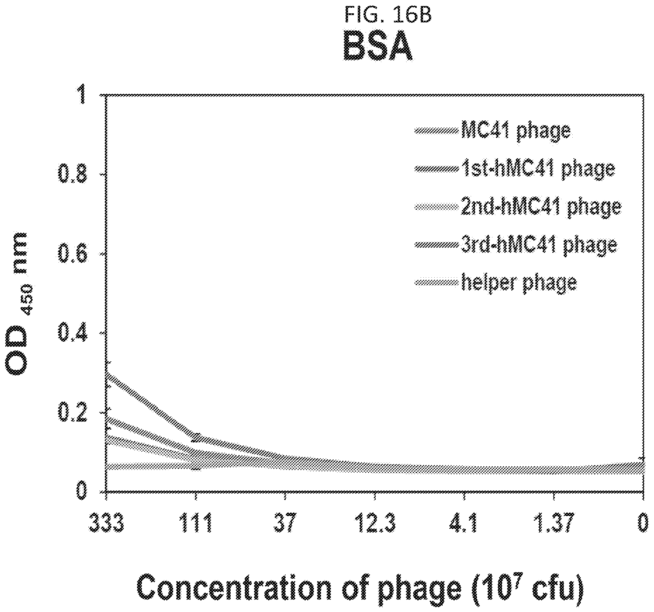

To identify the antibodies that bind to SSEA-4, we used phage-displayed human naive scFv library containing 2.5×10¹⁰ members which was established as our previous report described (Lu et al., 2011). This library was first removed Dynabeads-binding phages and then selected for SSEA-4-binding phages by SSEA-4-PEG-conjugated Dynabeads. We used two buffer systems, PBS and PBS containing 0.01% Tween20 (PBST0.01), during biopanning. After five rounds of affinity selection, the phage recovery of the fifth round had increased about 55-fold and 80-fold than that of the first round in PBS and PBST0.01 system, respectively (Fig. 10). The phage clones were randomly selected and tested for SSEA-4 binding by ELISA (Fig. 11). We found seven clones that specifically bound to SSEA-4-BSA, but not to BSA control protein. By sequencing all 8 individual clones, we identified two unique anti-SSEA-4 phage clones (p1-52 and p2-78) which contain distinct human VH and VL coding regions (Fig 16A).

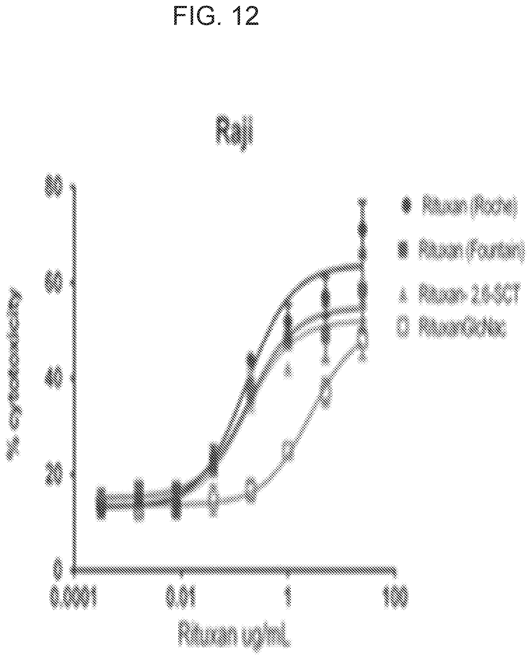

To examine the specificity and binding affinity of the two phage clones, we performed a comparative ELISA using the same phage titer to Globo-series glycans including SSEA-4-BSA, Globo H-BSA and SSEA-3-BSA (Fig. 12). The p2-78 phage clone showed the strong binding to SSEA-4-BSA and SSEA-3-BSA, and slightly weaker binding to Globo H-BSA. However, we found that the binding activity of p1-52 phage clone to SSEA-4-BSA is very weak. Thus we focused on p2-78 clone for further study.

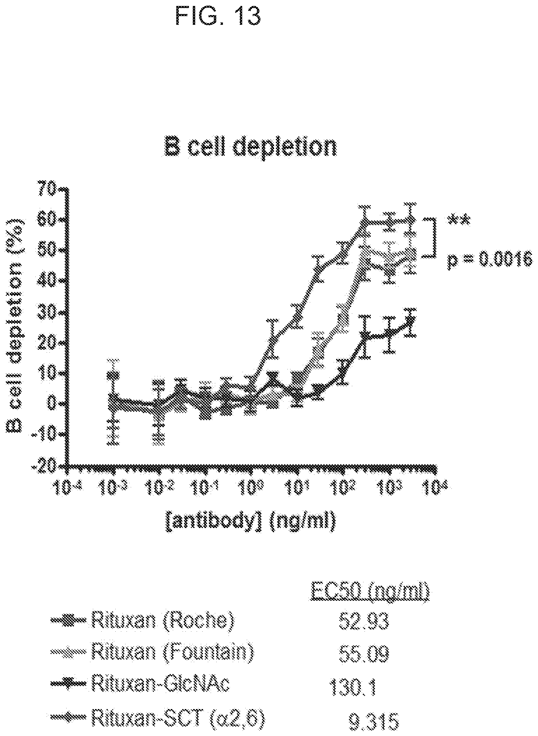

To establish the fully human antibody (hAb) against SSEA-4, we molecularly engineered the VH and VL coding sequences of p2-78 scFv into human IgG1 backbone, respectively. The anti-SSEA-4 p2-78 hAb was produced using FreeStyle 293 expression system and then purified through the protein G sepharose column. We examined the purity of antibody by SDS-PAGE analysis with coomassie blue staining (Fig. 13A). The result shows the purity level of antibody exceed 95%. Subsequently, we performed ELISA to investigate the binding activity of p2-78 hAb for Globo-series glycans (Fig. 13B). We found that p2-78 hAb bound to SSEA-4 and SSEA-3, but not to Globo H, which demonstrates the human IgG version of p2-78 retains the activity of its parental scFv version to recognize the binding epitope of SSEA-4.

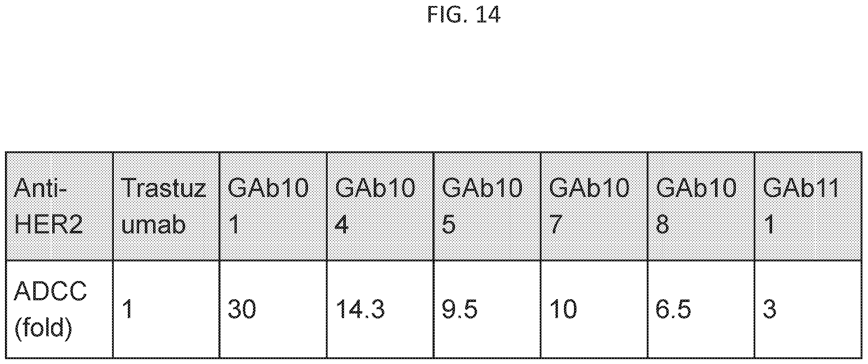

We used glycan array containing 203 different glycans to further confirm the specificity of p2-78 hAb. The results showed that p2-78 hAb recognized SSEA4, Sialyl-SSEA4, SSEA4Gc, and Gb5 (SSEA3) (Fig. 14B). Interestingly, p2-78 hAb also recognized GloboH, similar to the results from ELISA assay (Fig. 12). The commercially available IgM antibody, MC631, was used as a positive control (Fig. 14A).

### Development of humanized MC48 mAbs

Non-humanized Murine mAbs may have certain limitations in clinical settings, including their short serum half-life, inability to trigger human effector functions and the production of human anti-murine antibodies (HAMA) response (LoBuglio et al., 1989). Therefore, mAbs can be humanized by grafting their CDRs onto the VH and VL FRs of human Ig molecules (Roguska et al., 1994).

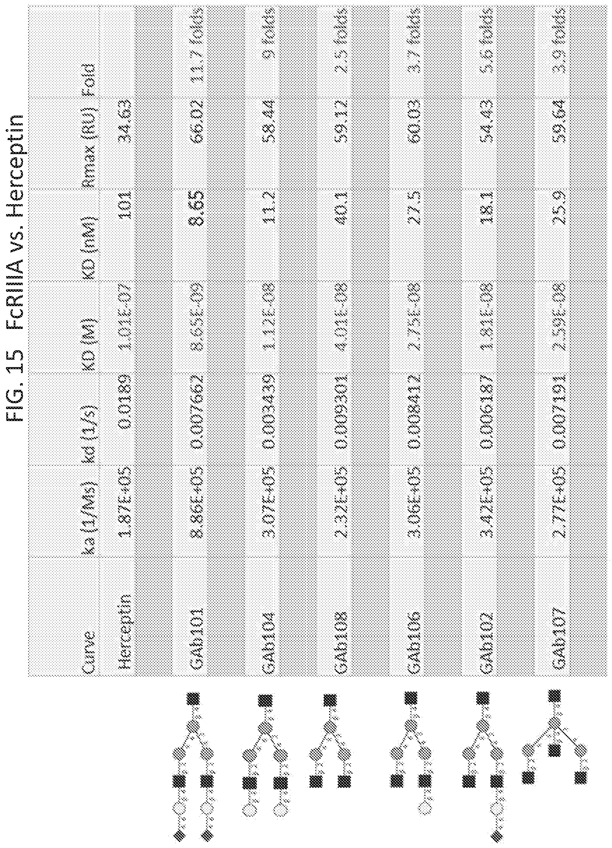

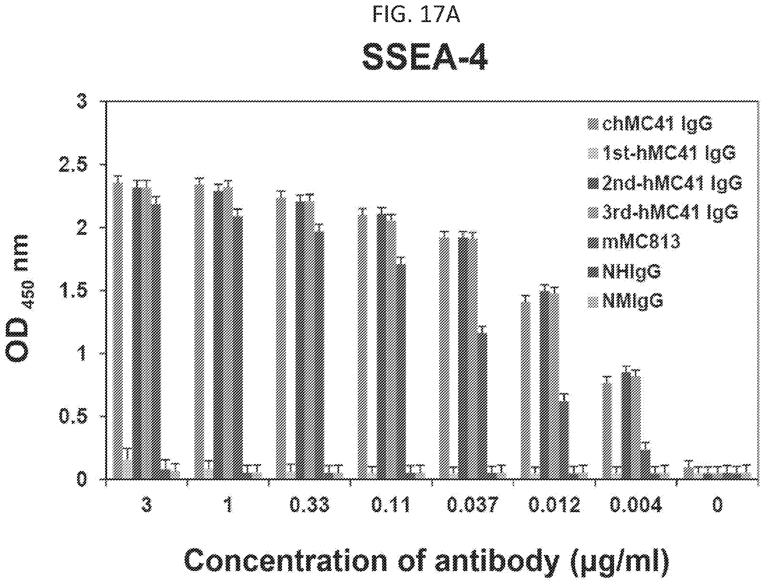

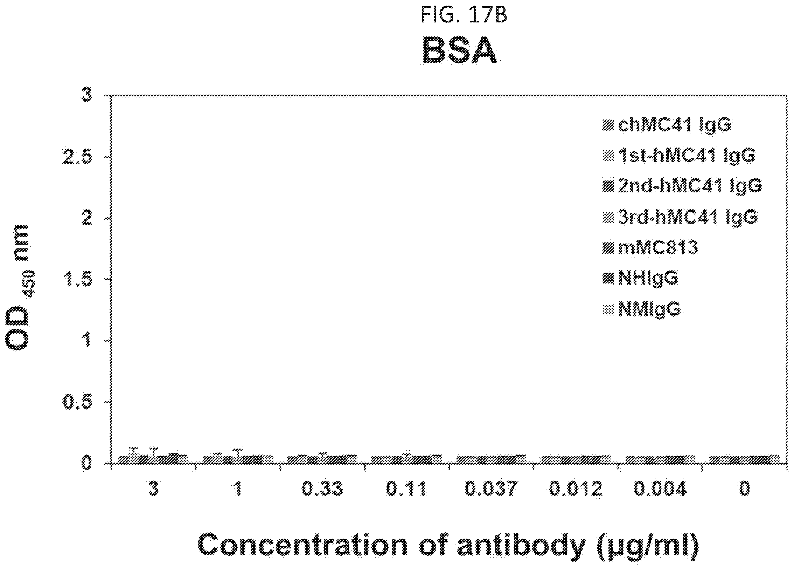

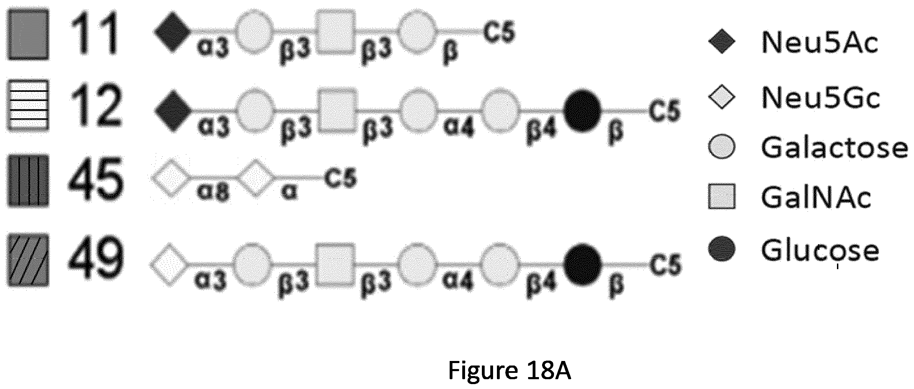

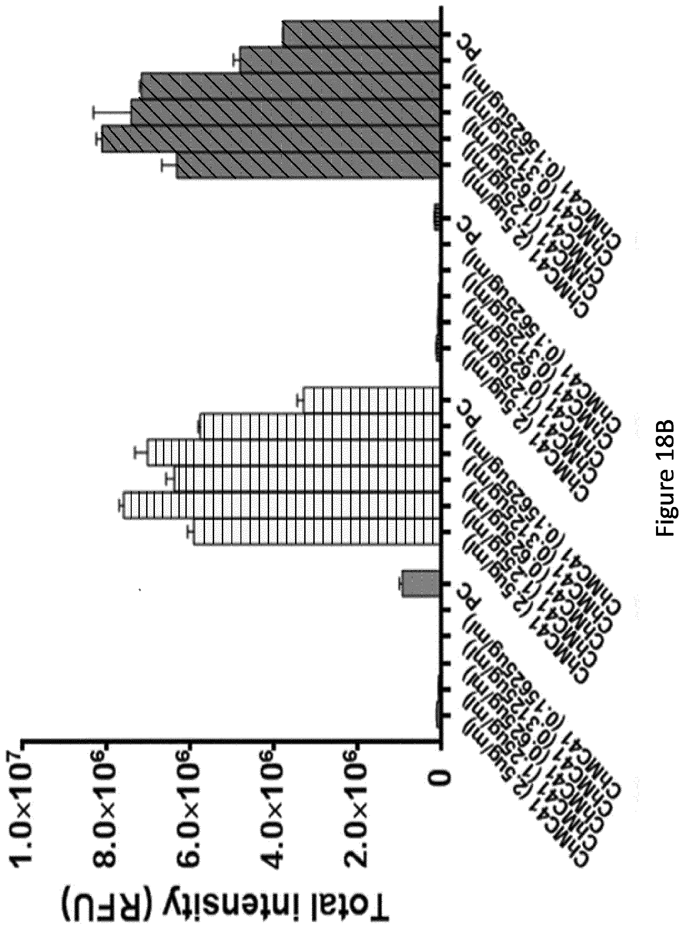

To develop humanized MC48, we sequenced V_{H} and V_{L} variable region of MC48 from a hybridoma cell (Table 4). After alignment of V_{H} and V_{L} variable region of MC48 with the NCBI IgBLAST database, we modified FRs of MC48 and generated 1^{st}, 2^{nd}, 3^{rd} and 4^{th} humanized MC48 sequences (Table 17, Figure 17). We next constructed and generated the phage-displayed scFv formats according to these humanized MC48 sequences. To determine the binding activity of the humanized MC48 phage clones, we carried out solid-based ELISA coating SSEA-4-BSA (Figs. 15 and 18). We found that the humanized MC48 scFv phage could recognize SSEA-4 in a dose-dependent manner. The data indicated that the 4^{th} humanized MC48 scFv phage maintained its binding affinity compared with the murine mAb MC48.

### Example 16

Complement-Dependent Cytotoxicity (CDC) Assay.

The ability of exemplary humanized MC 48 to mediate CDC of SSEA-4 expressing cells is examined. Homo sapiens breast or pancreatic carcinoma cells were plated in each well of 96-well plates for growth of overnight prior to the assay. The cells were then incubated with serially diluted concentrations of humanized MC 48 or human IgG1 isotype control in RPMI in the presence of rabbit serum as a source of complement (dilution of 1:5; Life Technologies).Cell death is assessed by the addition of the viability probe 7-AAD. Based on the results of the 7-AAD measurement, percentage-specific lysis is calculated using a FACScan flow cytometer.The antibodies show significant killing activity at 10µg/mL compared to isotype control. As shown, humanized MC48-4 successfully mediates CDC of SSEA-4 expressing cells.

### Example 17

### Materials and Methods

### Construction of exemplary single chain fragments variable (scFv) of MC41, 1^{st}-hMC41, 2^{nd}-hMC41 and 3^{rd}-hMC41 phage clones

The scFv form of MC41, 1^{st}-hMC41, 2^{nd}-hMC41 and 3^{rd}-hMC41 sequences (VH-GGGGSGGGGSGGGGS-V_{L}) were gene synthesized (Genomics) and cut by Sfi I and Not I (Fermentas). After gel extraction, the digested products were cloned to pCANTAB-5E phagemid (GE Healthcare). hMC41 variant phagemids were transformed to TG1 *E-coli* and recovered in 2 × YT medium (BD Pharmingen) containing 100 µg/ml ampicillin and 2% glucose and rescued by M13KO7 helper phage (NEB) for 1 hour at 37°C. After centrifugation by 1,500 × g for 10 min, these pellets were resuspended in 2 × YT medium containing 100 µg/ml ampicillin and 50 µg/ml kanamycin overnight to generate scFv-phages.

### Demonstration of efficacy: Binding assay of MC41 and hMC41 scFv phage clones or IgGs by ELISA

SSEA-4-BSA was coated on an ELISA plate at the concentration of 0.2 µg/ml. After washing and blocking, the serial diluted phages or IgGs were incubated at RT for 1.5 hour. After washing, 1:1000 diluted HRP-conjugated anti-M13 antibody (GE Healthcare), 1:2000 diluted HRP-conjugated anti-human or -mouse IgG antibodies were added at RT for 1 hour. Then, liquid substrate 3,3',5,5'-tetramethylbenzidine (TMB) developed and was terminated with 3N HCl. Optical density was measured at 450 nm.

### Demonstration of efficacy: Humanization of MC41

The two human genes, IGHJ4*08 and IGKV6-21*02, were the most similar to MC41 V_{H} and V_{L}. As such, we chose FRs from these two genes for humanization of MC41. CDR1 to CDR3 of V_{H} and V_{L} in all of the humanized MC41 were conserved.

### Demonstration of efficacy: Construction and expression of anti-SSEA-4 humanized IgG

The V_{H} region of humanized MC41 was cloned with AgeI and NheI site into modified expression vector pcDNA5-FRT-Gammal containing a signal peptide and the constant region of human immunoglobulin gamma 1 heavy chain. The V_{L} region of humanized MC41 was cloned with AgeI and EcoRV site into modified expression vector p-Kappa-HuGs containing a signal peptide and constant region of human immunoglobulin kappa light chain. Both plasmids were transfected into FreeStyle293 cells (Invitrogen) and continuously incubated in serum-free medium at 37°C for 1 week to produce humanized antibody.

### Demonstration of efficacy: Purification of anti-SSEA-4 humanized IgG

The culture medium was collected, centrifuged and filtrated with 0.45 µm pore-size membrane. The supernatant then was subjected to protein G column chromatography (GE healthcare) for purification of anti-SSEA-4 humanized IgG. After dialysis of eluents with PBS, the antibody was examined by SDS-PAGE analysis with coomassie blue staining as usual. The concentration of antibody was assessed by Bradford reagent (Thermo Scientific) and spectrophotometer.

### Demonstration of efficacy: Binding specificity of chMC41 and hMC41 by glycan array

Glycan array slides were blocked by 1% BSA for 45 min and then incubated with serially diluted chMC41 or hMC41 IgGs for another 45 mins at RT. After washing, donkey anti-human IgG Fcγ-F674 was used as second antibody for 40 min at RT. Finally, the slides were washed, dried and subsequently scanned with wavelength 674 nm.

### Demonstration of efficacy: Antibody-dependent cell mediated cytotoxicity (ADCC) assay

HPAC (5 × 10³ cells) pancreatic cancer cell were seeded in a 96-well plate and cultured until ∼80% confluent. These cells were then incubated with antibodies chMC41, hMC41, MC813, NHIgG or NMIgG, together with PBMCs (effectors, E) at 37°C for 16 hours. After treatment, the LDH expression level was detected by CytoTox-ONE™ Homogeneous Membrane Integrity Assay Kit (Promega). The reaction was read by fluorescence with an excitation wavelength of 560 nm and emission wavelength of 590 nm (Molecular Device, SpectraMax M5).

### Demonstration of efficacy: Complement-dependent cytotoxicity (CDC) assay

HPAC (5 × 10³ cells) pancreatic cancer cell lines were cultured overnight to -80% confluent and reacted with mixture containing antibodies chMC41, hMC41, MC813, NHIgG or NMIgG and rabbit complement (20%) (Low-Tox-M rabbit complement, Cedarlane) at 37°C for 16 hours. Then, cell viability was measured by CytoTox-ONE™ Homogeneous Membrane Integrity Assay Kit (Promega), following the same procedures as that of ADCC assay.

### Demonstration of efficacy:Development of humanized MC41 mAbs

Murine mAbs have limited clinical use, including their short serum half-life, inability to trigger human effector functions and the production of human anti-murine antibodies (HAMA) response (LoBuglio et al., 1989). Therefore, mAbs have to humanize by grafting their CDRs onto the V_{H} and V_{L} FRs of human Ig molecules (Roguska et al., 1994).

After alignment of V_{H} and V_{L} variable region of MC41 with the NCBI IgBLAST or IMGT database, we generated 1^{st}, 2^{nd} and 3^{rd} humanized MC41 sequences. We next constructed and generated the phage-displayed scFv formats according to these humanized MC41 sequences. To determine the binding activity of the humanized MC41 phage clones, we carried out solid-based ELISA coating SSEA-4-BSA (Figs. 1). We found 2^{nd} and 3^{rd} humanized MC41 scFv phages could recognize SSEA-4 in a dose-dependent manner, whereas the 1^{st} MC41 scFv lost the binding activity to SSEA-4 (Fig. 1). To evaluate the binding activity by intact humanized MC41 IgG, we constructed intact IgGs of 1^{st}, 2^{nd}, 3^{rd} humanized MC41, and chimeric MC41 (chMC41). The ELISA results showed that the humanized 2^{nd} and 3^{rd} MC41 could react to SSEA-4 (Fig. 2A) but not to BSA (Fig. 2B) in a dose-dependent pattern, same results were observed for chMC41. The binding affinity of the 2^{nd} and 3^{rd} humanized MC41 was maintained, compared to that of the murine MC41. We named humanized 2^{nd} IgG as hMC41. In order to determine the binding specificity of chMC41 and hMC41, glycan array was performed. The chimeric and humanized MC41 showed more specific binding than commercial SSEA4 antibody (MC813). They only recognized SSEA4 or glycolyl modified SSEA4 (Fig. 3).

**Demonstration of efficacy: ADCC and CDC of chMC41 and hMC41.**

To demonstrate the effector function of chMC41 and hMC41, ADCC and CDC assays were performed. HPAC pancreatic cancer cell line was used to evaluate the ADCC and CDC activities of chMC41, hMC41, positive control MC813 or negative controls NHIgG and NMIgG (Figs. 4 and 5). The data showed that the effector function of hMC41 was similar to chMC41. Interestingly, the humanized MC41 not only maintain its original activity, but it also showed stronger cancer cell killing activity than MC813 through ADCC and CDC (Fig. 5).

### References

LoBuglio, A.F., Wheeler, R.H., Trang, J., Haynes, A., Rogers, K., Harvey, E.B., Sun, L., Ghrayeb, J., and Khazaeli, M.B. (1989). Mouse/human chimeric monoclonal antibody in man: kinetics and immune response. Proc Natl Acad Sci U S A 86, 4220-4224.
Roguska, M.A., Pedersen, J.T., Keddy, C.A., Henry, A.H., Searle, S.J., Lambert, J.M., Goldmacher, V.S., Blattler, W.A., Rees, A.R., and Guild, B.C. (1994). Humanization of murine monoclonal antibodies through variable domain resurfacing. Proc Natl Acad Sci U S A 91, 969-973.

### Example 18

### Demonstration of efficacy: Materials and Methods

### Phage display biopanning procedures

The phage-displayed human naive scFv library containing 2.5×10¹⁰ clones (Lu et al., 2011) was subtracted with non-specific binding in PEG-conjugated carboxyl Dynabeads (Invitrogen) at room temperature (RT) for 1 hour, and subsequently incubated with SSEA-4-PEG immobilized Dynabeads at 4°C for 1 hour. After washing with PBS or PBS containing 0.01% Tween 20 (PBST_{0.01}), the phages that bound to SSEA-4-PEG-Dynabeads were recovered by infection with *E-coli* TG1 cells at 37°C for 0.5 hour. Some of the infected cells were serially diluted to determined titer, and the others were rescued by M13KO7 phage and amplified. After determination of rescued phages titer, the next round of biopanning was performed. In the fourth and fifth round of biopanning, the phage clones were randomly selected to culture for ELISA screening.

### ELISA screening of selected phage clones

For detection of antigen recognition, microwell plates (Nunc) were coated with 0.2 µg/ml of SSEA-4-BSA, Globo H-BSA, SSEA-3-BSA and BSA, respectively. The selected phage clones were diluted 1:2 in PBS containing 3% BSA and added to each well. The plates were incubated at RT for 1 hour, washed with PBST_{0.1}, and incubated with horseradish peroxidase (HRP)-conjugated mouse anti-M13 phage antibody (GE Healthcare). The plates were washed again, and OPD and H₂O₂ were added. After termination of reaction by 3 N HCl, the absorbance was measured using a 490 nm using microplate reader (Model 680, BioRad). We extracted phagemids from ELISA-positive phage clones to identity scFv coding regions by auto-sequencing.

### Demonstration of efficacy: Construction and expression of anti-SSEA-4 human IgG

The V_{H} region of selected scFv was cloned with AgeI and NheI site into modified expression vector pcDNA5-FRT-Gamma1 containing a signal peptide and the constant region of human immunoglobulin gamma 1 heavy chain. The V_{L} region of selected scFv was cloned with AgeI and EcoRV site into modified expression vector p-Kappa-HuGs containing a signal peptide and constant region of human immunoglobulin kappa light chain. Both plasmids were transfected into FreeStyle293 cells (Invitrogen) and continuously incubated in serum-free medium at 37°C for 1 week to produce human antibody.

### Demonstration of efficacy: Purification of anti-SSEA-4 human IgG

The culture medium was collected, centrifuged and filtrated with 0.45 µm pore-size membrane. The supernatant then was subjected to protein G column chromatography (GE healthcare) for purification of anti-SSEA-4 human IgG. After dialysis of eluents with PBS, the antibody was examined by SDS-PAGE analysis with coomassie blue staining as usual. The concentration of antibody was assessed by Bradford reagent (Thermo Scientific) and spectrophotometer.

### Demonstration of efficacy: Humanization of MC48 and MC41

Two human genes, GenBank accession Q9UL73 and AY577298, were the most similar to MC48 V_{H} and V_{L}, respectively. We humanized three sequences of MC48, including the 1^{st} humanized MC48 (hMC48) V_{H} consisted of modified framework (FR) 1 to FR4 of Q9UL73 gene, the 1^{st} hMC48 V_{L} consisted of four FRs from the accession AY577298, the 2^{nd} hMC48 FRs of V_{H} followed by 1YY8 from PDB, while the 2^{nd} hMC48 V_{L} same as 1^{st} sequence, and the 3^{rd} hMC48 V_{H} sequence modified FR1, 2 and 4 of Q9UL73 gene and the 3^{rd} hMC48 V_{L} only changed FR2 and FR4 to human AY577298 gene. The other two human genes, IGHJ4*08 and IGKV6-21*02, were the most similar to MC41 V_{H} and V_{L}. As such, we chose FRs from these two genes for humanization of MC41. CDR1 to CDR3 of V_{H} and V_{L} in all of the humanized MC48 and MC41 were conserved.

### Demonstration of efficacy: Construction of single chain fragments variable (scFv) of humanized MC48 and MC41 phage clones

The scFv form of humanized MC48 (hMC48) and MC41 (hMC41) sequences (V_{H}-GGGGSGGGGSGGGGS-V_{L}) were gene synthesized (Genomics) and cut by Sfi I and Not I (Fermentas). After gel extraction, the digested products were cloned to pCANTAB-5E phagemid (GE Healthcare). hMC48 and hMC41 variant phagemids were transformed to TG1 *E-coli* and recovered in 2 × YT medium (BD Pharmingen) containing 100 µg/ml ampicillin and 2% glucose and rescued by M13KO7 helper phage (NEB) for 1 hour at 37°C. After centrifugation by 1,500 × g for 10 min, these pellets were resuspended in 2 × YT medium containing 100 µg/ml ampicillin and 50 µg/ml kanamycin overnight to generate scFv-phages.

### Demonstration of efficacy: Binding assay of hMC48 and hMC41 scFv phage clones or IgGs by ELISA

SSEA-4-BSA was coated on an ELISA plate at the concentration of 0.2 µg/ml. After washing and blocking, the serial diluted phages or IgGs were incubated at RT for 1.5 hour. After washing, 1:1000 diluted HRP-conjugated anti-M13 antibody (GE Healthcare), 1:2000 diluted HRP-conjugated anti-human or -mouse IgG antibodies were added at RT for 1 hour. Then, liquid substrate 3,3',5,5'-tetramethylbenzidine (TMB) developed and was terminated with 3N HCl. Optical density was measured at 450 nm.

### Demonstration of efficacy: Binding specificity of p2-78 hAb, chMC41 and hMC41 by glycan array

Glycan array slides were blocked by 1% BSA for 45 min and then incubated with serially diluted p2-78 hAb, chMC41 or hMC41 IgGs for another 45 mins at RT. After washing, donkey anti-human IgG Fcγ-F674 was second antibody for 40 min at RT. Finally, the slides were washed, dried and subsequently scanned with wavelength 674 nm.

### Demonstration of efficacy: Antibody-dependent cell mediated cytotoxicity (ADCC) assay

HPAC, BxPC3 or PL45 (5 × 10³ cells) pancreatic cancer cell were seeded in a 96-well plate and cultured until ∼80% confluent. Then, these cells were incubated with antibodies hMC48, hMC41 or NHIgG, together with PBMCs (effectors, E) at 37°C for 16 hours. After treatment, the LDH expression level was detected by CytoTox-ONE™ Homogeneous Membrane Integrity Assay Kit (Promega). The reaction was read by fluorescence with an excitation wavelength of 560 nm and emission wavelength of 590 nm (Molecular Device, SpectraMax M5).

### Demonstration of efficacy: Complement-dependent cytotoxicity (CDC) assay

HPAC, BxPC3 or PL45 (5 × 10³ cells) pancreatic cancer cell lines were cultured overnight to ∼80% confluent and reacted with mixture containing antibodies hMC48, hMC41 or NHIgG and rabbit complement (10% and 20%) (Low-Tox-M rabbit complement, Cedarlane) at 37°C for 16 hours. Then, cell viability was measured by CytoTox-ONE™ Homogeneous Membrane Integrity Assay Kit (Promega), following the same procedures as that of ADCC assay.

### Demonstration of efficacy:

### Identification of phage-displayed scFv that binds to SSEA-4

To identify the antibodies that bind to SSEA-4, we used phage-displayed human naive scFv library containing 2×10¹⁰ members, which was established as described in our previous report (Lu et al., 2011). This library was first removed by Dynabeads-binding phages, and then SSEA-4-binding phages were selected by SSEA-4-PEG-conjugated Dynabeads. We used two buffer systems, PBS and PBS containing 0.01% Tween20 (PBST_{0.01}), during biopanning. After five rounds of affinity selection, the phage recovery of the fifth round increased by about 55-fold and 80-fold, compared to that of the first round in PBS and PBST_{0.01} system, respectively (Fig. 1). The phage clones were randomly selected and tested for SSEA-4 binding by ELISA (Fig. 2). We found seven clones that specifically bound to SSEA-4-BSA, but not to BSA control protein. By sequencing all 8 individual clones, we identified two unique anti-SSEA-4 phage clones (p1-52 and p2-78) which contained distinct human V_{H} and V_{L} coding regions (Table 1).

To examine the specificity and binding affinity of the two phage clones, we performed a comparative ELISA using the same phage titer to Globo-series glycans including SSEA-4-BSA, Globo H-BSA and SSEA-3-BSA (Fig. 3). The p2-78 phage clone showed the strong binding to SSEA-4-BSA and SSEA-3-BSA, and more slight binding to Globo H-BSA. However, we found that the binding activity of p1-52 phage clone to SSEA-4-BSA was very weak. Thus we focused on p2-78 clone for further study.

To establish the fully human antibody (hAb) against SSEA-4, we molecularly engineered the V_{H} and V_{L} coding sequences of p2-78 scFv into human IgG₁ backbone, respectively. The anti-SSEA-4 p2-78 hAb was produced using FreeStyle 293 expression system and then purified through the protein G sepharose column. We examined the purity of antibody by SDS-PAGE analysis with coomassie blue staining (Fig. 4A). The result shows the purity level of antibody exceed 95%. Subsequently, we performed ELISA to investigate the binding activity of p2-78 hAb for Globo-series glycans (Fig. 4B). We found that p2-78 hAb bound to SSEA-4 and SSEA-3, but not to Globo H, demonstrating that the human IgG version of p2-78 retains the activity of its parental scFv version to recognize the binding epitope of SSEA-4.

We used glycan array containing 203 different glycans to further confirm the specificity of p2-78 hAb. The results showed that p2-78 hAb recognized SSEA4, Sialyl-SSEA4, SSEA4Gc, and Gb5 (SSEA3) (Fig. 5B). Interestingly, p2-78 hAb also slightly recognized Globo H, similar to the results from ELISA assay (Fig. 3). The commercially available IgM antibody, MC631, was used as a positive control (Fig. 5A).

### Demonstration of efficacy: Development of humanized MC48 and MC41 mAbs

Murine mAbs have limited clinical use, including their short serum half-life, inability to trigger human effector functions and the production of human anti-murine antibodies (HAMA) response (LoBuglio et al., 1989). Therefore, mAbs have to humanize by grafting their CDRs onto the V_{H} and V_{L} FRs of human Ig molecules (Roguska et al., 1994).

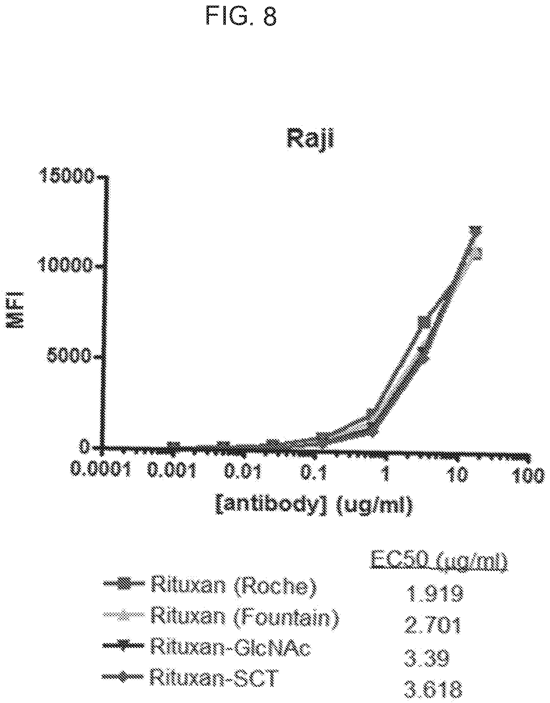

After alignment of V_{H} and V_{L} variable region of MC48 and MC41 with the NCBI IgBLAST or IMGT database, we generated 1^{st}, 2^{nd}, 3^{rd} and 4^{th} humanized MC48 sequences and 1^{st}, 2^{nd} and 3^{rd} humanized MC41 sequences. We next constructed and generated the phage-displayed scFv formats according to these humanized MC48 and MC41 sequences. To determine the binding activity of the humanized MC48 and MC41 phage clones, we carried out solid-based ELISA coating SSEA-4-BSA (Figs. 6, 7 and 8). We found that the 3^{rd} and 4^{th} humanized MC48, and 2^{nd} and 3^{rd} humanized MC41 scFv phages could recognize SSEA-4 in a dose-dependent manner, whereas the 1^{st} and 2^{nd} humanized MC48 and 1^{st} MC41 scFv lost the binding activity to SSEA-4 (Figs. 6, 7 and 8). The data showed that the binding affinities of the 4^{th} humanized MC48, and 3^{rd} humanized MC41 scFv phage clones were maintained, compared to that of the murine mAbs MC48 or MC41. To evaluate the binding activity by intact humanized MC41 IgG, we constructed intact IgGs of 1^{st}, 2^{nd}, 3^{rd} humanized MC41 and chimeric MC41 (chMC41). The ELISA results showed that the humanized 2^{nd} and 3^{rd} MC41 could react to SSEA-4 (Fig. 9A) but not to BSA (Fig. 9B) in a dose-dependent pattern, same results were observed for chMC41. We named humanized 2^{nd} IgG as hMC41. In order to determine the binding specificity of chMC41 and hMC41, glycan array was performed. The chimeric and humanized MC41 showed more specific binding than commercial SSEA4 antibody (MC813). They only recognized SSEA4 or glycolyl modified SSEA4 (Fig. 10).

### Demonstration of efficacy: ADCC and CDC test of hMC48, chMC41 and hMC41

To investigate the effector function of hMC48, chMC41 and hMC41, ADCC and CDC assays were performed. HPAC, BxPC3 and PL45 pancreatic cancer cell lines were used to evaluate the ADCC and CDC activities at the concentration of 10 µg/ml for hMC48 or NHIgG (Fig. 11). Further, HPAC cells were treated with chMC41, hMC41, positive control MC813 or negative control NHIgG (Figs. 12 and 13). The data showed that the effector function of hMC41 and chMC41 was superior to that of hMC48. Interestingly, the humanized MC41 not only maintain its original activity, but it also showed stronger cancer cell killing activity than MC813 through ADCC and CDC (Fig. 13).

### Example 19

### binding of MC41 vs MC 48

The binding abilities of hMC41 and hMC48 to SSEA-4 were examined by ELISA. The result showed that the binding of hMC41 to SSEA-4 was much better than hMC48. The humanized MC41 has a higher binding maximum and a smaller Kd (0.2 µg/ml and 4.6 µg/ml for hMC41 and hMC48, respectively) value as compared to hMC48.

### References

LoBuglio, A.F., Wheeler, R.H., Trang, J., Haynes, A., Rogers, K., Harvey, E.B., Sun, L., Ghrayeb, J., and Khazaeli, M.B. (1989). Mouse/human chimeric monoclonal antibody in man: kinetics and immune response. Proc Natl Acad Sci U S A 86, 4220-4224.
Lu, R.-M., Chang, Y.-L., Chen, M.-S., and Wu, H.-C. (2011). Single chain anti-c-Met antibody conjugated nanoparticles for in vivo tumor-targeted imaging and drug delivery. Biomaterials 32, 3265-3274.
Roguska, M.A., Pedersen, J.T., Keddy, C.A., Henry, A.H., Searle, S.J., Lambert, J.M., Goldmacher, V.S., Blattler, W.A., Rees, A.R., and Guild, B.C. (1994). Humanization of murine monoclonal antibodies through variable domain resurfacing. Proc Natl Acad Sci U S A 91, 969-973.

## Claims

1. An isolated monoclonal antibody or a binding fragment thereof that binds to Neu5Acα2→ 3Galβ1→ 3GalNAcβ1→ 3Galα1→ 4Galβ1→ 4Glcβ1 (SSEA-4 hexasaccharide) wherein the isolated monoclonal antibody or the binding fragment thereof is a defucosylated antibody comprising a Fc glycoform attached to the Fc region, wherein said glycoform has the formula:
Sia₂(α2-6)Gal₂GlcNAc₂Man₃GlcNAc₂ 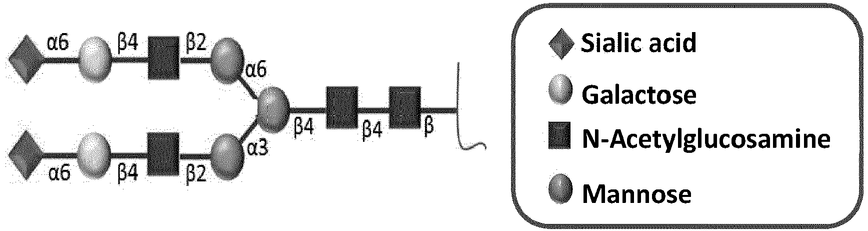
wherein the antibody comprises a VH having SEQ ID NO: 147 and a VL having SEQ ID NO: 148.

2. The isolated monoclonal antibody or a binding fragment thereof of claim 1, wherein the antibody is an IgG1.

3. An isolated monoclonal antibody or a binding fragment thereof that binds to Neu5Acα2→ 3Galβ1→ 3GalNAcβ1→ 3Galα1→ 4Galβ1→ 4Glcβ1 (SSEA-4 hexasaccharide), wherein the isolated monoclonal antibody or the binding fragment thereof is a defucosylated antibody comprising a Fc glycoform attached to the Fc region, wherein said glycoform has the formula:
Sia₂(α2-6)Gal₂GlcNAc₂Man₃GlcNAc₂ 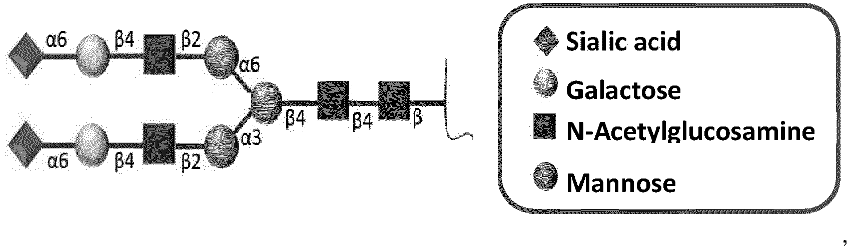
wherein the isolated monoclonal antibody or the binding fragment thereof comprises an H-CDR1, H-CDR2, H-CDR3 L-CDR1, L-CDR2, and L-CDR3, wherein:
(i) the H-CDR1 has the SEQ ID NO:207;
(ii) the H-CDR2 has the SEQ ID NO: 208;
(iii) the H-CDR3 thas the SEQ ID NO: 209;
(iv) the L-CDR1 has the SEQ ID NO: 204;
(v) the L-CDR2 has the SEQ ID NO:205;
(vi) the L-CDR3 thas the SEQ ID NO: 206.

4. The isolated monoclonal antibody or a binding fragment thereof of claim 3, wherein the isolated monoclonal antibody or the binding fragment thereof comprises a VH having SEQ ID NO: 202 and a VL having SEQ ID NO: 203.

5. An isolated monoclonal antibody or a binding fragment thereof that binds to Neu5Acα2→ 3Galβ1→ 3GalNAcβ1→ 3Galα1→ 4Galβ1→ 4Glcβ1 (SSEA-4 hexasaccharide), wherein the isolated monoclonal antibody or the binding fragment thereof is a defucosylated antibody comprising a Fc glycoform attached to the Fc region, wherein said glycoform has the formula:
Sia₂(α2-6)Gal₂GlcNAc₂Man₃GlcNAc₂ 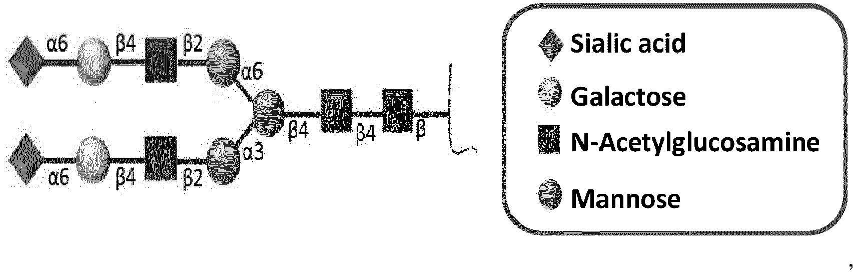
wherein the antibody or the fragment thereof comprises an H-CDR1, H-CDR2, H-CDR3, L-CDR1, L-CDR2, and L-CDR3, wherein:
(i) the H-CDR1 has the sequence of SEQ ID NO: 217;
(ii) the H-CDR2 has the sequence of SEQ ID NO: 218;
(iii) the H-CDR3 has the sequence of SEQ ID NO: 219;
(iv) the L-CDR1 has the sequence of SEQ ID NO: 214;
(v) the L-CDR2 has the sequence of SEQ ID NO: 215; and
(vi) the L-CDR3 has the sequence of SEQ ID NO: 216.

6. The isolated monoclonal antibody or a binding fragment thereof of claim 5, wherein the isolated monoclonal antibody or the binding fragment thereof comprises a VH having SEQ ID NO: 212 and a VL having SEQ ID NO: 213.

7. An isolated monoclonal antibody or a binding fragment thereof that binds to Neu5Acα2→ 3Galβ1→ 3GalNAcβ1→ 3Galα1→ 4Galβ1→ 4Glcβ1 (SSEA-4 hexasaccharide), wherein the isolated monoclonal antibody or the binding fragment thereof is a defucosylated antibody comprising a Fc glycoform attached to the Fc region, wherein said glycoform has the formula: 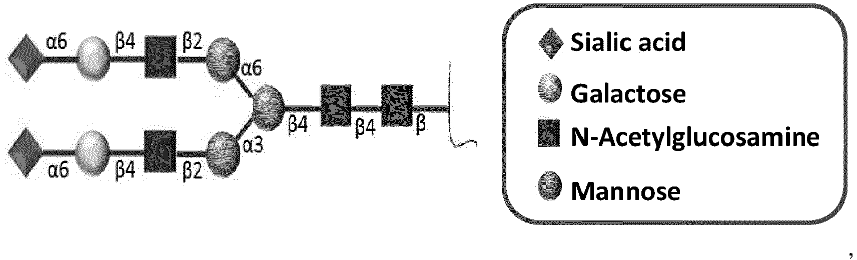
Sia₂(α2-6)Gal₂GlcNAc₂Man₃GlcNAc₂
wherein the isolated monoclonal antibody or the binding fragment thereof comprises an H-CDR1, H-CDR2, H-CDR3, L-CDR1, L-CDR2, and L-CDR3, wherein:
(i) the H-CDR1 has the sequence of SEQ ID NO: 227;
(ii) the H-CDR2 has the sequence of SEQ ID NO: 228;
(iii) the H-CDR3 has the sequence of SEQ ID NO: 229;
(iv) the L-CDR1 has the sequence of SEQ ID NO: 224;
(v) the L-CDR2 has the sequence of SEQ ID NO: 225; and
(vi) the L-CDR3 has the sequence of SEQ ID NO: 226.

8. The isolated monoclonal antibody or a binding fragment thereof of claim 7, wherein the isolated monoclonal antibody or the binding fragment thereof comprises a VH having SEQ ID NO: 222 and a VL having SEQ ID NO: 223.

9. The isolated monoclonal antibody or a binding fragment thereof of any one of claims 1 to 8, wherein the Fc glycoform is attached to Asn-297 of the Fc region.

10. The isolated monoclonal antibody or a binding fragment thereof of any of claims 1 to 9, wherein the antibody is a humanized antibody.

11. A pharmaceutical composition comprising the isolated monoclonal antibody or a binding fragment thereof of any one of claims 1 to 10 and a pharmaceutically acceptable carrier.

12. The pharmaceutical composition of claim 11, wherein the composition is for use in the treatment against a hyperproliferative disease.

13. An isolated monoclonal antibody or a binding fragment thereof of any one of claims 1-10 for use in a method of treating cancer in a subject in need thereof, wherein the isolated monoclonal antibody or a binding fragment thereof enhances ADCC activity in said subject.

14. The isolated monoclonal antibody or a binding fragment thereof for use according to claim 13, wherein the cancer is selected from the group consisting of brain cancer, lung cancer, breast cancer, oral cancer, esophageal cancer, stomach cancer, liver cancer, bile duct cancer, pancreatic cancer, colon cancer, kidney cancer, bone cancer, skin cancer, cervical cancer, ovarian cancer, and prostate cancer.

15. The isolated monoclonal antibody or a binding fragment thereof for use according to claim 14, wherein the monoclonal antibody or the binding fragment thereof is administered with at least one other chemotherapeutic agent.

16. A method for making the isolated monoclonal antibody or the binding fragment thereof of any one of claims 1 to 10, comprising:
(a) contacting an isolated monoclonal antibody or a binding fragment thereof with an α-fucosidase and at least one endoglycosidase, wherein the isolated monoclonal antibody or the binding fragment thereof comprises VH having SEQ ID NO: 147 and VL having SEQ ID NO: 148 as defined in claim 1, or H-CDR1, H-CDR2, H-CDR3, L-CDR1, L-CDR2, and L-CDR3 as defined in any one of claims 3, 5 and 7;
(b) generating a defucosylated antibody having a single N-acetylglucosamine (GlcNAc); and (c) adding a glycan to GlcNAc of Fc region of antibody to form the isolated monoclonal antibody or the binding fragment thereof with said glycoform as defined in any one of claims 1, 3, 5, and 7.

17. The isolated monoclonal antibody or a binding fragment thereof of any one of claims 1 to 10, wherein the isolated monoclonal antibody is a multispecific antibody that further binds to one or more of the antigens selected from the group consisting of Globo H and SSEA-3.

## Patentansprüche

1. Isolierter monoklonaler Antikörper oder bindendes Fragment davon, der/das an Neu5Acα2→ 3Galβ1→ 3GalNAcβ1→ 3Galα1→ 4Galβ1→ 4G1cβ1 (SSEA-4-Hexasaccharid) bindet, wobei der isolierte monoklonale Antikörper oder das Bindungsfragment davon ein defucosylierter Antikörper ist, umfassend eine Fc-Glykoform, die an die Fc-Region angelagert ist, wobei die Glykoform die folgende Formel aufweist:
Sia₂(α2-6)Gal₂GlcNAc₂Man₃GlcNAc₂ 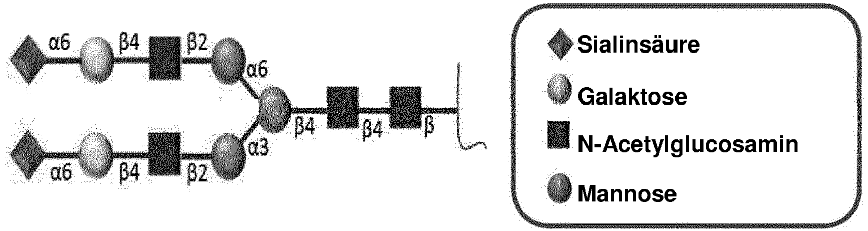
wobei der Antikörper eine VH, die SEQ ID NO: 147 aufweist, und eine VL, die SEQ ID NO: 148 aufweist, umfasst.

2. Isolierter monoklonaler Antikörper oder bindendes Fragment davon nach Anspruch 1, wobei der Antikörper ein IgG1 ist.

3. Isolierter monoklonaler Antikörper oder bindendes Fragment davon, der/das an Neu5Acα2→ 3Galβ1→ 3GalNAcβ1→ 3Galα1→ 4Galβ1→ 4G1cβ1 (SSEA-4-Hexasaccharid) bindet, wobei der isolierte monoklonale Antikörper oder das Bindungsfragment davon ein defucosylierter Antikörper ist, umfassend eine Fc-Glykoform, die an die Fc-Region angelagert ist, wobei die Glykoform die folgende Formel aufweist:
Sia₂(α2-6)Gal₂GlcNAc₂Man₃GlcNAc₂ 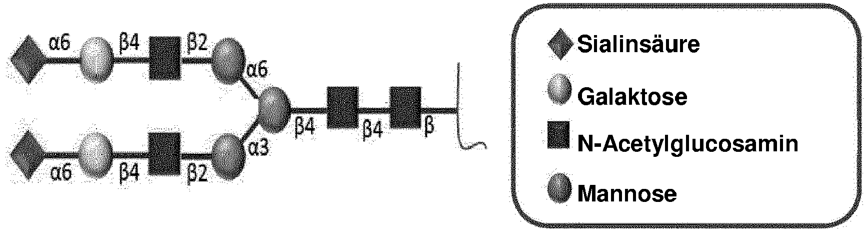
wobei der isolierte monoklonale Antikörper oder das bindende Fragment davon eine H-CDR1, H-CDR2, H-CDR3, L-CDR1, L-CDR2 und L-CDR3 umfasst, wobei:
(i) die H-CDR1 die SEQ ID NO: 207 aufweist;
(ii) die H-CDR2 die SEQ ID NO: 208 aufweist;
(iii) die H-CDR3 die SEQ ID NO: 209 aufweist;
(iv) die L-CDR1 die SEQ ID NO: 204 aufweist;
(v) die L-CDR2 die SEQ ID NO: 205 aufweist;
(vi) die L-CDR3 die SEQ ID NO: 206 aufweist.

4. Isolierter monoklonaler Antikörper oder bindendes Fragment davon nach Anspruch 3, wobei der isolierte monoklonale Antikörper oder das bindende Fragment davon eine VH, die SEQ ID NO: 202 aufweist, und eine VL, die SEQ ID NO: 203 aufweist, umfasst.

5. Isolierter monoklonaler Antikörper oder bindendes Fragment davon, der/das an Neu5Acα2→ 3Galβ1→ 3GalNAcβ1→ 3Galα1→ 4Ga1β1→ 4G1cβ1 (SSEA-4-Hexasaccharid) bindet, wobei der isolierte monoklonale Antikörper oder das Bindungsfragment davon ein defucosylierter Antikörper ist, umfassend eine Fc-Glykoform, die an die Fc-Region angelagert ist, wobei die Glykoform die folgende Formel aufweist:
Sia₂(α2-6)Gal₂GlcNAc₂Man₃GlcNAc₂ 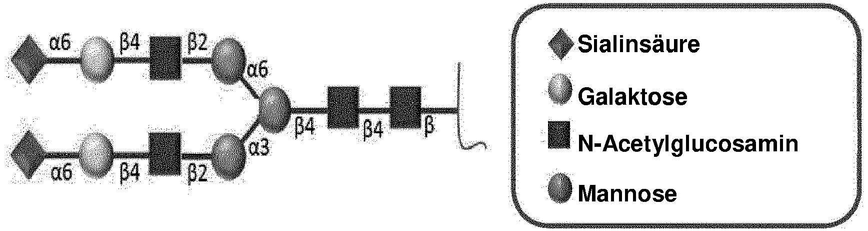
wobei der Antikörper oder das Fragment davon eine H-CDR1, H-CDR2, H- CDR3, L-CDR1, L-CDR2 und L-CDR3 umfasst, wobei:
(i) die H-CDR1 die SEQ ID NO: 217 aufweist;
(ii) die H-CDR2 die SEQ ID NO: 218 aufweist;
(iii) die H-CDR3 die SEQ ID NO: 219 aufweist;
(iv) die L-CDR1 die SEQ ID NO: 214 aufweist;
(v) die L-CDR2 die SEQ ID NO: 215 aufweist;
(vi) die L-CDR3 die SEQ ID NO: 216 aufweist.

6. Isolierter monoklonaler Antikörper oder bindendes Fragment davon nach Anspruch 5, wobei der isolierte monoklonale Antikörper oder das bindende Fragment davon eine VH, die SEQ ID NO: 212 aufweist, und eine VL, die SEQ ID NO: 213 aufweist, umfasst.

7. Isolierter monoklonaler Antikörper oder bindendes Fragment davon, der/das an Neu5Acα2→ 3Galβ1→ 3GalNAcβ1→ 3Galα1→ 4Galβ1→ 4G1cβ1 (SSEA-4-Hexasaccharid) bindet, wobei der isolierte monoklonale Antikörper oder das Bindungsfragment davon ein defucosylierter Antikörper ist, umfassend eine Fc-Glykoform, die an die Fc-Region angelagert ist, wobei die Glykoform die folgende Formel aufweist: 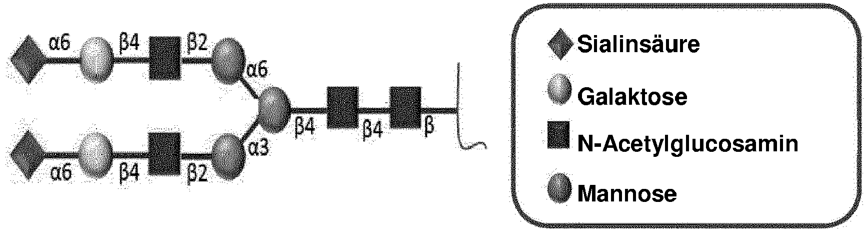
Sia₂(α2-6)Gal₂GlcNAc₂Man₃GlcNAc₂
wobei der isolierte monoklonale Antikörper oder das bindende Fragment davon eine H-CDR1, H-CDR2, H-CDR3, L-CDR1, L-CDR2 und L-CDR3 umfasst, wobei:
(i) die H-CDR1 die SEQ ID NO: 227 aufweist;
(ii) die H-CDR2 die SEQ ID NO: 228 aufweist;
(iii) die H-CDR3 die SEQ ID NO: 229 aufweist;
(iv) die L-CDR1 die SEQ ID NO: 224 aufweist;
(v) die L-CDR2 die SEQ ID NO: 225 aufweist;
(vi) die L-CDR3 die SEQ ID NO: 226 aufweist.

8. Isolierter monoklonaler Antikörper oder bindendes Fragment davon nach Anspruch 7, wobei der isolierte monoklonale Antikörper oder das bindende Fragment davon eine VH, die SEQ ID NO: 222 aufweist, und eine VL, die SEQ ID NO: 223 aufweist, umfasst.

9. Isolierter monoklonaler Antikörper oder bindendes Fragment davon nach einem der Ansprüche 1 bis 8, wobei die Fc-Glykoform an Asn-297 der Fc-Region angelagert ist.

10. Isolierter monoklonaler Antikörper oder bindendes Fragment davon nach einem der Ansprüche 1 bis 9, wobei der Antikörper ein humanisierter Antikörper ist.

11. Pharmazeutische Zusammensetzung, umfassend den isolierten monoklonalen Antikörper oder ein bindendes Fragment davon nach einem der Ansprüche 1 bis 10 und einen pharmazeutisch annehmbaren Träger.

12. Pharmazeutische Zusammensetzung nach Anspruch 11, wobei die Zusammensetzung zur Verwendung bei der Behandlung gegen eine hyperproliferative Erkrankung bestimmt ist.

13. Isolierter monoklonaler Antikörper oder bindendes Fragment davon nach einem der Ansprüche 1 bis 10 zur Verwendung in einem Verfahren zum Behandeln von Krebs bei einem Subjekt, das dessen bedarf, wobei der isolierte monoklonale Antikörper oder ein bindendes Fragment davon eine ADCC-Aktivität bei dem Subjekt verstärkt.

14. Isolierter monoklonaler Antikörper oder bindendes Fragment davon zur Verwendung gemäß Anspruch 13, wobei der Krebs ausgewählt ist aus der Gruppe bestehend aus Gehirnkrebs, Lungenkrebs, Brustkrebs, Mundkrebs, Speiseröhrenkrebs, Magenkrebs, Leberkrebs, Gallengangskrebs, Bauchspeicheldrüsenkrebs, Dickdarmkrebs, Nierenkrebs, Knochenkrebs, Hautkrebs, Gebärmutterhalskrebs, Eierstockkrebs und Prostatakrebs.

15. Isolierter monoklonaler Antikörper oder bindendes Fragment davon zur Verwendung gemäß Anspruch 14, wobei der monoklonale Antikörper oder das bindende Fragment davon mit mindestens einem anderen chemotherapeutischen Mittel verabreicht wird.

16. Verfahren zum Herstellen des isolierten monoklonalen Antikörpers oder des bindenden Fragments davon nach einem der Ansprüche 1 bis 10, umfassend:
(a) Inkontaktbringen eines isolierten monoklonalen Antikörpers oder eines bindenden Fragments davon mit einer α-Fucosidase und mindestens einer Endoglycosidase, der isolierte monoklonale Antikörper oder das bindende Fragment davon umfassend VH, die SEQ ID NO: 147 aufweist, und VL, die SEQ ID NO: 148 aufweist, wie es in Anspruch 1 definiert ist, oder H-CDR1, H-CDR2, H-CDR3, L-CDR1, L-CDR2 und L-CDR3 wie es in einem der Ansprüche 3, 5 und 7 definiert ist;
(b) Erzeugen eines defucosylierten Antikörpers, der ein einzelnes N-Acetylglucosamin (GlcNAc) aufweist; und (c) Hinzufügen eines Glycans zum GlcNAc der Fc-Region des Antikörpers, um den isolierten monoklonalen Antikörper oder das bindende Fragment davon mit der Glykoform zu bilden, wie es in einem der Ansprüche 1, 3, 5 und 7 definiert ist.

17. Isolierter monoklonaler Antikörper oder bindendes Fragment davon nach einem der Ansprüche 1 bis 10, wobei der isolierte monoklonale Antikörper ein multispezifischer Antikörper ist, der ferner an eines oder mehrere der Antigene bindet, die ausgewählt sind aus der Gruppe, bestehend aus Globo H und SSEA-3.

## Revendications

1. Anticorps monoclonal isolé ou fragment de liaison de celui-ci qui se lie à Neu5Acα2→ 3Galβ1→ 3GalNAcβ1→- 3Galα1→ 4Galβ1→- 4G1cβ1 (SSEA-4 hexasaccharide) dans lequel l'anticorps monoclonal isolé ou fragment de liaison de celui-ci est un anticorps défucosylé comprenant une glycoforme de Fc attachée à la région Fc, dans lequel ladite glycoforme est de formule :
Sia₂(α2-6)Gal₂GlcNAc₂Man₃GlcNAc₂ 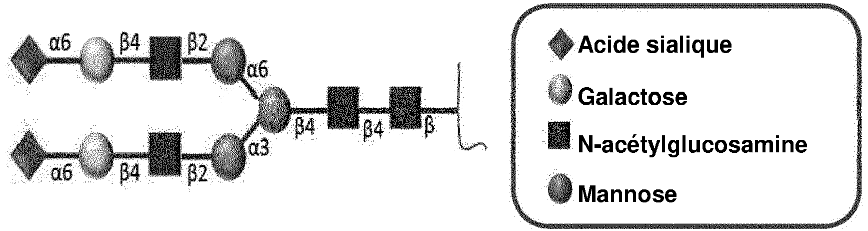
dans lequel l'anticorps comprend une VH de SEQ ID N° : 147 et une VL de SEQ ID N° : 148.

2. Anticorps monoclonal isolé ou fragment de liaison de celui-ci selon la revendication 1, dans lequel l'anticorps est une IgG1.

3. Anticorps monoclonal isolé ou fragment de liaison de celui-ci qui se lie à Neu5Acα2→ 3Galβ1→ 3GalNAcβ1→ 3Galα1→ 4Galβ1→- 4G1cβ1 (SSEA-4 hexasaccharide), dans lequel l'anticorps monoclonal isolé ou fragment de liaison de celui-ci est un anticorps défucosylé comprenant une glycoforme de Fc attachée à la région Fc, dans lequel ladite glycoforme est de formule :
Sia₂(α2-6)Gal₂GlcNAc₂Man₃GlcNAc₂ 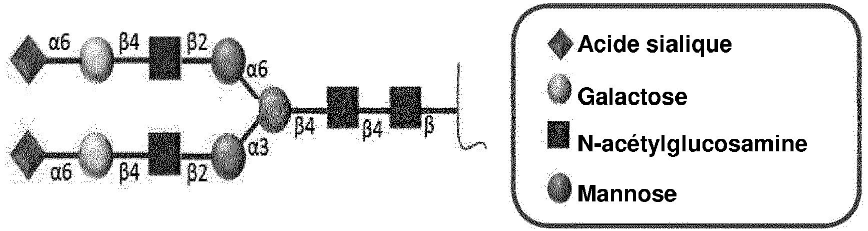
dans lequel l'anticorps monoclonal isolé ou fragment de liaison de celui-ci comprend une H-CDR1, une H-CDR2, une H-CDR3, une L-CDR1, une L-CDR2, et une L-CDR3, dans lequel :
(i) la H-CDR1 a la SEQ ID N° : 207 ;
(ii) la H-CDR2 a la SEQ ID N° : 208 ;
(iii) la H-CDR3 a la SEQ ID N° : 209 ;
(iv) la L-CDR1 a la SEQ ID N° : 204 ;
(v) la L-CDR2 a la SEQ ID N° : 205 ;
(vi) la L-CDR3 a la SEQ ID N° : 206.

4. Anticorps monoclonal isolé ou fragment de liaison de celui-ci selon la revendication 3, dans lequel l'anticorps monoclonal isolé ou fragment de liaison de celui-ci comprend une VH de SEQ ID N° : 202 et une VL de SEQ ID N° : 203.

5. Anticorps monoclonal isolé ou fragment de liaison de celui-ci qui se lie à Neu5Acα2→ 3Galβ1→ 3GalNAcβ1→ 3Galα1→ 4Galβ1→- 4G1cβ1 (SSEA-4 hexasaccharide) dans lequel l'anticorps monoclonal isolé ou fragment de liaison de celui-ci est un anticorps défucosylé comprenant une glycoforme de Fc attachée à la région Fc, dans lequel ladite glycoforme est de formule :
Sia₂(α2-6)Gal₂GlcNAc₂Man₃GlcNAc₂ 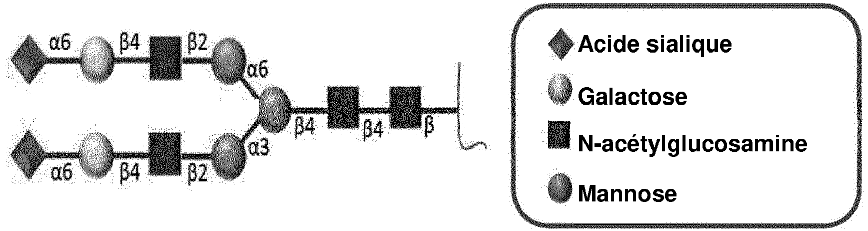
dans lequel l'anticorps ou fragment de liaison de celui-ci comprend une H-CDR1, une H-CDR2, une H-CDR3, une L-CDR1, une L-CDR2, et une L-CDR3, dans lequel :
(i) la H-CDR1 a la séquence de SEQ ID N° : 217 ;
(ii) la H-CDR2 a la séquence de SEQ ID N° : 218 ;
(iii) la H-CDR3 a la séquence de SEQ ID N° : 219 ;
(iv) la L-CDR1 a la séquence de SEQ ID N° : 214 ;
(v) la L-CDR2 a la séquence de SEQ ID N° : 215 ; et
(vi) la L-CDR3 a la séquence de SEQ ID N° : 216.

6. Anticorps monoclonal isolé ou fragment de liaison de celui-ci selon la revendication 5, dans lequel l'anticorps monoclonal isolé ou fragment de liaison de celui-ci comprend une VH de SEQ ID N° : 212 et une VL de SEQ ID N° : 213.

7. Anticorps monoclonal isolé ou fragment de liaison de celui-ci qui se lie à Neu5Acα2→ 3Galβ1→ 3GalNAcβ1→ 3Galα1→ 4Galβ1→- 4G1cβ1 (SSEA-4 hexasaccharide) dans lequel l'anticorps monoclonal isolé ou fragment de liaison de celui-ci est un anticorps défucosylé comprenant une glycoforme de Fc attachée à la région Fc, dans lequel ladite glycoforme est de formule : 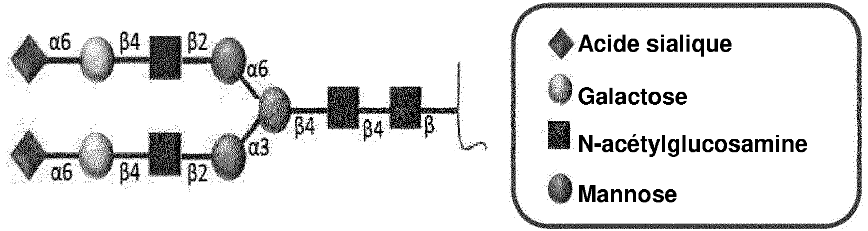
Sia₂(α2-6)Gal₂GlcNAc₂Man₃GlcNAc₂
dans lequel l'anticorps monoclonal isolé ou fragment de liaison de celui-ci comprend une H-CDR1, une H-CDR2, une H-CDR3, une L-CDR1, une L-CDR2, et une L-CDR3, dans lequel :
(i) la H-CDR1 a la séquence de SEQ ID N° : 227 ;
(ii) la H-CDR2 a la séquence de SEQ ID N° : 228 ;
(iii) la H-CDR3 a la séquence de SEQ ID N° : 229 ;
(iv) la L-CDR1 a la séquence de SEQ ID N° : 224 ;
(v) la L-CDR2 a la séquence de SEQ ID N° : 225 ; et
(vi) la L-CDR3 a la séquence de SEQ ID N° : 226.

8. Anticorps monoclonal isolé ou fragment de liaison de celui-ci selon la revendication 7, dans lequel l'anticorps monoclonal isolé ou fragment de liaison de celui-ci comprend une VH de SEQ ID N° : 222 et une VL de SEQ ID N° : 223.

9. Anticorps monoclonal isolé ou fragment de liaison de celui-ci selon l'une quelconque des revendications 1 à 8, dans lequel la glycoforme de Fc est attachée à Asn-297 de la région Fc.

10. Anticorps monoclonal isolé ou fragment de liaison de celui-ci selon l'une quelconque des revendications 1 à 9, dans lequel l'anticorps est un anticorps humanisé.

11. Composition pharmaceutique comprenant l'anticorps monoclonal isolé ou fragment de liaison de celui-ci selon l'une quelconque des revendications 1 à 10 et un support pharmaceutiquement acceptable.

12. Composition pharmaceutique selon la revendication 11, dans laquelle la composition est destinée à une utilisation dans le traitement contre une maladie hyperproliférative.

13. Anticorps monoclonal isolé ou fragment de liaison de celui-ci selon l'une quelconque des revendications 1 à 10, destiné à une utilisation dans un procédé de traitement du cancer chez un sujet qui en éprouve le besoin, dans lequel l'anticorps monoclonal isolé ou fragment de liaison de celui-ci renforce l'activité ADCC chez ledit sujet.

14. Anticorps monoclonal isolé ou fragment de liaison de celui-ci destiné à une utilisation selon la revendication 13, dans lequel le cancer est choisi dans le groupe constitué par le cancer du cerveau, le cancer du poumon, le cancer du sein, le cancer buccal, le cancer de l'œsophage, le cancer de l'estomac, le cancer du foie, le cancer des voies biliaires, le cancer du pancréas, le cancer du côlon, le cancer du rein, le cancer des os, le cancer de la peau, le cancer du col de l'utérus, le cancer de l'ovaire, et le cancer de la prostate.

15. Anticorps monoclonal isolé ou fragment de liaison de celui-ci destiné à une utilisation selon la revendication 14, dans lequel l'anticorps monoclonal ou fragment de liaison de celui-ci est administré avec au moins un autre agent chimiothérapeutique.

16. Procédé de fabrication de l'anticorps monoclonal isolé ou fragment de liaison de celui-ci selon l'une quelconque des revendications 1 à 10, comprenant :
(a) la mise en contact d'un anticorps monoclonal isolé ou fragment de liaison de celui-ci avec une α-fucosidase et au moins une endoglycosidase, dans lequel l'anticorps monoclonal isolé ou fragment de liaison de celui-ci comprend une VH de SEQ ID N° : 147 et une VL de SEQ ID N° : 148 comme défini dans la revendication 1, ou une H-CDR1, une H-CDR2, une H-CDR3, une L-CDR1, une L-CDR2, et une L-CDR3 comme défini dans l'une quelconque des revendications 3, 5 et 7 ;
(b) la génération d'un anticorps défucosylé ayant une seule N-acétylglucosamine (GlcNAc) ; et (c) l'ajout d'un glycane à la GlcNAc de la région Fc de l'anticorps pour former l'anticorps monoclonal isolé ou fragment de liaison de celui-ci avec ladite glycoforme comme défini dans l'une quelconque des revendications 1, 3, 5, et 7.

17. Anticorps monoclonal isolé ou fragment de liaison de celui-ci selon l'une quelconque des revendications 1 à 10, dans lequel l'anticorps monoclonal isolé est un anticorps multispécifique qui se lie en outre à au moins l'un des antigènes choisis dans le groupe constitué par Globo H et SSEA-3.
